# EUROPÄISCHE PATENTSCHRIFT

(11) **EP 1 098 874 B1**
(45) Veröffentlichungstag und Bekanntmachung des Hinweises auf die Patenterteilung: **18.06.2003**
(21) Anmeldenummer: 99938277.3
(22) Anmeldetag: 19.07.1999
(51) Int. Cl.: C07C 317/18, C07C 323/12, C07C 317/28, C07C 323/25, C07C 327/28, C07C 317/44, C07C 323/60, C07D 213/32, C07D 307/38, C07D 333/18, C07C 217/18, A61K 31/10, A61K 31/133, A61K 31/165, A61K 31/4402, A61K 31/381, A61K 31/341

(54) **BENZOCYCLOHEPTENE, VERFAHREN ZU IHRER HERSTELLUNG, PHARMAZEUTISCHE PRÄPARATE DIE DIESE ENTHALTEN SOWIE DEREN VERWENDUNG ZUR HERSTELLUNG VON ARZNEIMITTELN**
BENZOCYCLOHEPTENES, METHOD FOR THE PRODUCTION THEREOF, PHARMACEUTICAL PREPARATIONS CONTAINING THESE COMPOUNDS, AND THEIR USE FOR PRODUCING MEDICAMENTS
BENZOCYCLOHEPTENES, PROCEDE DE PRODUCTION DE CES COMPOSES PREPARATIONS PHARMACEUTIQUES CONTENANT CES COMPOSES ET UTILISATION DE CES COMPOSES POUR LA PREPARATION DE MEDICAMENTS

(30) Priorität: 18.07.1998 DE 19833786
(43) Veröffentlichungstag der Anmeldung: 16.05.2001
(73) Patentinhaber: Schering Aktiengesellschaft, 13353 Berlin (DE)
(72) Erfinder: BOHLMANN, Rolf, D-14055 Berlin (DE); KROLL, Jorg, D-12157 Berlin (DE); KÜNZER, Hermann, D-13347 Berlin (DE); FRITZEMEIER, Karl-Heinrich, D-13503 Berlin (DE); HEGELE-HARTUNG, Christa, D-45470 Mülheim a.d. Ruhr (DE); KNAUTHE, Rudolf, D-13467 Berlin (DE); LESSL, Monika, D-13467 Berlin (DE); LICHTNER, Rosemarie, D-10823 Berlin (DE); NISHINO, Yukishige, D-14089 Berlin (DE); PARCZYK, Karsten, D-12207 Berlin (DE); SCHNEIDER, Martin, D-13469 Berlin (DE)
(86) Internationale Anmeldenummer: EP9905093
(87) Internationale Veröffentlichungsnummer: WO00003979

(56) Entgegenhaltungen:
- WO-A-96/21656
- C.K. OSBORNE, ET AL.: "The importance of tamoxifen metabolism in tamoxifen-stimulated breast tumour growth" CANCER CHEMOTHERAPY AND PHARMACOLOGY., Bd. 34, Nr. 2, August 1994 (1994-08), Seiten 89-95, XP002121527 Springer Verlag, Berlin., DE ISSN: 0344-5704
- C.S. MURPHY, ET AL.: "Structure-activity relationships of nonisomerizable derivatives of tamoxifen: importance of hydroxyl group and side chain positioning for biological activity" MOLECULAR PHARMACOLOGY, Bd. 39, Nr. 3, März 1991 (1991-03), Seiten 421-828, XP002121528 Baltimore, MD, US ISSN: 0026-895X in der Anmeldung erwähnt
- R. MCCAGUE, ET AL.: "Nonisomerisable analogues of (Z)- and (E)-4-hydroxytamoxifen. Synthesis and endocrinological properties of substituted diphenylbenzocycloheptenes" JOURNAL OF MEDICINAL CHEMISTRY, Bd. 31, Nr. 7, Juli 1988 (1988-07), Seiten 1285-1290, XP002121529 American Chemical Society, Washington, DC, US ISSN: 0022-2623
- G.M. ANSTEAD, ET AL.: "2,3-Diarylindenes and 2,3-diarylindenones: synthesis, molecular structure, photochemistry, oestrogen receptor binding affinity, and comparisons with related triarylethylenes" JOURNAL OF MEDICINAL CHEMISTRY, Bd. 31, Nr. 7, Juli 1988 (1988-07), Seiten 1316-1326, XP002121530 American Chemical Society, Washington, DC, US ISSN: 0022-2623 in der Anmeldung erwähnt
- R. MCCAGUE, ET AL.: "Synthesis and oestrogen receptor binding of 6,7-dihydro- 8-phenyl-9-[4-[2-(dimethylamino)ethoxy]- phenyl]-5H-benzocycloheptene, a nonisomerisable analogue of tamoxifen. X-ray crystallographic studies" JOURNAL OF MEDICINAL CHEMISTRY, Bd. 29, Nr. 10, Oktober 1988 (1988-10), Seiten 2053-2059, XP002121531 American Chemical Society, Washington, DC, US ISSN: 0022-2623 in der Anmeldung erwähnt

## Beschreibung

Die vorliegende Erfindung betrifft Benzocycloheptene der allgemeinen Formel I 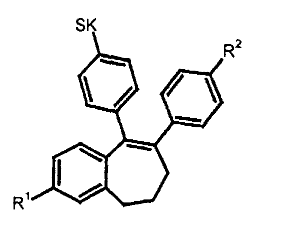 worin
- R¹ und R²: unabhängig voneinander für ein Wasserstoffatom, eine Hydroxygruppe, eine gegebenenfalls substituierte C₁-C₁₀-Alkoxygruppe, eine gegebenenfalls substituierte C₁-C₁₀-Alkanoyloxygruppe oder eine gegebenenfalls substituierte C₇-C₁₅-Aroyloxygruppe, sowie
- SK: für eine Seitenkette -A-B-Z,
wobei
A eine direkte Bindung oder ein Sauerstoffatom,
B eine gerad- oder verzweigtkettige, gegebenenfalls substituierte
Alkylen-, Alkenylen- oder Alkinylengruppe mit bis zu 10
Kohlenstoffatomen,
Z eine Gruppe -D-SOₓ-E-G, eine Aminogruppe -NR⁷R⁸ oder ein
Substituent G,
worin
D eine direkte Bindung oder eine Gruppe -NR³(R⁴-), R³ eine gerad- oder verzweigtkettige Alkyl-, Alkenyl- oder Alkinylgruppe mit bis zu 10 Kohlenstoffatomen sowie R⁴ eine gerad- oder verzweigtkettige, gegebenenfalls substituierte Alkylen-, Alkenylenoder Alkinylengruppe mit bis zu 10 Kohlenstoffatomen, wobei das Stickstoffatom auch in ein 4- bis 7-gliedriges Ringsystem eingebaut sein kann,
x 0, 1 oder 2,
E eine gerad- oder verzweigtkettige, gegebenenfalls substituierte Alkylen-, Alkenylen- oder Alkinylengruppe mit bis zu 10 Kohlenstoffatomen,
G eine teilweise oder vollständig fluorierte, gerad- oder verzweigtkettige Alkylgruppe mit bis zu 5 Kohlenstoffatomen, ein gegebenenfalls substituierter Aryl- oder Heteroarylrest, ein Carbamoylrest -C(O)-NR⁵R⁶ mit R⁵ und R⁶ in der Bedeutung von R⁷ und R⁸, ein Halogenatom oder ein Wasserstoffatom,
R⁷ und R⁸ unabhängig voneinander ein Wasserstoffatom, ein gerad- oder verzweigtkettiger, gegebenenfalls teilweise fluorierter Alkyl-, Alkenyl- oder Alkinylrest mit bis zu 14 Kohlenstoffatomen, der durch ein bis drei Heteroatome -O- und -S- und
Gruppierungen -NR⁹-, worin R⁹ ein Wasserstoffatom oder ein C₁-C₃-Alkylrest ist, unterbrochen sein kann, ein gegebenenfalls einoder zweifach substituierter Aryl- oder Heteroarylrest, ein gegebenenfalls ein- oder zweifach substituierter C₃-C₁₀-Cycloalkylrest, ein gegebenenfalls ein- oder zweifach substituierter C₄-C₁₅-Cycloalkylalkylrest, ein gegebenenfalls ein- oder zweifach substituierter C7-C20-Aralkylrest, ein gegebenenfalls ein- oder zweifach substituierter Heteroaryl-C₁-C₈-alkylrest oder ein gegebenenfalls substituerter Aminoalkylrest, ein Biphenylenrest oder ein Rest der Formel -C(O)R¹⁰, worin R¹⁰ die vorstehend für R⁷ bzw. R⁸ angegebenen Bedeutungen haben kann,
R⁷ und R⁸ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält und gegebenenfalls substituiert ist, bilden, ist,
bedeutet, und in -A-B-Z, wenn A für ein Sauerstoffatom und Z für G steht, G nicht ein Wasserstoffatom oder ein Halogenatom sein kann, oder wenn A für ein Sauerstoffatom und Z für eine Aminogruppe -NR⁷R⁸, worin R⁷ und R⁸ jeweils eine Methylgruppe bedeuten oder gemeinsam mit dem Stickstoffatom einen Pyrrolidinring bilden, steht, B mindestens 3 Kohlenstoffatome aufweist,
steht, sowie ausgenommen den Verbindung der allgemeinen Formel I, worin R¹=R²=Wasserskoff, SK=-A-B-Z, A=O, B=-(CH₂)₂- und Z = N(C₂H₅)₂.

Als C₁-C₁₀-Alkoxygruppe R¹ bzw. R² kommt beispielsweise eine Methoxy-, Ethoxy-, Propoxy-, Isopropoxy-, Butoxy-, Isobutoxy-, tert.-Butoxy-, Pentoxy-, Isopentoxy-, Neopentoxy, Heptyloxy-, Hexyloxy- oder Decyloxy-Gruppe in Betracht.

Die in R¹ und R² der allgemeinen Formel I enthaltenen Alkanoylgruppen sollen jeweils 1 bis 20 Kohlenstoffatome enthalten, wobei Formyl, Acetyl, Propionyl- und Isopropionylgruppen bevorzugt sind.

Aroylreste R¹ bzw. R² sind in erster Linie Benzoate sowie im Phenylrest substituierte Benzoate; es können dies aber auch die anderen, aus den weiter unten näher erläuterten Arylresten abgeleiteten Aroyl- sowie Heteroaroylreste sein.

Für B kommt in erster Linie eine geradkettige Alkylengruppe mit 1 bis 6 Kohlenstoffatomen in Betracht.

Als Alkylgruppe R³, R⁷ und R⁸ sind gerad- oder verzweigtkettige Alkylgruppen mit bis zu 10 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Diese können bis zu 3 Unsättigungen (Doppel- und/oder Dreifachbindungen) aufweisen.

Die Alkylgruppen R³, R⁷ und R⁸ können teilweise oder vollständig fluoriert oder substituiert sein.

Als teilweise oder vollständig fluorierte geradkettige oder verzweigte C₁-C₁₀-Alkylgruppe ist beispielsweise die Trifluormethyl-, Pentafluorethylgruppe-, 2,2,2-Trifluorethyl-, 4,4,4-Trifluorbutyl-, 3,3,4,4,4-Pentafluorbutyl-, 4,4,5,5,5-Pentafluorpentyl- oder die Nonafluorbutylgruppe zu nennen.
Diese können ebenfalls bis zu 3 Unsättigungen (Doppel- und/oder Dreifachbindungen) aufweisen.

Bei dem für R⁹ stehenden C₁-C₃-Alkylrest handelt es sich um einen Methyl-, Ethyl-, Propyl- oder Isopropylrest; der Methylrest ist bevorzugt.

Für den Arylrest R⁷ bzw. R⁸ und G und den Arylrest innerhalb des Arylalkylrestes R⁷ bzw. R⁸ können die folgenden, gegebenenfalls ein- oder mehrfach substituierten Reste stehen: ein monocyclischer carbocyclischer Rest, beispielsweise der Phenylrest; ein monocyclischer heterocyclischer Rest, beispielsweise der Thienyl-, Furyl-, Pyrrolyl-, Imidazolyl-, Pyrazolyl-, Pyridyl-, Pyrazinyl-, Pyrimidinyl-, Pyridazinyl-, Thiazolyl-, Oxazolyl-, Furazanyl-, Pyrrolinyl-, Imidazolinyl-, Pyrazolinyl-, Thiazolinyl-, Triazolyl-, Tetrazolylrest, und zwar alle möglichen Isomeren bezüglich der Positionen der Heteroatome sowie der Verknüpfungsstelle zum Schwefelatom in der Seitenkette;
ein kondensierter carbocyclischer Rest, beispielsweise der Naphthyl- oder Phenanthrenylrest,
ein kondensierter Rest, der sich aus carbocyclischen und heterocyclischen Resten zusammensetzt, beispielsweise der Benzofuranyl-, Benzothienyl-, Benzimidazolyl-, Benzothiazolyl-, Naphto[2,3-b]thienyl-, Thianthrenyl-, Isobenzofuranyl-, Chromenyl-, Xanthenyl-, Phenoxathiinyl-, Indolizinyl-, Isoindolyl-, 3H-Indolyl, Indolyl-, Indazolyl-, Purinyl-, Chinolizinyl-, Isochinolyl-, Chinolyl-, Phthalazinyl-, Naphthyridinyl-,
Chinoxalinyl-, Chinazolinyl-, Cinnolinyl-, Pteridinyl-, Carbazolyl-, β-Carbolinyl-, Acridinyl-, Phenazinyl-, Phenothiazinyl-, Phenoxazinyl-, Indolinyl-, Isoindolinyl-, Imidazopyridyl-, Imidazopyrimidinyl- oder ein kondensiertes polyheterocyclisches System, beispielsweise Furo[2,3-b]pyrrol oder Thieno[2,3-b]furan.

Als Substituenten an den Resten B, G, R³, R⁴, R⁵, R⁶, R⁷, R⁸ und R¹⁰ sowie R³ gemeinsam mit R⁴ kommen die nachstehenden Substituenten in Betracht, wobei die Reste einfach oder mehrfach, identisch oder unterschiedlich, mit diesen Substituenten substituiert sein können:
Halogenatome: Fluor, Chlor, Brom, Iod;
Amino-, Mono(C₁₋₈-alkyl)- oder Di(C₁₋₈-alkyl)amino, wobei beide Alkylgruppen identisch oder verschieden sind, insbesondere Methylamino oder Ethylamino, Dimethylamino, Diethylamino oder Methylethylamino; Di(aralkyl)amino, wobei beide Aralkylgruppen identisch oder verschieden sind;
Hydroxylgruppen;
freie, veresterte oder in Form eines Salzes vorliegende Carboxylgruppen: verestert mit einer Carboxycarbonylgruppe, beispielsweise Methoxycarbonyl oder Ethoxycarbonyl; als Salz beispielsweise in Form des Natrium- oder Kaliumsalzes;
Alkylgruppen mit 1 bis 8 Kohlenstoffatomen, wie beispielsweise die Methyl-, Ethyl-, n-oder iso-Propyl-, n-, iso- oder tert.-Butylgruppe, gegebenenfalls substituiert mit einem oder mehreren Halogenatomen, zum Beispiel mit Fluor wie die Trifluormethyl- oder Pentafluorethylgruppe;
Oxo-, Azido-, Cyano-, Nitro- oder Formylgruppen;
Acylgruppen wie Acetyl, Propionyl, Butyryl, Benzoyl;
Acyloxygruppen wie Acetoxy, Reste der Formel-O-CO-(CH₂)ₙ-COOH mit n = 1 bis 5;
C₁-C₄-Alkoxygruppen wie zum Beispiel Methoxy, Ethoxy, Propoxy, Isopropoxy, Butoxy;
Alkylthiogruppen, beispielsweise Methylthio, Ethylthio, Propylthio, Isopropylthio, Butylthio, alle gegebenenfalls fluoriert;
Carbamoylgruppen;
Alkenylgruppen, beispielsweise Vinyl, Propenyl;
Alkinylgruppen, beispielsweise Ethinyl, Propinyl;
C₆-C₁₂-Arylgruppen wie Phenyl, Furyl, Thienyl, die wiederum ein- bis dreifach substituiert sein können.

Als Cycloalkylgruppe für die Substituenten R⁷ und R⁸ kommen substituierte und unsubstituierte Reste mit 3 bis 10 Kohlenstoffatomen in Betracht; vor allem ist die Cyclopropyl- und Cyclopentylgruppe und als Alkylcycloalkylgruppe die Methylcyclopropyl- und Methylcyclopentylgruppe zu nennen.

Die C₇-C₂₀-Aralkylreste in R⁷ und R⁸ können im Ring bis 14 C-Atome, bevorzugt 6 bis 10 und in der Alkylkette 1 bis 8, bevorzugt 1 bis 4 C-Atome, enthalten.
Ein Heteroaryl-C₁-C₈-alkylrest in R⁷ und R⁸ weist als Heteroarylteil einen der bereits genannten Heteroarylreste auf; die Alkylkette verfügt über 1 bis 8, bevorzugt über 1 bis 4 C-Atome.
Als Aralkylreste kommen beispielweise in Betracht Benzyl, Phenylethyl, Naphtylmethyl, Naphtylethyl und als Heteroaryl-alkylrest Furylmethyl, Thienylethyl, Pyridylpropyl.
Die Ringe können einfach oder mehrfach substituiert sein.

Wenn R⁷ und R⁸ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält, ist dies insbesondere ein Pyrrolidin-, Piperidin-, Morpholin- oder Piperazinring.

Als Substituenten für den Aryl-, Heteroaryl-, Aralkyl- und Heteroarylalkylrest seien insbesondere ein Trifluormethyl-, Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind, oder der 1-Methoxyacetylaminorest genannt.

Das Schwefelatom in der Seitenkette kann als einfache Schwefelbrücke (Sulfid), als Sulfon oder Sulfoxid vorliegen.

Freie Hydroxygruppen in den Verbindungen der allgemeinen Formel I können funktionell abgewandelt sein, beispielsweise durch Veretherung oder Veresterung; freie Hydroxygruppen sind jedoch bevorzugt.
Als Ether- und Acylreste (Schutzgruppen) kommen die dem Fachmann bekannten Reste wie z.B. der Methoxymethyl-, Methoxyethyl-, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Methyl-, tert.-Butyl-, Benzyl-, para-Nitrobenzyl-, para-Methoxybenzyl, Formyl-, Acetyl-, Propionyl-, Isopropionyl-, Butyryl-, Pivalyl-, Benzoyl-Rest in Frage. Eine Übersicht befindet sich z.B. in "Protective Groups in Organic Synthesis" Theodora W. Green, John Wiley and Sons).

Als spezifische Seitenketten, in denen A für ein Sauerstoffatom steht, seien genannt
-O-(CH₂)₅S(CH₂)₃C₂F₅
-O-(CH₂)₅SO(CH₂)₃C₂F₅
-O-(CH₂)₅SO₂(CH₂)₃C₂F₅
-O-(CH₂)₂-N(CH₃)-(CH₂)₃-S-(CH₂)₃C₂F₅
-O-(CH₂)₂-N(CH₃)-(CH₂)₃-SO-(CH₂)₃C₂F₅
-O-(CH₂)₅S(CH₂)-C(O)N(CH₃)-(CH₂)₃CH₃
-O-(CH₂)₅SO(CH₂)-C(O)N(CH₃)-(CH₂)₃CH₃
-O-(CH₂)₂-NH(CH₂)OH
-O-(CH₂)₂-N(CH₃)-(CH₂)₂-SO(CH₂)-C(O)N(CH₃)-(CH₂)₃CH₃
-O-(CH₂)₂-N(CH₃)-(CH₂)₂-SO₂(CH₂)-C(O)N(CH₃)-(CH₂)₃CH₃
-O-(CH₂)₆S(CH₂)-C(O)N(CH₃)-(CH₂)₃CH₃
-O-(CH₂)₆SO(CH₂)-C(O)N(CH₃)-(CH₂)₃CH₃
-O-CH₃
-O-(CH₂)₅-F
-O-(CH₂)₄-F
-O-(CH₂)₃-F
-O-(CH₂)₂-F
-O-(CH₂)₅-Cl
-O-(CH₂)₄-Cl
-O-(CH₂)₃-Cl
-O-(CH₂)₂-Cl
-O-(CH₂)₆S(CH₂)₃C₂F₅
-O-(CH₂)₆SO(CH₂)₃C₂F₅
-O-(CH₂)₆SO(CH₂)-2-Pyridyl
-O-(CH₂)₅SO(CH₂)-2-Pyridyl
-O-(CH₂)₅S(CH₂)₂C₃F₇
-O-(CH₂)₄S(CH₂)₃C₂F₅
-O-(CH₂)₄SO₂(CH₂)₃C₂F₅
-O-(CH₂)₄SO₂(CH₂)₃C₂F₅
-O-(CH₂)₄S(CH₂)-2-Pyridyl
-O-(CH₂)₄SO(CH₂)-2-Pyridyl
-O-(CH₂)₅S(CH₂)-2-Pyridyl
-O-(CH₂)₅SO(CH₂)-2-Pyridyl
-O-(CH₂)₆S(CH₂)-2-Pyridyl
-O-(CH₂)₆SO(CH₂)-2-Pyridyl
-O-(CH₂)₅S(CH₂)-2-Furyl
-O-(CH₂)₅SO(CH₂)-2-Furyl
-O-(CH₂)₅SO₂(CH₂)-2-Furyl
-O-(CH₂)₅S(CH₂)-2-Thienyl
-O-(CH₂)₅SO(CH₂)-2-Thienyl
-O-(CH₂)₅S(CH₂)₄-F
-O-(CH₂)₅SO(CH₂)₄-F
-O-(CH₂)₅S(CH₂)₃-CF₃
-O-(CH₂)₅SO(CH₂)₃-CF₃
-O-(CH₂)₅-N(CH₃)-(CH₂)₃-C₂F₅
-O-(CH₂)₅S(CH₂)-Phenyl
-O-(CH₂)₅SO(CH₂)-2-Phenyl
-O-(CH₂)₅S(CH₂)-p-Tolyl
-O-(CH₂)₅SO(CH₂)-p-Tolyl
-O-(CH₂)₅S(CH₂)-p-CF₃-Phenyl
-O-(CH₂)₅SO(CH₂)-p-CF₃-Phenyl
-O-(CH₂)₅S-Phenyl
-O-(CH₂)₅SO-Phenyl
-O-(CH₂)₅S-(p-Tolyl)
-O-(CH₂)₅SO-(p-Tolyl)
-O-(CH₂)₅S-(p-CF₃-Phenyl)
-O-(CH₂)₅SO-(p-CF₃-Phenyl)

Als Seitenketten, worin A für eine direkte Bindung steht, kommen beispielsweise in Betracht (DE 1 98 06 357.1)
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂)₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₈F₁₇
-(CH₂)₅N(CH₃)H
-(CH₂)₅N(CH₃)(CH₂)₉H
-(CH₂)₅-1-Pyrrolidinyl
-(CH₂)₉S(CH₂)₃C₂F₅
-(CH₂)₉SO(CH₂)₃C₂F₅
-(CH₂)₉SO₂(CH₂)₃C₂F₅.

Desweiteren kommen in Betracht die Seitenketten der allgemeinen Teilformel 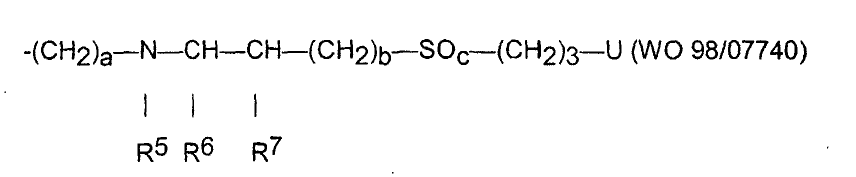 wobei
a 4, 5 oder 6,
b 0, 1 oder 2,
c 0, 1 oder 2,
R⁵ ein Wasserstoffatom oder eine C₁₋₅-Alkylgruppe,
R⁶ und R⁷ je ein Wasserstoffatom, oder
R⁵ und R⁶ gemeinsam eine Alkylengruppe ―(CH₂)_{d}― mit d = 2, 3, 4 oder 5 und R⁷ ein Wasserstoffatom oder
R⁵ und R⁷ gemeinsam eine Alkylengruppe ―(CH₂)ₑ― mit e'= 2, 3 oder 4 und R⁶ ein Wasserstoffatom, und
U ein unsubstituierter oder ein- bis fünffach fluorierter Ethylrest ist, oder der terminale Substituent -(CH₂)₃-U in der Seitenkette durch einen gegebenenfalls substituierten Aryl- oder Heteroarylrest, der direkt oder über eine Mono-, Di- oder Trimethylengruppe an das Schwefelatom gebunden ist, ersetzt ist,
und von diesen wiederum insbesondere die Seitenketten
-(CH₂)₅N(CH₃)(CH₂)₃S(CH₂)₃C₂F₅ und
-(CH₂)₅N(R⁵)(CHR⁶)CH₂S(CH₂)₃C₂F₅ mit R⁵+R⁶ = -(CH₂)₃-.

Im Sinne der vorliegenden Erfindung bevorzugte Verbindungen sind
(4,4,5,5,5-Pentafluorpentyl)-{5-[4-(6-phenyl-8,9-dihydro-7H-benzocyclohepten-5-yl)-phenoxy]-pentyl}-sulfid
(4,4,5,5,5-Pentafluorpentyl)-{5-[4-(6-phenyl-8,9-dihydro-7H-benzocyclohepten-5-yl)-phenoxy]-pentyl}-sulfoxid
Methyl-[3-(4,4,5,5,5-pentafluorpentylthio)-propyl]-{2-[4-(6-phenyl-8,9-dihydro-7Hbenzocyclohepten-5-yl)-phenoxy]-ethyl}-amin
Methyl-[3-(4,4,5,5,5-pentafluorpentylsulfinyl)-propyl]-{2-[4-(6-phenyl-8,9-dihydro-7H-benzocyclohepten-5-yl)-phenoxy]-ethyl}-amin
S-{5-[4-(6-Phenyl-8,9-dihydro-7H-benzocyclohepten-5-yl)-phenoxy]-pentyl}thioacetat
N-Butyl-N-methyl-2-{5-[4-(6-phenyl-8,9-dihydro-7H-benzocyclohepten-5-yl)-phenoxy]-pentylthio}-acetamid
N-Butyl-N-methyl-2-{5-[4-(6-phenyl-8,9-dihydro-7H-benzocyclohepten-5-yl)-phenoxy]-pentansulfinyl}-acetamid
5-{4-[5-(4,4,5,5,5-Pentafluor-pentylthio)-pentyloxy]-phenyl}-6-phenyl-8,9-dihydro-7H-benzocyclohepten-2-ol
5-{4-[5-(4,4,5,5,5-Pentafluor-pentanesulfinyl)-pentyloxy]-phenyl}-6-phenyl-8,9-dihydro-7H-benzocyclohepten-2-ol
5-[4-(2-{Methyl-[3-(4,4,5,5,5-pentafluor-pentansulfinyl)-propyl]-amino}-ethoxy)-phenyl]-6-phenyl-8,9-dihydro-7H-benzocyclohepten-2-ol
5-[4-(2-{Methyl-[3-(4,4,5,5,5-pentafluor-pentylthio)-propyl]-amino}-ethoxy)-phenyl]-6-phenyl-8,9-dihydro-7H-benzocyclohepten-2-ol
N-Butyl-N-methyl-2-{5-[4-(2-hydroxy-6-phenyl-8,9-dihydro-7H-benzocyclohepten-5-yl)-phenoxy]-pentylthio}-acetamid
5-{4-[5-(4,4,5,5,5-Pentafluor-pentanesulfonyl)-pentyloxy]-phenyl}-6-phenyl-8,9-dihydro-7H-benzocyclohepten-2-ol
N-Butyl-N-methyl-2-{5-[4-(2-hydroxy-6-phenyl-8,9-dihydro-7H-benzocyclohepten-5-yl)-phenoxy]-pentansulfinyl}-acetamid
N-Butyl-2-[2-({2-[4-(2-hydroxy-6-phenyl-8,9-dihydro-7H-benzocyclohepten-5-yl)-phenoxy]-ethyl}-methyl-amino)-ethansulfinyl]-N-methyl-acetamid
N-Butyl-2-[2-({2-[4-(2-hydroxy-6-phenyl-8,9-dihydro-7H-benzocyclohepten-5-yl)-phenoxy]-ethyl}-methyl-amino)-ethansulfonyl]-N-methyl-acetamid
6-(4-Hydroxy-phenyl)-5-{4-[5-(4,4,5,5,5-pentafluor-pentansulfonyl)-pentyloxy]-phenyl}-8,9-dihydro-7H-benzocyclohepten-2-ol
N-Butyl-2-{6-[4-(2-hydroxy-6-phenyl-8,9-dihydro-7H-benzocyclohepten-5-yl)-phenoxy]-hexansulfinyl}-N-methyl-acetamid
N-Butyl-2-{6-[4-(2-hydroxy-6-phenyl-8,9-dihydro-7H-benzocyclohepten-5-yl)-phenoxy]-hexylthio}-N-methyl-acetamid
6-(4-Hydroxy-phenyl)-5-{4-[5-(4,4,5,5,5-pentafluorpentylthio)-pentyloxy]-phenyl}-8,9-dihydro-7H-benzocyclohepten-2-ol
6-(4-Hydroxy-phenyl)-5-{4-[5-(4,4,5,5,5-pentafluorpentan-sulfinyl)-pentyloxy]-phenyl}-8,9-dihydro-7H-benzocyclohepten-2-ol
5-{4-[4-(4,4,5,5,5-Pentafluor-pentylthio)-butyloxy]-phenyl}-6-phenyl-8,9-dihydro-7H-benzocyclohepten-2-ol
6-Phenyl-5-{4-[4-(pyridin-2-ylmethylthio)-butyloxy]-phenyl}-8,9-dihydro-7Hbenzocyclohepten-2-ol
6-Phenyl-5-{4-[5-(pyridin-2-ylmethylthio)-pentyloxy]-phenyl}-8,9-dihydro-7Hbenzocyclohepten-2-ol
6-Phenyl-5-{4-[6-(pyridin-2-ylmethylthio)-hexyloxy]-phenyl}-8,9-dihydro-7Hbenzocyclohepten-2-ol
5-{4-[4-(4,4,5,5,5-Pentafluor-pentansulfinyl)-butyloxy]-phenyl}-6-phenyl-8,9-dihydro-7H-benzocyclohepten-2-ol
6-Phenyl-5-{4-[4-(pyridin-2-ylmethansulfinyl)-butyloxy]-phenyl}-8,9-dihydro-7Hbenzocyclohepten-2-ol
5-{4-[6-(4,4,5,5,5-Pentafluor-pentylthio)-hexyloxy]-phenyl}-6-phenyl-8,9-dihydro-7H-benzocyclohepten-2-ol
5-{4-[6-(4,4,5,5,5-Pentafluor-pentansulfinyl)-hexyloxy]-phenyl}-6-phenyl-8,9-dihydro-7H-benzocyclohepten-2-ol
6-Phenyl-5-{4-[6-(pyridin-2-ylmethansulfinyl)-hexyloxy]-phenyl}-8,9-dihydro-7Hbenzocyclohepten-2-ol
6-Phenyl-5-{4-[5-(pyridin-2-ylmethansulfinyl)-pentyloxy]-phenyl}-8,9-dihydro-7Hbenzocyclohepten-2-ol
5-{4-[5-(Furan-2-ylmethylthio)-pentyloxy]-phenyl}-6-phenyl-8,9-dihydro-7Hbenzocyclohepten-2-ol
6-Phenyl-5-{4-[5-(thien-2-ylmethylthio)-pentyloxy]-phenyl}-8,9-dihydro-7Hbenzocyclohepten-2-ol
5-{4-[5-(Furan-2-ylmethansulfinyl)-pentyloxy]-phenyl}-6-phenyl-8,9-dihydro-7Hbenzocyclohepten-2-ol
5-{4-[5-(Furan-2-ylmethansulfonyl)-pentyloxy]-phenyl}-6-phenyl-8,9-dihydro-7Hbenzocyclohepten-2-ol
6-Phenyl-5-{4-[5-(thien-2-ylmethansulfonyl)-pentyloxy]-phenyl}-8,9-dihydro-7Hbenzocyclohepten-2-ol
6-Phenyl-5-{4-[5-(thien-2-ylmethansulfinyl)-pentyloxy]-phenyl}-8,9-dihydro-7Hbenzocyclohepten-2-ol
5-{4-[5-(3,3,4,4,5,5,5-Heptafluor-pentylthio)-pentyloxy]-phenyl}-6-phenyl-8,9-dihydro-7H-benzocyclohepten-2-ol
5-{4-[2-(2-Hydroxy-ethylamino)-ethoxy]-phenyl}-6-phenyl-8,9-dihydro-7Hbenzocyclohepten-2-ol
5-{4-[5-(4-Fluor-butylthio)-pentyloxy]-phenyl}-6-phenyl-8,9-dihydro-7Hbenzocyclohepten-2-ol
5-{4-[5-(4-Fluor-butansulfinyl)-pentyloxy]-phenyl}-6-phenyl-8,9-dihydro-7Hbenzocyclohepten-2-ol
6-Phenyl-5-{4-[5-(4,4,4-trifluor-butylthio)-pentyloxy]-phenyl}-8,9-dihydro-7Hbenzocyclohepten-2-ol
6-Phenyl-5-{4-[5-(4,4,4-trifluor-butansulfinyl)-pentyloxy]-phenyl}-8,9-dihydro-7Hbenzocyclohepten-2-ol
5-(4-{5-[Methyl-(4,4,5,5,5-pentafluorpentyl)-amino]-pentyloxy}-phenyl)-6-phenyl-8,9-dihydro-7H-benzocyclohepten-2-ol
5-[4-(5-Benzylthio-pentyloxy)-phenyl]-6-phenyl-8,9-dihydro-7H-benzocyclohepten-2-ol
5-[4-(5-Benzylsulfinyl-pentyloxy)-phenyl]-6-phenyl-8,9-dihydro-7Hbenzocyclohepten-2-ol
5-{4-[5-(4-Methyl-benzylthio)-pentyloxy]-phenyl}-6-phenyl-8,9-dihydro-7Hbenzocyclohepten-2-ol
5-{4-[5-(4-Methyl-benzylsulfinyl)-pentyloxy]-phenyl}-6-phenyl-8,9-dihydro-7Hbenzocyclohepten-2-ol
6-Phenyl-5-{4-[5-(4-trifluormethyl-benzylthio)-pentyloxy]-phenyl}-8,9-dihydro-7Hbenzocyclohepten-2-ol
6-Phenyl-5-{4-[5-(4-trifluormethyl-benzylsuffinyl)-pentyloxy]-phenyl}-8,9-dihydro-7H-benzocyclohepten-2-ol
6-Phenyl-5-[4-(5-phenylthio-pentyloxy)-phenyl]-8,9-dihydro-7H-benzocyclohepten-2-ol
6-Phenyl-5-[4-(5-phenylsulfinyl-pentyloxy)-phenyl]-8,9-dihydro-7Hbenzocyclohepten-2-ol
6-Phenyl-5-[4-(5-phenylsulfonyl-pentyloxy)-phenyl]-8,9-dihydro-7Hbenzocyclohepten-2-ol
5-{4-[5-(4-tert.-Butyl-phenylthio)-pentyloxy]-phenyl}-6-phenyl-8,9-dihydro-7Hbenzocyclohepten-2-ol
5-{4-[5-(4-tert.-Butyl-phenylsulfinyl)-pentyloxy]-phenyl}-6-phenyl-8,9-dihydro-7Hbenzocyclohepten-2-ol
5-{4-[5-{4-tert.-Butyl-phenylsulfonyl)-pentyloxy]-phenyl}-6-phenyl-8,9-dihydro-7Hbenzocyclohepten-2-ol
6-Phenyl-5-{4-[5-(4-trifluormethyl-phenylthio)-pentyloxy]-phenyl}-8,9-dihydro-7Hbenzocyclohepten-2-ol
6-Phenyl-5-{4-[5-(4-trifluormethyl-phenylsulfinyl)-pentyloxy]-phenyl}-8,9-dihydro-7H-benzocyclohepten-2-ol
6-Phenyl-5-{4-[5-(4-trifluormethyl-phenylsulfonyl)-pentyloxy]-phenyl}-8,9-dihydro-7H-benzocyclohepten-2-ol

Die vorliegende Erfindung betrifft außer diesen Verbindungen der allgemeinen Formel I, wenn in der Seitenkette SK ein Stickstoffatom enthalten ist, auch deren physiologisch verträgliche Additionssalze mit organischen und anorganischen Säuren, diese Verbindungen der allgemeinen Formel I inclusive der Additionssalze enthaltende pharmazeutische Präparate sowie deren Verwendung zur Herstellung von Arzneimitteln.

Zur Bildung von Säureadditionssalzen sind anorganische und organische Säuren geeignet, wie sie dem Fachmann zur Bildung physiologisch verträglicher Salze bekannt sind. Als Additionssalze mit Säuren sind insbesondere die Hydrochloride, Hydrobromide, Acetate, Citrate, Oxalate, Tartrate und die Methansulfonate zu nennen.

Die Verbindungen der allgemeinen Formel 1 stellen Verbindungen mit starker antiestrogener Wirksamkeit dar.

Bei den erfindungsgemäßen Verbindungen handelt es sich um selektive Estrogene, deren Wirkung gewebeselektiv auftritt. Insbesondere tritt die estrogene Wirkung am Knochen auf. Am Uterus und in der Leber tritt dagegen keine oder nur eine geringfügige estrogene Wirkung auf.
Die Verbindungen können auch über antiestrogene Wirksamkeit verfügen, die sich beispielsweise im Antiuteruswachstumstest oder in Tumormodellen nachweisen läßt. Verbindungen mit einem derartigen Profil werden neuerdings als Selective Estrogen Receptor Modulators (SERMs) bezeichnet (Structure-Activity Relationships of Selective Estrogen Receptor Modulators: Modifications to the 2-Arylbenzothiophene Core of Raloxifene, T. A. Grese et al, J. Med. Chem. 1997, 40, 146- 167).
Prominentester Vertreter dieser Verbindungsklasse ist das Raloxifen, welches inzwischen als Medikament für die Prävention und die Behandlung der postmenopausalen Osteoporose zugelassen ist.

Verbindungen mit antiestrogenen Eigenschaften, d.h. Stoffe mit Hemmwirkungen gegenüber Estrogenen, sind bereits zahlreich beschrieben worden. Es handelt sich dabei um Verbindungen sowohl mit steroidalem als auch mit nicht-steroidalem Grundgerüst.

Das erstmals aus BE 637,389 hervorgehende Tamoxifen, (Z)-2-[4-(1,2-Diphenyl-1-butenyl)-phenoxy]-N,N-dimethylethylamin, wird seit längerem als Antiestrogen zur Therapie von Brustkrebs eingesetzt, wie dies auch in "Cancer Chemother Pharmacol., Bd. 34(2), 1994, 89-95 beschrieben wird.

Raloxifen, 6-Hydroxy-2-(4-hydroxyphenyl)-3-[4-(2piperidinoethoxy)benzoyl]benzo[b]-thiophen, und dessen Hydrochlorid, können zur Behandlung und Prophylaxe der Osteoporose verwendet werden (EP 0 584 952 B1).

Das aus der EP A 0 138 504 B1 hervorgehende Steroid-Derivat 7α-[9-(4,4,5,5,5-Pentafluorpentylsulfinyl)-n-nonyl]-estra-1,3,5(10)-trien-3,17β-diol (EP A 0 138 504, Seite 58, vorletzte Verbindung) befindet sich gegenwärtig in klinischer Entwicklung für hormonabhängige Tumoren (Brustkrebs).

Pharmazeutische Zusammensetzungen, die Sexualsteroid-Inhibitoren enthalten, welche ein steroidales Grundgerüst, das eine 7α-Seitenkette bei gleichzeitiger Anwesenheit mindestens eines weiteren Substituenten in Position 14, 15 oder 16 aufweist, sind Gegenstand der EP-A 0 367 576. Diese Patentanmeldung betrifft auch nicht-steroidale, antiestrogene Verbindungen, u. a. die Verbindung EM 800. Diese Verbindung wurde ursprünglich als reines Antiestrogen beschrieben; inzwischen wurde aber gefunden, daß diese Verbindung ebenfalls über eine deutliche estrogene Partialwirkung verfügt.

Bei den aus der WO 96/21656 hervorgehenden Estrogenagonisten und -antagonisten handelt es sich u. a. um Benzocyclopentan-, hexan und heptan-Derivate, die im aromatischen Teil eine Hydroxygruppe, am Kohlenstoffatom 5 einen Stickstoffaromaten oder einen in 4-Stellung mit einer Seitenkette versehenen Phenylrest und am Kohlenstoffatom 6 u. a. einen gegebenenfalls eine Seitenkette aufweisenden Phenylrest tragen. Verbindungen mit einer Unsättigung im ankondensierten Strukturteil sind dort nicht offenbart. Tatsächlich beschrieben sind sogar nur Benzocyclohexan-Derivate.
Einige Verbindungen mit Benzocyclohepten-Grundstruktur sind verschiedenen Publikationen zu entnehmen (Mol. Pharmacol. 1991, 39: 421 - 428; J. Med. Chem., 1986, 29, 2053 - 2059; J. Med. Chem., 1988, 31, 1316 - 1326; ). Diese Verbindungen weisen in 4-Stellung des an das Kohlenstoffatom 5 gebundenen Phenylrest eine Methoxy-, 2-(Dimethylamino)ethoxy- oder 2-(1-Pyrrolidinyl)ethoxygruppe auf. Von diesen Verbindungen ist nicht gesagt, daß es sich um selektive Estrogene handelt.

### Pharmakologische Untersuchung der erfindungsgemäßen Verbindungen

Der Einfluß der erfindungsgemäßen Verbindungen auf den Uterus wurde im Uteruswachstumstest (estrogene Wirkung) und im Antiuteruswachstumstest (antiestrogene Wirkung), beide an der infantilen Ratte durchgeführt, untersucht.

### Estrogene/antiestrogene Wirkung in vivo

### Uteruswachstumstest an der infantilen Ratte (n=5 Tiere/Gruppe)

Sowohl Uterus wie auch Vagina zeigen bei infantilen Tieren bei deren Behandlung mit einer estrogen wirksamen Substanz eine von der estrogenen Wirksamkeit abhängige Gewichtszunahme. Am Uterus kommt es unter estrogener Wirkung zudem zu einer Proliferation und Höhenzunahme des luminalen Epithels.
Immature, intakte Ratten (Körpergewicht 40-50g) erhalten über 3 Tage (d1-d3) die Substanz s.c.. Am Tag 4 (d4) werden die Tiere mit CO₂ getötet. Die Uteri werden herauspräpariert und gewogen. Ein Uterusstück, vorzugsweise ein Uterushorn, wird für die histologische Auswertung in Formaldehyd fixiert und in Paraffin eingebettet. Die Stimulierung der Organgewichte (bezogen auf mg/100g Körpergewicht) sowie die Epithelhöhe wird in prozentualer Stimulierung im Vergleich zur Referenzverbindung 17β-Estradiol angegeben. (Substitutiondosis E₂ 0,3 µg/Tier).

Die erfindungsgemäßen Verbindungen weisen keine oder nur geringe stimulierende Wirkung auf den Uterus auf.

### Antiuteruswachstumstest an der infantilen Ratte (n=5 Tiere/Gruppe)

Der Uterus infantiler estrogensubstituierter Ratten kann als Testmodell genutzt werden, um eine direkte Wirkung von Substanzen mit antiestrogenen Eigenschaften nachzuweisen. Der Parameter der Estrogenwirkung ist das bei infantilen Ratten durch Estradiol induzierte Uteruswachstum, das durch gleichzeitige Gabe einer Substanz mit antiöstogener Wirkung gehemmt wird.
Die Testsubstanzen werden s.c. an 3 aufeinanderfolgenden Tagen (d1-d3) in Kombination mit einer Substitutionsdosis 0.3 µg/Tier/Tag 17β-Estradiol behandelt. Als Positivkontrolle dient 17β-Estradiol alleine, als Negativkontrolle die Vehikel-Gruppe. An Tag 4 (d4) werden die Tiere getötet, Uteri und Vaginae werden herauspräpariert und gewogen. Die Organgewichte werden auf mg/100 g Körpergewicht umgerechnet, dann der Mittelwert und die Standardabweichung für jede Dosierung berechnet. Die Hemmung des durch 17β-Estradiol induzierten Uterus- bzw. Vaginalwachstums wird als Hemmung in % angegeben.

Die erfindungsgemäßen Verbindungen weisen zum großen Teil eine deutlich ausgeprägte Hemmung des durch 17β-Estradiol induzierten Uteruswachstums auf.

Die erfindungsgemäßen Verbindungen sind somit hinsichtlich ihrer Wirkung am Uterus den Verbindungen des Standes der Technik im Sinne vorliegender Erfindung dahingehend überlegen, daß sie an diesem Organ geringere oder gar keine estrogene Wirkung aufweisen.

### Knochenuntersuchungen

### Methode

3 Monate alte weibliche Ratten werden ovarektomiert und unmittelbar nach der Operation 28 Tage lang 1 mal täglich mit der Testverbindung behandelt. Die Applikation erfolgt subcutan in Arachisöl/Ethanol. Die Tiere werden am Tag nach der letzten Applikation getötet und Tibia sowie die Uteri entnommen. Die Uteri werden gewogen, fixiert und für histologische Untersuchungen aufgearbeitet. Die Bestimmung der Knochendichte erfolgt ex vivo an präparierten Langknochen mittels pQCT (Quantitative Computertomographie). Die Messungen werden im Abstand von 4 - 6 mm vom Gelenkkopf der proximalen Tibia durchgeführt.

Durch die Ovarektomie vermindert sich die Dichte des trabekulären Knochens im gemessenen Bereich von ca. 400 mg Ca²⁺/cm³ auf ca. 300 mg Ca²⁺/cm³. Durch die Behandlung mit einer Verbindung der allgemeinen Formel I gemäß vorliegender Erfindung (Dosierungen von 0.1 - 100 µg/Tier/Tag) wird der Abbau der Knochendichte verhindert bzw. gehemmt. Gemessen wurde die Knochendichte an der proximalen Tibia.

Hinsichtlich ihrer knochenprotektiven Wirkung weisen die erfindungsgemäßen Verbindungen eine den Verbindungen des Standes der Technik vergleichbare Wirkung bei gleichzeitig abgeschwächter oder nicht vorhandener uterotropher Estrogenwirkung auf.

Die erfindungsgemäßen Verbindungen sind also im Sinne einer selektiven Wirkung am Knochen hinsichtlich einer abgeschwächten Wirkung am Uterus stärker dissoziiert als die Verbindungen des Standes der Technik.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die nachstehenden Indikationen.

Die Verbindungen können, sowohl nach oraler als auch parenteraler Gabe, für die folgenden Indikationen eingesetzt werden: Linderung der Symptome der Andropause und Menopause, d.h. zur männlichen und weiblichen Hormonersatz-Therapie (HRT), und zwar sowohl zur Prävention als auch zur Behandlung; zur Behandlung der mit einer Dysmenorrhoe einhergehenden Beschwerden; Behandlung dysfunktioneller uteriner Blutungen; Behandlung der Akne; Prävention und Behandlung kardiovaskulärer Erkrankungen; Behandlung von Hypercholesterinämie und Hyperlipidämie; Prävention und Behandlung der Artherosclerose; zur Hemmung der Proliferation der arteriellen Glattmuskelzellen; zur Behandlung des Atemnotsyndroms bei Neugeborenen; Behandlung des primären pulmonaren Bluthochdrucks; zur Vorbeugung und Behandlung der Osteoporose (Black, L.J., Sato,M.,Rowley, E.R.,Magee, D.E., Bekele, A., Williams, D.C., Cullinan, G.J., Bendele, R., Kauffman, R.F., Bensch, W.R., Frolik, C.A., Termine, J.D. and Bryant, H.U.: Raloxifene [LY 139481 HCI] prevents bone loss and reduces serum cholesterol without causing uterine hypertrophy in ovariectomized rats; J. Clin. Invest. 93: 63 - 69, 1994); zur Vorbeugung des Knochenverlusts bei postmenopausalen Frauen, bei hysterektomierten Frauen oder bei Frauen, die mit LHRH Agonisten oder Antagonisten behandelt wurden; Hemmung der Spermienreifung; Behandlung von rheumatoider Arthritis; zur Vorbeugung der Alzheimer'schen Krankheit; Behandlung der Endometriose; Behandlung von Myomen; Behandlung von Myomen und der Endometriose in Kombination mit LHRH-Analoga; Behandlung hormonabhängiger Tumoren, z.B. des Mammacarcinoms, Behandlung prostatischer Erkrankungen.

Außerdem eignen sich die erfindungsgemäßen Verbindungen aufgrund ihres pharmakologischen Profils sowohl für die männliche als auch für die weibliche Kontrazeption.

Die Verbindungen können auch in Kombination mit dem natürlichen Vitamin D3 oder mit Calcitriol-Analoga für den Knochenaufbau oder als unterstützende Therapie zu Therapien, welche einen Knochenmassenverlust verursachen (beispielsweise eine Therapie mit Glucocorticoiden, Chemotherapie) eingesetzt werden.

Schließlich können die Verbindungen der allgemeinen Formel I in Verbindung mit Progesteronrezeptor-Antagonisten oder in Verbindung mit reinen Estrogenen verwendet werden, und zwar insbesondere zur Verwendung in der Hormonersatz-Therapie und zur Behandlung gynäkologischer Störungen und für die weibliche Fertilitätskontrolle.
Ein therapeutisches Produkt, enthaltend ein Estrogen und ein reines Antiestrogen für gleichzeitige, sequentielle oder getrennte Anwendung für die selektive Estrogentherapie perimenopausaler oder postmenopausaler Zustände ist bereits in der EP-A 0 346 014 beschrieben.

Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 10 mg / kg Körpergewicht, vorzugsweise 0,1 - 5 mg / kg Körpergewicht, je Tag betragen.
Beim Menschen entspricht dies einer Dosis von 0,8 bis 800 mg, vorzugsweise 8 bis 400 mg, täglich.

Eine Dosiseinheit enthält erfindungsgemäß 0,4 bis 400 mg einer oder mehrerer Verbindungen der allgemeinen Formel I.

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P. Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. in Frage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.
Die Verbindungen lassen sich auch in Form einer Depotinjektion oder eines Implantatpräparats anwenden, die so formuliert sein können, daß eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk.

Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder Intrauterinsystemen (z.B. Pessare, Spiralen, lUSs, Mirena®) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656).

Erfindungsgemäß können die Verbindungen der allgemeinen Formel I auch mit Liposomen verkapselt werden.

Die erfindungsgemäßen Verbindungen der allgemeinen Formel I werden wie in den Beispielen beschrieben hergestellt. Durch analoge Vorgehensweise unter Verwendung homologer Reagenzien zu den in den Beispielen beschriebenen Reagenzien lassen sich alle Verbindungen der allgemeinen Formel I erhalten.

Veretherung und/oder Veresterung freier Hydroxygruppen erfolgt nach dem Fachmann gängigen Methoden.

Diejenigen Verbindungen der allgemeinen Formel I, worin A für eine direkte Bindung steht, lassen sich beispielsweise analog zu den in WO 98/07740 und DE 1 98 06 357.1 beschriebenen Verfahren erhalten. Zur Einführung der Seitenkette SK wird zunächst die 4-Hydroxygruppe des Phenylrestes in 5-Stellung des Ausgangsproduktes in eine Trifluormethylsulfonyloxygruppe überführt und dann Palladium-katalysiert eine Alkylierung am Phenylrest zur Einführung des endständig funktionalisierten Restes B vorgenommen (J. Org. Chem., 58;8; 1993, S- 2201 - 2208; Tetrahedron Lett. 28; 21; 1987, S. 2387 - 2388). Die Weiterverarbeitung zum Aufbau der vollständigen Seitenkette SK erfolgt dann wie in in WO 98/07740 oder DE 1 98 06 357.1 beschrieben.

### Beispiele:

### Beispiel 1

### (4,4,5,5,5-Pentafluorpentyl)-{5-[4-(6-phenyl-8,9-dihydro-7H-benzocyclohepten-5-yl)-phenoxy]-pentyl}-sulfid

### a) 9-[4-(5-Chlorpentyloxy)-phenyl]-8-phenyl-6,7-dihydro-5H-benzocyclohepten

Eine Suspension von 3,0 g 4-(6-Phenyl-8,9-dihydro-7H-benzocyclohepten-5-yl)-phenol [Raymond McCague, Reiko Kuroda, Guy Leclerq, Susanna Stoessel, *J. Med. Chem.* (29) 10 1986 p. 2053-2059] in 51 ml Acetonitril wird mit 1,68 g Kaliumcarbonat und 1,49 ml 1-Brom-5-chlorpentan 9 Stunden bei 100 °C gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 3,52 g 9-[4-(5-Chlorpentyloxy)-phenyl]-8-phenyl-6,7-dihydro-5H-benzocyclohepten als Kristalle vom Schmelzpunkt 118-120°C.

### b) 9-[4-(5-lodpentyloxy)-phenyl]-8-phenyl-6,7-dihydro-5H-benzocyclohepten

Eine Lösung von 3,32 g 9-[4-(5-Chlorpentyloxy)-phenyl]-8-phenyl-6,7-dihydro-5Hbenzocyclohepten in 120 ml Ethylmethylketon wird mit 4,5 g Natriumiodid 9,5 Stunden bei 100 °C Badtemperatur gerührt. Dann wird auf Wasser gegeben, dreimal mit Essigester extrahiert, neutralgewaschen, über Natriumsulfat getrocknet und i.Vak. eingeengt. Man erhält 4,02 g 9-[4-(5-lodpentyloxy)phenyl]-8-phenyl-6,7-dihydro-5Hbenzocyclohepten als Kristalle vom Schmelzpunkt 104-106°C.

### c) (4,4,5,5,5-Pentafluorpentyl)-{5-[4-(6-phenyl-8,9-dihydro-7H-benzocyclohepten-5-yl)-phenoxy]-pentyl}-sulfid

Eine Lösung von 2,35 g 4,4,5,5,5-Pentafluorpentylthioacetat in 12 ml Methanol wird mit 1,88 ml einer 30%igen, methanolischen Natriummethylatlösung bei Raumtemperatur 0,5 Stunden gerührt. Diese Lösung tropft man zu einer Suspension von 4,0 g 9-[4-(5-lodpentyloxy)-phenyl]-8-phenyl-6,7-dihydro-5H-benzocyclohepten in 44 ml Methanol und 44 ml Diethylether und rührt 6 Stunden bei Raumtemperatur.

Anschließend wird das Methanol i. Vak. eingeengt, auf Wasser gegeben, dreimal mit Essigester extrahiert, neutralgewaschen, über Natriumsulfat getrocknet und i.Vak. eingeengt. Man erhält 4,5 g (4,4,5,5,5-Pentafluorpentyl)-{5-[4-(6-phenyl-8,9-dihydro-7H-benzocyclohepten-5-yl)-phenoxy]-pentyl}-sulfid als Kristalle vom Schmelzpunkt 84-85°C.

### Beispiel 2

### (4,4,5,5,5-Pentafluorpentyl)-{5-[4-(6-phenyl-8,9-dihydro-7H-benzocyclohepten-5-yl) phenoxy]-pentyl}-sulfoxid

Eine Lösung von 4,4 g (4,4,5,5,5-Pentafluorpentyl)-{5-[4-(6-phenyl-8,9-dihydro-7Hbenzocyclohepten-5-yl)-phenoxy]-pentyl}-sulfid in 130 ml Methanol wird bei Raumtemperatur mit 7,1 ml Wasser und 1,86 g Natriumperiodat versetzt und 24 Stunden gerührt. Dann wird i.Vak. zur Trockne eingeengt, mit Dichlormethan / Wasser aufgenommen, mit Wasser gewaschen, über Natriumsulfat getrocknet und i.Vak. eingeengt und an Kieselgel mit Dichlormethan / Aceton chromatographiert. Man erhält 3,6 g (4,4,5,5,5-Pentafluorpentyl)-{5-[4-(6-phenyl-8,9-dihydro-7H-benzocyclohepten-5-yl)-phenoxy]-pentyl}-sulfoxid als farblose Kristalle vom Schmelzpunkt 114-116°C.

### Beispiel 3

### Methyl-[3-(4,4,5,5,5-pentafluorpentylthio)-propyl]-{2-[4-(6-phenyl-8,9-dihydro-7Hbenzocyclohepten-5-yl)-phenoxy]-ethyl}-amin

### a) 9-[4-(2-Chlorethoxy)-phenyl]-8-phenyl-6,7-dihydro-5H-benzocyclohepten

Eine Suspension von 3,0 g 4-(6-Phenyl-8,9-dihydro-7H-benzocyclohepten-5-yl)-phenol [Raymond McCague, Reiko Kuroda, Guy Leclerq, Susanna Stoessel, *J. Med. Chem.* (29) 10 1986 p. 2053-2059] in 51 ml Acetonitril wird mit 1,68 g Kaliumcarbonat und 0,95 ml 1-Brom-2-chlorethan 28 Stunden bei 100°C Badtemperatur gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 2,93 g 9-[4-(2-Chlorethoxy)-phenyl]-8-phenyl-6,7-dihydro-5H-benzocyclohepten als Kristalle vom Schmelzpunkt 171-172°C.

### b) 9-[4-(2-lodethoxy)-phenyl]-8-phenyl-6,7-dihydro-5H-benzocyclohepten

Eine Lösung von 2,73 g 9-[4-(2-Chlorethoxy)-phenyl]-8-phenyl-6,7-dihydro-5Hbenzocyclohepten in 110 ml Ethylmethylketon wird mit 4,11 g Natriumiodid 28 Stunden bei 100°C Badtemperatur gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 2,60 g 9-[4-(2-lodethoxy)-phenyl]-8-phenyl-6,7-dihydro-5H-benzocyclohepten als Kristalle vom Schmelzpunkt 154-156 °C.

### c) Methyl-{2-[4-(6-phenyl-8,9-dihydro-7H-benzocyclohepten-5-yl)-phenoxy]-ethyl}amin

Eine Lösung von 2,2 g 9-[4-(2-lodethoxy)-phenyl]-8-phenyl-6,7-dihydro-5Hbenzocyclohepten in 55 ml Dimethylformamid und 1,1 ml Triethylamin wird mit 4,4 ml einer 40%igen, wässrigen Methylaminlösung 1 Stunde bei 80 °C Badtemperatur gerührt. Dann wird auf gesättigte Kochsalzlösung gegeben, dreimal mit Essigester extrahiert, neutralgewaschen, über Natriumsulfat getrocknet, i.Vak. eingeengt und an Kieselgel mit Hexan / Essigester chromatographiert. Man erhält 0,9 g Methyl-{2-[4-(6-phenyl-8,9-dihydro-7H-benzocyclohepten-5-yl)-phenoxy]-ethyl}-amin als Kristalle vom Schmelzpunkt 165-167 °C.

### d) Methyl-[3-(4,4,5,5,5-pentafluorpentylthio)-propyl]-{2-[4-(6-phenyl-8,9-dihydro-7Hbenzocyclohepten-5-yl)-phenoxy]-ethyl}-amin

Eine Lösung von 0,9 g Methyl-{2-[4-(6-phenyl-8,9-dihydro-7H-benzocyclohepten-5-yl)-phenoxy]-ethyl}-amin und 0,4 g Kaliumcarbonat in 14 ml Dimethylformamid wird tropfenweise mit einer Lösung von 1,0 g 1-lod-3-(4,4,5,5,5-pentafluorpentylthio)-propan in 2 ml Dimethylformamid versetzt und 2 Stunden bei 80°C Badtemperatur gerührt. Anschließend wird auf Wasser gegeben, dreimal mit Essigester extrahiert, neutralgewaschen, über Natriumsulfat getrocknet und i.Vak. eingeengt. Man erhält 570 mg Methyl-[3-(4,4,5,5,5-pentafluorpentylthio)-propyl]-{2-[4-(6-phenyl-8,9-dihydro-7H-benzocyclohepten-5-yl)-phenoxy]-ethyl}-amin als Öl.

### Beispiel 4

### Methyl-[3-(4,4,5,5,5-pentafluorpentylsulfinyl)-propyl]-{2-[4-(6-phenyl-8,9-dihydro-7Hbenzocyclohepten-5-yl)-phenoxy]-ethyl}-amin

Analog wie im Beispiel 2 beschrieben oxidiert man 0,55 g Methyl-[3-(4,4,5,5,5-pentafluorpentylthio)-propyl]-{2-[4-(6-phenyl-8,9-dihydro-7H-benzocyclohepten-5-yl)-phenoxy]-ethyl}-amin und erhält 334 mg Methyl-[3-(4,4,5,5,5-pentafluorpentylsulfinyl)-propyl]-{2-[4-(6-phenyl-8,9-dihydro-7H-benzocyclohepten-5-yl)-phenoxy]-ethyl}-amin als Kristalle vom Schmezpunkt 74-77°C.

### Beispiel 5

### S-{5-[4-(6-Phenyl-8,9-dihydro-7H-benzocyclohepten-5-yl)-phenoxy]-pentyl}-thioacetat

Eine Lösung von 8,0 g 9-[4-(5-lodpentyloxy)-phenyl]-8-phenyl-6,7-dihydro-5Hbenzocyclohepten in 170 ml Aceton wird mit 5,36 g Kaliumthioacetat 2,5 Stunden bei Raumtemperatur gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 6,5 g S-{5-[4-(6-Phenyl-8,9-dihydro-7H-benzocyclohepten-5-yl)-phenoxy]-pentyl}-thioacetat als Kristalle vom Schmelzpunkt 118-120°C.

### Beispiel 6

### N-Butyl-N-methyl-2-{5-[4-(6-phenyl-8,9-dihydro-7H-benzocyclohepten-5-yl)-phenoxy]-pentylthio}-acetamid

Eine Lösung von 1 g S-{5-[4-(6-Phenyl-8,9-dihydro-7H-benzocyclohepten-5-yl)-phenoxy]-pentyl}-thioacetat in 15 ml Methanol und 10 ml Tetrahydrofuran wird mit 0,42 ml einer 30%igen, methanolischen Natriummethylatlösung 0,5 Stunden bei Raumtemperatur gerührt und nach Zugabe von 0,5 ml 2-Brom-N-Butyl-N-methylacetamind eine weitere Stunde gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 593 mg N-Butyl-N-methyl-2-{5-[4-(6-phenyl-8,9-dihydro-7H-benzocyclohepten-5-y!)-phenoxy]-pentylthio}-acetamid als Öl.

### Beispiel 7

### N-Butyl-N-methyl-2-{5-[4-(6-phenyl-8,9-dihydro-7H-benzocydohepten-5-yl)-phenoxy]-pentansulfinyl}-acetamid

Analog wie im Beispiel 2 beschrieben oxidiert man 0,4 g N-Butyl-N-methyl-2-{5-[4-(6-phenyl-8,9-dihydro-7H-benzocyclohepten-5-yl)-phenoxy)-pentylthio}-acetamid und erhält 301 mg N-Butyl-N-methyl-2-{5-[4-(6-phenyl-8,9-dihydro-7H-benzocyclohepten-5-yl)-phenoxy]-pentansulfinyl}-acetamid als Kristalle vom Schmezpunkt 120-121°C.

### Beispiel 8

### 5-{4-[5-(4,4,5,5,5-Pentafluor-pentylthio)-pentyloxy]-phenyl}-6-phenyl-8,9-dihydro-7Hbenzocyclohepten-2-ol

### a) 2-Benzyloxy-5-(4-methoxyphenyl)-6,7,8,9-tetrahydro-benzocyclohepten-5-ol

Eine Suspension von 14,57 g Magnesiumspänen in 150 ml Diethylether wird tropfenwiese mit einer Lösung von 88 ml 4-Bromanisol in 100 ml Diethylether so versetzt, daß die Reaktionmischung ohne externe Wärmezufuhr rückfluxiert. Nach beendeter Zugabe läßt man auf Raumtemperatur abkühlen und gibt eine Lösung von 35,2 g 2-Benzyloxy-6,7,8,9-tetrahydro-benzocyclohepten-5-on [Lal, B. et al., *J. Med. Chem.* (15) 1972 p. 23-27] in 150 ml Diethylether langsam zu und rührt weitere 4,5 Stunden bei Raumtemperatur. Dann kühlt man auf 0°C und gibt gesättigte Ammoniumchloridlösung zu. Anschließend wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 37 g 2-Benzyloxy-5-(4-methoxyphenyl)-6,7,8,9-tetrahydro-benzocyclohepten-5-ol als Kristalle vom Schmelzpunkt 112-114°C.

### b) 2-Benzyloxy-5-(4-methoxyphenyl)-8,9-dihydro-7H-benzocyclohepten

Eine Suspension von 35,8 g 2-Benzyloxy-5-(4-methoxyphenyl)-6,7,8,9-tetrahydro benzocyclohepten-5-ol in 1 I Methanol wird mit 32 ml konz. Salzsäure 3 Stunden bei Raumtemperatur gerührt. Dann wird mit Natriumhydrogencarbonatlösung neutralisiert, i. Vak. auf 300 ml eingeengt, auf Wasser gegeben, dreimal mit Essigester extrahiert, neutralgewaschen, über Natriumsulfat getrocknet und i.Vak. eingeengt. Man erhält 34 g 2-Benzyloxy-5-(4-methoxyphenyl)-8,9-dihydro-7Hbenzocyclohepten als Kristalle vom Schmelzpunkt 76-78°C.

### c) 2-Benzyloxy-6-brom-5-(4-methoxyphenyl)-8,9-dihydro-7H-benzocyclohepten

Eine Lösung von 34 g 2-Benzyloxy-5-(4-methoxyphenyl)-8,9-dihydro-7Hbenzocyclohepten in 310 ml Dichlormethan wird bei 0°C portionsweise mit 29 g Pyridiniumhydrobromidperbromid während 1 Stunde versetzt und 0,5 Stunden bei 0°C gerührt. Dann werden bei 0°C 200 ml einer wässrigen Natriumhydrogensulfitlösung zugesetzt, auf Wasser gegeben, zweimal mit Dichlormethan extrahiert, mit Natriumhydrogencarbonat und Kochsalzlösung gewaschen, über Natriumsulfat getrocknet, i.Vak. eingeengt und an Kieselgel mit Hexan / Essigester chromatographiert. Man erhält 35 g 2-Benzyloxy-6-brom-5-(4-methoxyphenyl)-8,9-dihydro-7H-benzocyclohepten als Kristalle vom Schmelzpunkt 141-143°C.

### d) 2-Benzyloxy-5-(4-methoxyphenyl)-6-phenyl-8,9-dihydro-7H-benzocyclohepten

Eine Suspension von 22.32 g Zinkchlorid in 60 ml Tetrahydrofuran wird bei Raumtemperatur mit 91,2 ml einer 1,8 M Phenyllithiumlösung innerhalb von drei Minuten versetzt und 0,5 Stunden bei 90°C Badtemperatur gerührt. Diese Lösung gibt man zu einer Lösung von 3,16 g Tetrakistriphenylphosphinpalladium(0) und 24 g 2-Benzyloxy-6-brom-5-(4-methoxyphenyl)-8,9-dihydro-7H-benzocyclohepten in 144 ml Tetrahydrofuran und rührt weitere 3 Stunden bei 90°C Badtemperatur. Dann wird auf 170 ml einer 1 M Salzsäure gegeben, dreimal mit Essigester extrahiert, über Natriumsulfat getrocknet, i.Vak. eingeengt und mit Diethylether ausgerührt. Man erhält 22,53 g 2-Benzyloxy-5-(4-methoxyphenyl)-6-phenyl-8,9-dihydro-7Hbenzocyclohepten als Kristalle vom Schmelzpunkt 163-164°C.

### e) 5-(4-Methoxyphenyl)-6-phenyl-8,9-dihydro-7H-benzocyclohepten-2-ol

Eine Lösung von 21,16 g 2-Benzyloxy-5-(4-methoxyphenyl)-6-phenyl-8,9-dihydro-7Hbenzocyclohepten in 900 ml Dichlormethan wird bei 0°C mit 19 ml N,N-Dimethylanilin 5 Minuten gerührt, portionsweise mit 26,1 g Aluminiumchlorid versetzt und weitere 3 Stunden bei 0°C gerührt. Dann wird mit 1 M Salzsäure versetzt, auf Wasser gegeben, dreimal mit Dichlormethan extrahiert, neutralgewaschen, über Natriumsulfat getrocknet, i.Vak. eingeengt und mit Hexan / Diethylether ausgerührt.
Man erhält 17,4 g 5-(4-Methoxyphenyl)-6-phenyl-8,9-dihydro-7H-benzocyclohepten-2-ol als Kristalle vom Schmelzpunkt 232-233°C.

### f) 2-[5-(4-Methoxyphenyl)-6-phenyl-8,9-dihydro-7H-benzocyclohepten-2-yloxy]-tetrahydropyran

Eine Suspension von 15,8 g 5-(4-Methoxyphenyl)-6-phenyl-8,9-dihydro-7Hbenzocyclohepten-2-ol in 150 ml Toluol wird mit 15 ml Dihydropyran und 60 mg *para*-Toluolsulfonsäure-monohydrat 18 Stunden bei Raumtemperatur gerührt. Dann wird mit Essigester verdünnt, mit Natriumhydrogencarbonat und Kochsalzlösung gewaschen, über Natriumsulfat getrocknet und i.Vak. eingeengt. Man erhält 19,7 g 2-[5-(4-Methoxyphenyl)-6-phenyl-8,9-dihydro-7H-benzocyclohepten-2-yloxy]-tetrahydropyran als Kristalle vom Schmelzpunkt 131-133°C.

### g) 4-[6-Phenyl-2-(tetrahydropyran-2-lyoxy)-8,9-dihydro-7H-benzocyclohepten-5-yl]-phenol

Eine Lösung von 19,7 g 2-[5-(4-Methoxyphenyl)-6-phenyl-8,9-dihydro-7Hbenzocyclohepten-2-yloxy]-tetrahydropyran in 500 ml Dimethylformamid wird mit 9,73 g Natriummethylthiolat 6,5 Stunden bei 140°C Badtemperatur gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 17,2 g 4-[6-Phenyl-2-(tetrahydropyran-2-lyoxy)-8,9-dihydro-7H-benzocyclohepten-5-yl]-phenol als Kristalle vom Schmelzpunkt 183-185°C.

### h) 2-{5-[4-(5-Chlorpentyloxy)-phenyl]-6-phenyl-8,9-dihydro-7H-benzocyclohepten-2-yloxy}-tetrahydropyran

Eine Lösung von 6,0 g 4-[6-Phenyl-2-(tetrahydropyran-2-lyoxy)-8,9-dihydro-7Hbenzocyclohepten-5-yl]-phenol in 77 ml Acetonitril wird mit 2,55 g Kaliumcarbonat und 2,27 ml 1-Brom-5-chlorpentan 9 Stunden bei 90°C gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 6,9 g 2-{5-[4-(5-Chlorpentyloxy)-phenyl]-6-phenyl-8,9-dihydro-7H-benzocyclohepten-2-yloxy}-tetrahydropyran als Kristalle vom Schmelzpunkt 99-101°C.

### i) 5-[4-(5-Chlorpentyloxy)-phenyl]-6-phenyl-8,9-dihydro-7H-benzocyclohepten-2-ol

Eine Lösung von 6,7 g 2-{5-[4-(5-Chlorpentyloxy)-phenyl]-6-phenyl-8,9-dihydro-7Hbenzocyclohepten-2-yloxy}-tetrahydropyran in 130 ml Methanol und 13 ml Wasser wird mit 5,7 g Oxalsäure 1 Stunde bei 100°C Badtemperatur gerührt. Dann wird i. Vak. auf 50 ml eingeengt, auf Wasser gegeben, dreimal mit Essigester extrahiert, neutralgewaschen, über Natriumsulfat getrocknet, i.Vak. zur Trockne eingeengt und aus Essigester umkristallisiert. Man erhält 5,48 g 5-[4-(5-Chlorpentyloxy)-phenyl]-6-phenyl-8,9-dihydro-7H-benzocyclohepten-2-ol als Kristalle vom Schmelzpunkt 200-202°C.

### j) 5-[4-(5-lodpentyloxy)-phenyl]-6-phenyl-8,9-dihydro-7H-benzocyclohepten-2-ol

Eine Lösung von 5,28 g 5-[4-(5-Chlorpentyloxy)-phenyl]-6-phenyl-8,9-dihydro-7Hbenzocyclohepten-2-ol in 185 ml Ethylmethylketon wird mit 6,9 g Natriumiodid 24 Stunden bei 100°C Badtemperatur gerührt. Dann wird auf Wasser gegeben, dreimal mit Essigester extrahiert, neutralgewaschen, über Natriumsulfat getrocknet, i.Vak. eingeengt und aus Aceton / Hexan kristallisiert. Man erhält 5,86 g 5-[4-(5-lodpentyloxy)-phenyl]-6-phenyl-8,9-dihydro-7H-benzocyclohepten-2-ol als Kristalle vom Schmelzpunkt 192-193°C.

### k) 5-{4-[5-(4,4,5,5,5-Pentafluor-pentylthio)-pentyloxy]-phenyl}-6-phenyl-8,9-dihydro-7H-benzocyclohepten-2-ol

Eine Lösung von 1,7 g 4,4,5,5,5-Pentafluorpentylthioacetat in 9 ml Methanol wird mit 1,36 ml einer 30%igen, methanolischen Natriummethylatlösung bei Raumtemperatur 0,5 Stunden gerührt. Diese Lösung tropft man zu einer Suspension von 3 g 5-[4-(5-lodpentyloxy)-phenyl]-6-phenyl-8,9-dihydro-7H-benzocyclohepten-2-ol in 35 ml Methanol und 35 ml Diethylether und rührt 4 Stunden bei Raumtemperatur. Anschließend wird i. Vak. auf 15 ml eingeengt, auf Wasser gegeben, dreimal mit Essigester extrahiert, neutralgewaschen, über Natriumsulfat getrocknet und i.Vak. zur Trockne eingeengt. Man erhält 3,38 g 5-{4-[5-(4,4,5,5,5-Pentafluor-pentylthio)-pentyloxy]-phenyl}-6-phenyl-8,9-dihydro-7H-benzocyclohepten-2-ol als Kristalle vom Schmelzpunkt 167-168°C.

### Beispiel 9

### 5-{4-[5-(4,4,5,5,5-Pentafluor-pentansulfinyl)-pentyloxy]-phenyl}-6-phenyl-8,9-dihydro-7H-benzocyclohepten-2-ol

Analog wie im Beispiel 2 beschrieben oxidiert man 2,38 g 5-{4-[5-(4,4,5,5,5-Pentafluor-pentylthio)-pentyloxy]-phenyl}-6-phenyl-8,9-dihydro-7H-benzocyclohepten-2-ol und erhält 2,2 g 5-{4-[5-(4,4,5,5,5-Pentafluor-pentansulfinyl)-pentyloxy]-phenyl}-6-phenyl-8,9-dihydro-7H-benzocyclohepten-2-ol als Kristalle vom Schmezpunkt 138-140°C.

### Beispiel 10

### 5-[4-(2-{Methyl-[3-(4,4,5,5,5-pentafluor-pentansulfinyl)-propyl]-amino}-ethoxy)-phenyl]-6-phenyl-8,9-dihydro-7H-benzocyclohepten-2-ol

Analog wie im Beispiel 2 beschrieben oxidiert man 0,8 g 5-[4-(2-{Methyl-[3-(4,4,5,5,5-pentafluor-pentylthio)-propyl]-amino}-ethoxy)-phenyl]-6-phenyl-8,9-dihydro-7Hbenzocyclohepten-2-ol und erhält 405 mg 5-[4-(2-{Methyl-[3-(4,4,5,5,5-pentafluorpentansulfinyl)-propyl]-amino}-ethoxy)-phenyl]-6-phenyl-8,9-dihydro-7Hbenzocyclohepten-2-ol als Kristalle vom Schmezpunkt 63-65°C.

### Beispiel 11

### 5-[4-(2-{Methyl-[3-(4,4,5,5,5-pentafluor-pentylthio)-propyl]-amino}-ethoxy)-phenyl]-6-phenyl-8,9-dihydro-7H-benzocyclohepten-2-ol

### a) 2-{5-[4-(2-Chlorethyloxy)-phenyl]-6-phenyl-8,9-dihydro-7H-benzocyclohepten-2-yloxy}-tetrahydropyran

Eine Lösung von 6,0 g 4-[6-Phenyl-2-(tetrahydropyran-2-yloxy)-8,9-dihydro-7Hbenzocyclohepten-5-yl]-phenol in 77 ml Acetonitril wird mit 2,55 g Kaliumcarbonat und 7,0 ml 1-Brom-2-chlorethan 48 Stunden bei 90°C gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 6,7 g 2-{5-[4-(2-Chlorethyloxy)-phenyl]-6-phenyl-8,9-dihydro-7H-benzocyclohepten-2-yloxy}-tetrahydropyran als Kristalle vom Schmelzpunkt 153-155°C.

### b) 5-[4-(2-Chlorethyloxy)-phenyl]-6-phenyl-8,9-dihydro-7H-benzocyclohepten-2-ol

Eine Lösung von 6,6 g 2-{5-[4-(2-Chlorethyloxy)-phenyl]-6-phenyl-8,9-dihydro-7Hbenzocyclohepten-2-yloxy}-tetrahydropyran in 130 ml Methanol und 13 ml Wasser wird mit 5,7 g Oxalsäure 1 Stunde bei 100°C Badtemperatur gerührt. Dann wird i. Vak. auf 50 ml eingeengt, auf Wasser gegeben, dreimal mit Essigester extrahiert, neutralgewaschen, über Natriumsulfat getrocknet, i.Vak. zur Trockne eingeengt und aus Essigester umkristallisiert. Man erhält 5,0 g 5-[4-(2-Chlorethyloxy)-phenyl]-6-phenyl-8,9-dihydro-7H-benzocyclohepten-2-ol als Kristalle vom Schmelzpunkt 238-240°C.

### c) 5-[4-(2-lodethyloxy)-phenyl]-6-phenyl-8,9-dihydro-7H-benzocyclohepten-2-ol

Eine Lösung von 5,0 g 5-[4-(2-Chlorethyloxy)-phenyl]-6-phenyl-8,9-dihydro-7Hbenzocyclohepten-2-ol in 190 ml Ethylmethylketon wird mit 7,24 g Natriumiodid 28 Stunden bei 100°C Badtemperatur gerührt. Dann wird auf Wasser gegeben, dreimal mit Essigester extrahiert, neutralgewaschen, über Natriumsulfat getrocknet, i.Vak. eingeengt und aus Aceton / Hexan kristallisiert. Man erhält 5,9 g 5-[4-(2-lodethyloxy)-phenyl]-6-phenyl-8,9-dihydro-7H-benzocyclohepten-2-ol als Kristalle vom Schmelzpunkt 229-231°C.

### d) 5-{4-[2-(Methylamino)-ethoxy]-phenyl}-6-phenyl-8,9-dihydro-7Hbenzocyclohepten-2-ol

Eine Lösung von 4,5 g 5-[4-(2-lodethyloxy)-phenyl]-6-phenyl-8,9-dihydro-7Hbenzocyclohepten-2-ol in 100 ml 2-Methoxyethanol wird mit 7,2 ml einer 40%igen, wässrigen Methylaminlösung 2 Stunden bei 100°C Badtemperatur gerührt. Dann wird auf gesättigte Kochsalzlösung gegeben, dreimal mit Essigester extrahiert, neutralgewaschen, über Natriumsulfat getrocknet, i.Vak. eingeengt und aus Essigester ausgerührt. Man erhält 2,7 g 5-{4-[2-(Methylamino)-ethoxy]-phenyl}-6-phenyl-8,9-dihydro-7H-benzocyclohepten-2-ol als Kristalle vom Schmelzpunkt 184-187°C.

### e) 5-[4-(2-{Methyl-[3-(4,4,5,5,5-pentafluor-pentylthio)-propyl]-amino}-ethoxy)-phenyl]-6-phenyl-8,9-dihydro-7H-benzocyclohepten-2-ol

Eine Lösung von 1,7 g 5-{4-[2-(Methylamino)-ethoxy]-phenyl}-6-phenyl-8,9-dihydro-7H-benzocyclohepten-2-ol in 34 ml 2-Methoxyethanol wird tropfenweise mit 3,4 ml 1-lod-3-(4,4,5,5,5-pentafluorpentylthio)-propan versetzt und 1 Stunden bei 140°C Badtemperatur gerührt. Anschließend wird auf Wasser gegeben, dreimal mit Essigester extrahiert, neutralgewaschen, über Natriumsulfat getrocknet, i.Vak. eingeengt und Aceton Hexan kristallisiert. Man erhält 1,1 g 5-[4-(2-{Methyl-[3-(4,4,5,5,5-pentafluorpentylthio)-propyl]-amino}-ethoxy)-phenyl]-6-phenyl-8,9-dihydro-7Hbenzocyclohepten-2-ol als Kristalle vom Schmelzpunkt 40-42°C.

### Beispiel 12

### N-Butyl-N-methyl-2-{5-[4-(2-hydroxy-6-phenyl-8,9-dihydro-7H-benzocyclohepten-5-yl )-phenoxy]-pentylthio}-acetamid

### a) 2-{5-[4-(5-lodpentyloxy)-phenyl]-6-phenyl-8,9-dihydro-7H-benzocyclohepten-2-yloxy}-tetrahydropyran

Eine Lösung von 1,25 g 2-{5-[4-(5-Chlorpentyloxy)-phenyl]-6-phenyl-8,9-dihydro-7Hbenzocyclohepten-2-yloxy}-tetrahydropyran in 40 ml Ethylmethylketon wird mit 1,38 g Natriumiodid 9 Stunden bei 100°C Badtemperatur gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 0,7 g 2-{5-[4-(5-lodpentyloxy)-phenyl]-6-phenyl-8,9-dihydro-7H-benzocyclohepten-2-yloxy}-tetrahydropyran als Kristalle vom Schmelzpunkt 109-111°C.

### b) S-{5-[4-(6-Phenyl-2-tetrahydropyranyloxy-8,9-dihydro-7H-benzocyclohepten-5-yl)-phenoxy]-pentyl}-thioacetat

Eine Lösung von 0,7 g 2-{5-[4-(5-lodpentyloxy)-phenyl]-6-phenyl-8,9-dihydro-7Hbenzocyclohepten-2-yloxy}-tetrahydropyran in 15 ml Aceton wird mit 392 mg Kaliumthioacetat 2 Stunden bei Raumtemperatur gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 550 mg S-{5-[4-(6-Phenyl-2-tetrahydropyranyloxy-8,9-dihydro-7H-benzocyclohepten-5-yl)-phenoxy]-pentyl}-thioacetat als Kristalle vom Schmelzpunkt 84-86 °C.

### c) N-Butyl-N-methyl-2-{5-[4-(6-phenyl-2-tetrahydropyranyloxy-8,9-dihydro-7Hbenzocyclohepten-5-yl)-phenoxy]-pentylthio}-acetamid

Eine Lösung von 0,5 g S-{5-[4-(6-Phenyl-2-tetrahydropyranyloxy-8,9-dihydro-7Hbenzocyclohepten-5-yl)-phenoxy]-pentyl}-thioacetat in 8 ml Methanol und 5 ml Tetrahydrofuran wird mit 0,2 ml einer 30%igen, methanolischen Natriummethylatlösung 0,5 Stunden bei Raumtemperatur gerührt und nach Zugabe von 0,25 ml 2-Brom-N-Butyl-N-methyl-acetamind eine weitere Stunde gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 450 mg N-Butyl-N-methyl-2-{5-[4-(6-phenyl-2-tetrahydropyranyloxy-8,9-dihydro-7H-benzocyclohepten-5-yl)-phenoxy]-pentylthio}acetamid als Öl.

### d) N-Butyl-N-methyl-2-{5-[4-(2-hydroxy-6-phenyl-8,9-dihydro-7H-benzocyclohepten-5-yl)-phenoxy]-pentylthio}-acetamid

Eine Lösung von 400 g N-Butyl-N-methyl-2-{5-[4-(6-phenyl-2-tetrahydropyranyloxy-8,9-dihydro-7H-benzocyclohepten-5-yl)-phenoxy]-pentylthio}-acetamid in 11 ml Methanol und 1,1 ml Wasser wird mit 0,4 g Oxalsäure 1 Stunde bei 100 °C Badtemperatur gerührt. Dann wird auf Wasser gegeben, dreimal mit Essigester extrahiert, neutralgewaschen, über Natriumsulfat getrocknet, i.Vak. zur Trockne eingeengt und aus Pentan umkristallisiert. Man erhält 360 mg N-Butyl-N-methyl-2-{5-[4-(2-hydroxy-6-phenyl-8,9-dihydro-7H-benzocyclohepten-5-yl)-phenoxy]-pentylthio}-acetamid als Kristalle vom Schmelzpunkt 112-114 °C.

### Beispiel 13

### 5-{4-[5-(4,4,5,5,5-Pentafluor-pentansulfonyl)-pentyloxy]-phenyl}-6-phenyl-8,9-dihydro-7H-benzocyclohepten-2-ol

### a) 2-{5-[4-(5-(4,4,5.5,5-Pentafluor-pentansulfonyl)-pentyloxy)-phenyl]-6-phenyl-8,9-dihydro-7H-benzocyclohepten-2-yloxy}-tetrahydropyran

Eine Suspension von 0,5 g 4-[6-Phenyl-2-(tetrahydropyran-2-lyoxy)-8,9-dihydro-7Hbenzocyclohepten-5-yl]-phenol (Beispiel 8g) in 7 ml Tetrahydrofuran wird mit 212,5 mg Kaliumcarbonat und 620 mg 1-lod-5-(4,4,5,5,5-pentafluorpentansulfonyl)-pentan 11 Sunden bei 100 °C Badtemperatur gerührt. Dann wird auf Wasser gegeben, dreimal mit Essigester extrahiert, neutralgewaschen, über Natriumsulfat getrocknet, i.Vak. eingeengt und an Kieselgel mit Hexan / Essigester chromatographiert. Man erhält 770 mg 2-{5-[4-(5-(4,4,5,5,5-Pentafluor-pentansulfonyl)-pentyloxy)-phenyl]-6-phenyl-8,9-dihydro-7H-benzocyclohepten-2-yloxy}-tetrahydropyran als Kristalle vom Schmelzpunkt 107-110 °C.

### b) 5-{4-[5-(4,4,5,5,5-Pentafluor-pentansulfonyl)-pentyloxy]-phenyl}-6-phenyl-8,9-dihydro-7H-benzocyclohepten-2-ol

Eine Lösung von 0,7 g 2-{5-[4-(5-(4,4,5,5,5-Pentafluor-pentansulfonyl)-pentyloxy)-phenyl]-6-phenyl-8,9-dihydro-7H-benzocyclohepten-2-yloxy}-tetrahydropyran in 16 ml Methanol und 1,6 ml Wasser wird mit 0,7 g Oxalsäure 1 Stunde bei 100 °C Badtemperatur gerührt. Dann wird i. Vak. auf 50 ml eingeengt, auf Wasser gegeben, dreimal mit Essigester extrahiert, neutralgewaschen, über Natriumsulfat getrocknet, i.Vak. zur Trockne eingeengt und aus Essigester umkristallisiert. Man erhält 0,46 g 5-{4-[5-(4,4,5,5,5-Pentafluor-pentansulfonyl)-pentyloxy]-phenyl}-6-phenyl-8,9-dihydro-7Hbenzocyclohepten-2-ol als Kristalle vom Schmelzpunkt 176-178 °C.

### Beispiel 14

### N-Butyl-N-methyl-2-{5-[4-(2-hydroxy-6-phenyl-8,9-dihydro-7H-benzocyclohepten-5-yl)-phenoxy]-pentansulfinyl}-acetamid

Analog wie im Beispiel 2 beschrieben oxidiert man 210 mg N-Butyl-N-methyl-2-{5-[4-(2-hydroxy-6-phenyl-8,9-dihydro-7H-benzocyclohepten-5-yl)-phenoxy]-pentylthio}acetamid (Beispiel 12) und erhält 132 mg N-Butyl-N-methyl-2-{5-[4-(2-hydroxy-6-phenyl-8,9-dihydro-7H-benzocyclohepten-5-yl)-phenoxy]-pentansulfinyl}-acetamid als Kristalle vom Schmelzpunkt 87-89 °C.

### Beispiel 15

### N-Butyl-2-[2-({2-[4-(2-hydroxy-6-phenyl-8,9-dihydro-7H-benzocyclohepten-5-yl)-phenoxy]-ethyl}-methyl-amino)-ethansulfinyl]-N-methyl-acetamid

### a) 2-{5-[4-(2-lodethyloxy)-phenyl]-6-phenyl-8,9-dihydro-7H-benzocyclohepten-2-yloxy}-tetrahydropyran

Analog wie im Beispiel 11c beschrieben iodiert man eine Lösung von 1,7 g 2-{5-[4-(2-Chlorethyloxy)-phenyl]-6-phenyl-8,9-dihydro-7H-benzocyclohepten-2-yloxy}tetrahydropyran (Beispiel 11a) und erhält 2 g 2-{5-[4-(2-lodethyloxy)-phenyl]-6-phenyl-8,9-dihydro-7H-benzocyclohepten-2-yloxy}-tetrahydropyran als Kristalle vom Schmelzpunkt 165-166 °C.

### b) 2-{5-{4-[2-(Methylamino)-ethoxy]-phenyl}-6-phenyl-8,9-dihydro-7Hbenzocyclohepten-2-yloxy}-tetrahydropyran

Eine Lösung von 2 g 2-{5-[4-(2-lodethyloxy)-phenyl]-6-phenyl-8,9-dihydro-7Hbenzocyclohepten-2-yloxy}-tetrahydropyran in 45 ml 2-Methoxyethanol wird mit 3,2 ml einer 40%igen, wässrigen Methylaminlösung 2 Stunden bei 100 °C Badtemperatur gerührt. Dann wird auf gesättigte Natriumhydrogencarbonatlösung gegeben, dreimal mit Essigester extrahiert, neutralgewaschen, über Natriumsulfat getrocknet und i.Vak. eingeengt. Man erhält 1,65 g 2-{5-{4-[2-(Methylamino)-ethoxy]-phenyl}-6-phenyl-8,9-dihydro-7H-benzocyclohepten-2-yloxy}-tetrahydropyran als Kristalle vom Schmelzpunkt 133-137 °C.

### c) N-Butyl-2-[2-({2-[4-(6-phenyl-2-tetrahydropyranyloxy-8,9-dihydro-7Hbenzocyclohepten-5-yl)-phenoxy]-ethyl}-methyl-amino)-ethansulfinyl]-N-methylacetamid

Eine Lösung von 825 mg 2-{5-{4-[2-(Methylamino)-ethoxy]-phenyl}-6-phenyl-8,9-dihydro-7H-benzocyclohepten-2-yloxy}-tetrahydropyran in 20 ml 2-Methoxyethanol wird mit 583 mg N-Butyl-2-iodethansulfinyl-N-methyl-acetamid 3,5 Stunden bei 120 °C Badtemperatur gerührt, Dann wird auf Kochsalzlösung gegeben, dreimal mit Essigester extrahiert, neutralgewaschen, über Natriumsulfat getrocknet, i.Vak. eingeengt und an Kieselgel mit Dichlormethan / Methanol chromatographiert. Man erhält 0, 72 g N-Butyl-2-[2-({2-[4-(6-phenyl-2-tetrahydropyranyloxy-8,9-dihydro-7Hbenzocyclohepten-5-yl)-phenoxy]-ethyl}-methyl-amino)-ethansulfinyl]-N-methylacetamid als Schaum.

### d) N-Butyl-2-[2-({2-[4-(2-hydroxy-6-phenyl-8,9-dihydro-7H-benzocyclohepten-5-yl)-phenoxy]-ethyl}-methyl-amino)-ethansulfinyl]-N-methyl-acetamid

Analog wie unter 11b beschrieben erhält man aus 720 mg N-Butyl-2-[2-({2-[4-(6-phenyl-2-tetrahydropyranyloxy-8,9-dihydro-7H-benzocyclohepten-5-yl)-phenoxy]-ethyl}-methyl-amino)-ethansulfinyl]-N-methyl-acetamid 245 mg N-Butyl-2-[2-({2-[4-(2-hydroxy-6-phenyl-8,9-dihydro-7H-benzocyclohepten-5-yl)-phenoxy]-ethyl}-methylamino)-ethansulfinyl]-N-methyl-acetamid als Kristalle vom Schmelzpunkt 72-74 °C.

### Beispiel 16

### N-Butyl-2-[2-({2-[4-(2-hydroxy-6-phenyl-8,9-dihydro-7H-benzocyclohepten-5-yl)-phenoxy]-ethyl}-methyl-amino)-ethansulfonyl]-N-methyl-acetamid

### a) N-Butyl-2-[2-({2-[4-(6-phenyl-2-tetrahydropyranyloxy-8,9-dihydro-7Hbenzocyclohepten-5-yl)-phenoxy]-ethyl}-methyl-amino)-ethansulfonyl]-N-methylacetamid

Eine Lösung von 825 mg 2-{5-{4-[2-(Methylamino)-ethoxy]-phenyl}-6-phenyl-8,9-dihydro-7H-benzocyclohepten-2-yloxy}-tetrahydropyran in 20 ml 2-Methoxyethanol wird mit 607 mg N-Butyl-2-iodethansulfonyl-N-methyl-acetamid 3,5 Stunden bei 120 °C Badtemperatur gerührt. Dann wird auf Kochsalzlösung gegeben, dreimal mit Essigester extrahiert, neutralgewaschen, über Natriumsulfat getrocknet, i.Vak. eingeengt und an Kieselgel mit Dichlormethan / Methanol chromatographiert. Man erhält 324 mg N-Butyl-2-[2-({2-[4-(6-phenyl-2-tetrahydropyranyloxy-8,9-dihydro-7Hbenzocyclohepten-5-yl)-phenoxy]-ethyl}-methyl-amino)-ethansulfonyl]-N-methylacetamid als Schaum.

### b) N-Butyl-2-[2-({2-[4-(2-hydroxy-6-phenyl-8,9-dihydro-7H-benzocyclohepten-5-yl)-phenoxy]-ethyl}-methyl-amino)-ethansulfonyl]-N-methyl-acetamid

Analog wie unter Beispiel 11 b beschrieben erhält man aus 300 mg N-Butyl-2-[2-({2-[4-(6-phenyl-2-tetrahydropyranyloxy-8,9-dihydro-7H-benzocyclohepten-5-yl)-phenoxy]-ethyl}-methyl-amino)-ethansulfonyl]-N-methyl-acetamid 231 mg N-Butyl-2-[2-({2-[4-(2-hydroxy-6-phenyl-8,9-dihydro-7H-benzocyclohepten-5-yl)-phenoxy]-ethyl}-methyl-amino)-ethansulfonyl]-N-methyl-acetamid als Kristalle vom Schmelzpunkt 57-60 °C.

### Beispiel 17

### 6-(4-Hydroxy-phenyl)-5-{4-[5-(4,4,5,5,5-pentafluor-pentansulfonyl)-pentyloxy]-phenyl}-8,9-dihydro-7H-benzocyclohepten-2-ol

### a) 2-Benzyloxy-6-(4-tert-butyldimethylsilyloxyphenyl)-5-(4-methoxyphenyl)-8,9-dihydro-7H-benzocyclohepten

Eine Lösung von 10 g 2-Benzyloxy-6-brom-5-(4-methoxyphenyl)-8,9-dihydro-7Hbenzocyclohepten (Beispiel 8c) in 114 ml Toluol und 57 ml Ethanol wird mit 5,81 g [4-(*tert*-Butyldimethylsilyloxy)-phenyl]-borsäure, 23 ml wässriger 2 M-Natriumcarbonatlösung und 246 mg Tetrakistriphenylphosphinpalladium(0) 1 Stunde bei +90 °C Badtemperatur gerührt. Dann wird mit Essigester verdünnt, zweimal mit Wasser und Kochsalzlösung gewaschen, über Natriumsulfat getrocknet und i.Vak. eingeengt. Man erhält 13 g 2-Benzyloxy-6-(4*-tert*-butyldimethylsilyloxyphenyl)-5-(4-methoxyphenyl)-8,9-dihydro-7H-benzocyclohepten.

### b) 4-[2-Benzyloxy-5-(4-methoxyphenyl)-8,9-dihydro-7H-benzocyclohepten-6-yl]-phenol

Eine Suspension von 13 g 2-Benzyloxy-6-(4-*tert*-butyldimethylsilyloxyphenyl)-5-(4-methoxyphenyl)-8,9-dihydro-7H-benzocyclohepten in 235 ml Methanol wird mit 7,25 ml conc. Salzsäure 7,5 Stunden bei 50 °C Badtemperatur gerührt. Dann wird mit Natriumhydrogencarbonat neutralisiert, i.Vak. eingeengt, auf Wasser gegeben, dreimal mit Essigester extrahiert, mit Kochsalzlösung gewaschen, über Natriumsulfat getrocknet, i.Vak. eingeengt und aus Methanol umkristallisiert. Man erhält 10,3 g 4-[2-Benzyloxy-5-(4-methoxyphenyl)-8,9-dihydro-7H-benzocyclohepten-6-yl]-phenol als Kristalle vom Schmelzpunkt 173-175 °C.

### c) 5-(4-Methoxyphenyl)-6-(4-hydroxyphenyl)-8,9-dihydro-7H-benzocyclohepten-2-ol

Eine Lösung von 10,3 g 4-[2-Benzyloxy-5-(4-methoxyphenyl)-8,9-dihydro-7Hbenzocyclohepten-6-yl]-phenol in 350 ml Dichlormethan wird bei 0 °C mit 8,76 ml N,NDimethylanilin 5 Minuten gerührt und mit 12,2 g Aluminiumchlorid 4,5 Stunden bei 0 °C gerührt. Dann wird bei 0 °C mit 2 M Salzsäure versetzt, auf Wasser gegeben, dreimal mit Dichlormethan extrahiert, neutralgewaschen, über Natriumsulfat getrocknet, i.Vak. eingeengt und mit Diethylether und wenig Dichlormethan ausgerührt. Man erhält 7,8 g 5-(4-Methoxyphenyl)-6-(4-hydroxyphenyl)-8,9-dihydro-7H-benzocyclohepten-2-ol als Kristalle vom Schmelzpunkt 250-252°C.

### d) 2-[5-(4-Methoxyphenyl)-6-(4-tetrahydropyranyloxyphenyl)-8,9-dihydro-7Hbenzocyclohepten-2-yloxy]-tetrahydropyran

Eine Lösung von 7,8 g 5-(4-Methoxyphenyl)-6-(4-hydroxyphenyl)-8,9-dihydro-7Hbenzocyclohepten-2-ol in 80 ml Toluol und 40 ml Tetrahydrofuran wird mit 17,4 ml Dihydropyran und 71,4 mg *para*-Toluolsulfonsäure 20 Stunden bei Raumtemperatur gerührt. Dann wird mit Essigester verdünnt, mit Natriumhydrogencarbonat und Kochsatzlösung gewaschen, über Natriumsulfat getrocknet, i.Vak. eingeengt und mit Diethylether und wenig Essigester ausgerührt. Man erhält 8,1 g 2-[5-(4-Methoxyphenyl)-6-(4-tetrahydropyranyloxyphenyl)-8,9-dihydro-7H-benzocyclohepten-2-yloxy]-tetrahydropyran als Kristalle vom Schmelzpunkt 160-161 °C.

### e) 4-[2-Tetrahydropyranyloxy-6-(4-tetrahydropyranyloxyphenyl)-8,9-dihydro-7Hbenzocyclohepten-5-yl]-phenol

Eine Lösung von 8,0 g 2-[5-(4-Methoxyphenyl)-6-(4-tetrahydropyranyloxyphenyl)-8,9-dihydro-7H-benzocyclohepten-2-yloxy]-tetrahydropyran in 160 ml Dimethylformamid wird mit 3,2 g Natriummethanthiolat 7 Stunden bei 140 °C gerührt. Dann wird auf Wasser gegeben, dreimal mit Essigester extrahiert, neutralgewaschen, über Natriumsulfat getrocknet, i.Vak. eingeengt und mit Diethylether ausgerührt. Man erhält 7,4 g 4-[2-Tetrahydropyranyloxy-6-(4-tetrahydropyranyloxyphenyl)-8,9-dihydro-7H-benzocyclohepten-5-yl]-phenol als Kristalle vom Schmelzpunkt 182-183 °C.

### f) 2-{5-[4-(5-(4,4,5,5,5-Pentafluor-pentansulfonyl)-pentyloxy)-phenyl]-6-(4-tetrahydropyranyloxyphenyl)-8,9-dihydro-7H-benzocyclohepten-2-yloxy}tetrahydropyran

Eine Suspension von 1 g -[2-Tetrahydropyranyloxy-6-(4-tetrahydropyranyloxyphenyl)-8,9-dihydro-7H-benzocyclohepten-5-yl]-phenol in 14 ml Acetonitril wird mit 342 mg Kaliumcarbonat und 1 g 1-lod-5-(4,4,5,5,5-pentafluorpentansulfonyl)-pentan 21 Stunden bei 100 °C Badtemperatur rückfluxiert. Dann wird i.Vak. eingeengt, auf Wasser gegeben, dreimal mit Essigester extrahiert, neutralgewaschen, über Natriumsulfat getrocknet, i.Vak. eingeengt und aus Diethylether umkristallisiert. Man erhält 1,6 g 2-{5-[4-(5-(4,4,5,5,5-Pentafluor-pentansulfonyl)-pentyloxy)-phenyl]-6-(4-tetrahydropyranyloxyphenyl)-8,9-dihydro-7H-benzocyclohepten-2-yloxy}tetrahydropyran als Kristalle vom Schmelzpunkt 108-110 °C.

### g) 6-(4-Hydroxy-phenyl)-5-{4-[5-(4,4,5,5,5-pentafluor-pentansulfonyl)-pentyloxy]-phenyl}-8,9-dihydro-7H-benzocyclohepten-2-ol

Eine Suspension von 1,5 g 2-{5-[4-(5-(4,4,5,5,5-Pentafluor-pentansulfonyl)-pentyloxy)-phenyl]-6-(4-tetrahydropyranyloxyphenyl)-8,9-dihydro-7Hbenzocyclohepten-2-yloxy}-tetrahydropyran in 34 ml Methanol sowie 3,4 ml Wasser wird mit 1,5 g Oxalsäure 1 Stunde bei 100°C gerührt. Dann wird auf Wasser gegeben, dreimal mit Essigester extrahiert, neutralgewaschen, über Natriumsulfat getrocknet, i.Vak. eingeengt und mit Hexan / Diethylether chromatographiert. Man erhält 924 mg 6-(4-Hydroxy-phenyl)-5-{4-[5-(4,4,5,5,5-pentafluor-pentansulfonyl)-pentyloxy]-phenyl}-8,9-dihydro-7H-benzocyclohepten-2-ol als Kristalle vom Schmelzpunkt 168-170 °C.

### Beispiel 18

### N-Butyl-2-{6-[4-(2-hydroxy-6-phenyl-8,9-dihydro-7H-benzocyclohepten-5-yl)-phenoxy]-hexansulfinyl}-N-methyl-acetamid

Analog wie im Beispiel 2 beschrieben oxidiert man 0,4 g N-Butyl-2-{6-[4-(2-hydroxy-6-phenyl-8,9-dihydro-7H-benzocyclohepten-5-yl)-phenoxy]-hexylthio}-N-methylacetamid (Beispiel 19) und erhält 394 mg N-Butyl-2-{6-[4-(2-hydroxy-6-phenyl-8,9-dihydro-7H-benzocyclohepten-5-yl)-phenoxy]-hexansulfinyl}-N-methyl-acetami als Kristalle vom Schmezpunkt 72-73 °C.

### Beispiel 19

### N-Butyl-2-{6-[4-(2-hydroxy-6-phenyl-8,9-dihydro-7H-benzocyclohepten-5-yl)-phenoxy]-hexylthio}-N-methyl-acetamid

### a) 2-{5-[4-(6-Chlorhexyloxy)-phenyl]-6-phenyl-8,9-dihydro-7H-benzocyclohepten-2-yloxy}-tetrahydropyran

Analog wie im Beispiel 8h alkyliert man 1,2 g 4-[6-Phenyl-2-(tetrahydropyran-2-lyoxy)-8,9-dihydro-7H-benzocyclohepten-5-yl]-phenol (Beispiel 8g) mit 0,52 ml 1-Brom-6-chlorhexan und erhält 1,1 g 2-{5-[4-(6-Chlorhexyloxy)-phenyl]-6-phenyl-8,9-dihydro-7H-benzocyclohepten-2-yloxy}-tetrahydropyran als Kristalle vom Schmelzpunkt 84-86 °C.

### b) 2-{5-[4-(6-lodhexyloxy)-phenyl]-6-phenyl-8,9-dihydro-7H-benzocyclohepten-2-yloxy}-tetrahydropyran

Analog wie im Beispiel 12a substituiert man 1 g 2-{5-[4-(6-Chlorhexyloxy)-phenyll-6-phenyl-8,9-dihydro-7H-benzocyclohepten-2-yloxy}-tetrahydropyran mit 1,07 g Natriumiodid und erhält 1,1 g 2-{5-[4-(6-lodhexyloxy)-phenyl]-6-phenyl-8,9-dihydro-7H-benzocyclohepten-2-yloxy}-tetrahydropyran als Kristalle vom Schmelzpunkt 108-110°C.

### c) S-{6-[4-(6-Phenyl-2-tetrahydropyranyloxy-8,9-dihydro-7H-benzocyclohepten-5-yl)-phenoxy]-hexyl}-thioacetat

Analog wie im Beispiel 12b thioacetyliert man 1 g 2-{5-[4-(6-Iodhexyloxy)-phenyl]-6-phenyl-8,9-dihydro-7H-benzocyclohepten-2-yloxy}-tetrahydropyran mit 549 mg Kaliumthioacetat und erhält 916 mg S-{6-[4-(6-Phenyl-2-tetrahydropyranyloxy-8,9-dihydro-7H-benzocyclohepten-5-yl)-phenoxy]-hexyl}-thioacetat als Kristalle vom Schmelzpunkt 98-100 °C.

### d) N-Butyl-N-methyl-2-{6-[4-(6-phenyl-2-tetrahydropyranyloxy-8,9-dihydro-7Hbenzocyclohepten-5-yl)-phenoxy]-hexylthio}-acetamid

Analog wie im Beispiel 12c setzt man 0,8 g S-{6-[4-(6-Phenyl-2-tetrahydropyranyloxy-8,9-dihydro-7H-benzocyclohepten-5-yl)-phenoxy]-hexyl}-thioacetat mit 0,31 g 2-Brom-N-Butyl-N-methyl-acetamind zu 632 mg N-Butyl-N-methyl-2-{6-[4-(6-phenyl-2-tetrahydropyranyloxy-8,9-dihydro-7H-benzocyclohepten-5-yl)-phenoxy]-hexylthio}acetamid um.

### e) N-Butyt-2-{6-[4-(2-hydroxy-6-phenyl-8,9-dihydro-7H-benzocyclohepten-5-yl)-phenoxy]-hexylthio}-N-methyl-acetamid

Analog wie im Beispiel 12d werden 0,6 g N-Butyl-N-methyl-2-{6-[4-(6-phenyl-2-tetrahydropyranyloxy-8,9-dihydro-7H-benzocyclohepten-5-yl)-phenoxy]-hexylthio}acetamid mit 0,6 g Oxalsäure zu 520 mg N-Butyl-2-{6-[4-(2-hydroxy-6-phenyl-8,9-dihydro-7H-benzocyclohepten-5-yl)-phenoxy]-hexylthio}-N-methyl-acetamid als Kristalle vom Schmelzpunkt 103-105 °C entschützt.

### Beispiel 20

### 6-(4-Hydroxy-phenyl)-5-{4-[5-(4,4,5,5,5-pentafluorpentylthio)-pentyloxy]-phenyl}-8,9-dihydro-7H-benzocyclohepten-2-ol

### a) 2-{5-[4-(5-Chlorpentyloxy)-phenyl]-6-(4-tetrahydropyranyloxyphenyl)-8,9-dihydro-7H-benzocyclohepten-2-yloxy}-tetrahydropyran

Analog wie im Beispiel 8h alkyliert man 2 g 4-[2-Tetrahydropyranyloxy-6-(4-tetrahydropyranyloxyphenyl)-8,9-dihydro-7H-benzocyclohepten-5-yl]-phenol (Beispiel 17e) mit 0,61 ml 1-Brom-5-chlorpentan und erhält 2,4 g 2-{5-[4-(5-Chlorpentyloxy)-phenyl]-6-(4-tetrahydropyranyloxyphenyl)-8,9-dihydro-7H-benzocyclohepten-2-yloxy}tetrahydropyran als Kristalle vom Schmelzpunkt 110-112 °C.

### b) 2-{5-[4-(5-lodpentyloxy)-phenyl]-6-(4-tetrahydropyranyloxyphenyl)-8,9-dihydro-7Hbenzocyclohepten-2-yloxy}-tetrahydropyran

Analog wie im Beispiel 12a substituiert man 2,3 g 2-{5-[4-(5-Chlorpentyloxy)-phenyl]-6-(4-tetrahydropyranyloxyphenyl)-8,9-dihydro-7H-benzocyclohepten-2-yloxy}tetrahydropyran mit 2,13 g Natriumiodid und erhält 2,6 g 2-{5-[4-(5-lodpentyloxy)-phenyl]-6-(4-tetrahydropyranyloxyphenyl)-8,9-dihydro-7H-benzocyclohepten-2-yloxy}tetrahydropyran.

### c) 6-(4-Hydroxy-phenyl)-5-[4-(5-iodpentyloxy)-phenyl]-8,9-dihydro-7Hbenzocyclohepten-2-ol

Analog wie im Beispiel 12d entschützt man 2,6 g 2-{5-[4-(5-lodpentyloxy)-phenyl]-6-(4-tetrahydropyranyloxyphenyl)-8,9-dihydro-7H-benzocyclohepten-2-yloxy}tetrahydropyran mit 2,6 g Oxalsäure und erhält 2 g 6-(4-Hydroxy-phenyl)-5-[4-(5-iodpentyloxy)-phenyl]-8,9-dihydro-7H-benzocyclohepten-2-ol als Kristalle vom Schmelzpunkt 202-204 °C.

### d) 6-(4-Hydroxy-phenyl)-5-{4-[5-(4,4,5,5,5-pentafluorpentylthio)-pentyloxy]-phenyl}-8,9-dihydro-7H-benzocyclohepten-2-ol

Analog wie im Beispiel 8k setzt man 1,9 g 6-(4-Hydroxy-phenyl)-5-[4-(5-iodpentyloxy)-phenyl]-8,9-dihydro-7H-benzocyclohepten-2-ol mit 1,7 g 4,4,5,5,5-Pentafluorpentylthioacetat um. Man erhält 1,4 g 6-(4-Hydroxy-phenyl)-5-{4-[5-(4,4,5,5,5-pentafluorpentylthio)-pentyloxy]-phenyl}-8,9-dihydro-7H-benzocyclohepten-2-ol als Kristalle vom Schmelzpunkt 157-158 °C.

### Beispiel 21

### 6-(4-Hydroxy-phenyl)-5-{4-[5-(4,4,5,5,5-pentafluorpentan-sulfinyl)-pentyloxy]-phenyl}-8,9-dihydro-7H-benzocyclohepten-2-ol

Analog wie im Beispiel 2 beschrieben oxidiert man 1,3 g 6-(4-Hydroxy-phenyl)-5-{4-[5-(4,4,5,5,5-pentafluorpentylthio)-pentyloxy]-phenyl}-8,9-dihydro-7Hbenzocyclohepten-2-ol und erhält 2,2 g 6-(4-Hydroxy-phenyl)-5-{4-[5-(4,4,5,5,5-pentafluorpentan-sulfinyl)-pentyloxy]-phenyl}-8,9-dihydro-7H-benzocyclohepten-2-ol als Kristalle vom Schmezpunkt 165-168 °C.

### Beispiel 22

### 5-{4-[4-(4,4,5,5,5-Pentafluor-pentylthio)-butyloxy]-phenyl}-6-phenyl-8,9-dihydro-7Hbenzocyclohepten-2-ol

### a) 2-{5-[4-(4-Chlor-butyloxy)-phenyl]-6-phenyl-8,9-dihydro-7H-benzocyclohepten-2-yloxy}-tetrahydropyran

4,8 g 4-[6-Phenyl-2-(tetrahydropyran-2-yloxy)-8,9-dihydro-7H-benzocyclohepten-5-yl]-phenol (Bsp.: 8g) werden in 50 ml Acetonitril vorgelegt, bei Raumtemperatur mit 2,04 g Kaliumcarbonat und 1,61 ml 1-Brom-4-chlorbutan versetzt und 8h bei 90 °C gerührt. Aufgrund einer unvollständigen Umsetzung werden nochmals 20% der am Anfang eingesetzten Menge an Kaliumcarbonat und 1-Brom-4-chlorbutan zugegeben und für weitere 6h bei 90 °C erwärmt. Zur Aufarbeitung wird der Ansatz im Vakuum eingeengt, mit Wasser versetzt, dreimal mit Essigester extrahiert, mit gesättigter Natriumchloridlösung gewaschen, über Magnesiumsulfat getrocknet und im Vakuum eingeengt. Der Rückstand wird an Kieselgel mit einem Hexan-Essigester-Gradienten aufgereinigt. Man erhält 5,18 g 2-{5-[4-(4-Chlor-butyloxy)-phenyl]-6-phenyl-8,9-dihydro-7H-benzocyclohepten-2-yloxy}-tetrahydropyran.

### b) 5-[4-(4-Chlor-butyloxy)-phenyl]-6-phenyl-8,9-dihydro-7H-benzocyclohepten-2-ol

5,15 g 2-{5-[4-(4-Chlor-butyloxy)-phenyl]-6-phenyl-8,9-dihydro-7H-benzocyclohepten-2-yloxy}-tetrahydropyran werden in 100 ml Methanol und 10 ml Wasser suspendiert, mit 4,47 g Oxalsäure versetzt und 75 min bei 100 °C gerührt. Das Lösungsmittel wird im Vakuum zur Hälfte abgezogen, der Ansatz in Wasser gegeben, dreimal mit Essigester extrahiert, mit gesättigter Natriumchloridlösung gewaschen, über Magnesiumsulfat getrocknet und im Vakuum eingeengt. Präparative Säulenchromatographie an Kieselgel mit einem Hexan-Essigester-Gradienten liefert 5,01 g 5-[4-(4-Chlor-butyloxy)-phenyl]-6-phenyl-8,9-dihydro-7H-benzocyclohepten-2-ol.

### c) 5-[4-(4-lod-butyloxy)-phenyl]-6-phenyl-8,9-dihydro-7H-benzocyclohepten-2-ol

2,0 g 5-[4-(4-Chlor-butyloxy)-phenyl]-6-phenyl-8,9-dihydro-7H-benzocyclohepten-2-ol werden in 72 ml Ethylmethylketon vorgelegt, mit 2,70 g Natriumiodid versetzt und 12h bei 100 °C am Rückfluß erhitzt. Die abgekühlte Reaktionsmischung wird im Vakuum eingeengt, mit Wasser versetzt und dreimal mit Essigester extrahiert. Die organische Phase wird anschließend mit Natriumthiosulfatlösung und gesättigter Natriumchloridlösung gewaschen. Trocknen über Magnesiumsulfat und Einengen im Vakuum liefert 2,14 g 5-[4-(4-lod-butyloxy)-phenyl]-6-phenyl-8,9-dihydro-7Hbenzocyclohepten-2-ol, welches ohne weitere Aufreinigung in der nächsten Stufe eingesetzt wird.

### d) 5-{4-[4-(4,4,5,5,5-Pentafluor-pentylthio)-butyloxy]-phenyl}-6-phenyl-8,9-dihydro-7H-benzocyclohepten-2-ol

Eine Lösung von 1,22 g 4,4,5,5,5-Pentafluorpentylthioacetat in 6 ml Methanol wird mit 0,98 ml einer 30%igen, methanolischen Natriummethylatlösung bei Raumtemperatur 0,5 Stunden gerührt. Diese Lösung tropft man zu einer Suspension von 2,1 g 5-[4-(4-lod-butyloxy)-phenyl]-6-phenyl-8,9-dihydro-7H-benzocyclohepten-2-ol in 25 ml Methanol und 25 ml Diethylether und rührt 4 Stunden bei Raumtemperatur.
Anschließend wird das Reaktionsgemisch i. Vak. eingeengt, auf Wasser gegeben, dreimal mit Essigester extrahiert, neutralgewaschen, über Magnesiumsulfat getrocknet und i.Vak. eingeengt. Säulenchromatographische Trennung an Kieselgel mit einem Hexan-Essigester-Gradienten liefert 1,84 g 5-{4-[4-(4,4,5,5,5-Pentafluorpentylthio)-butyloxy]-phenyl}-6-phenyl-8,9-dihydro-7H-benzocyclohepten-2-ol vom Schmp.: 167 °C.

### Beispiel 23

### 6-Phenyl-5-{4-[4-(pyridin-2-ylmethylthio)-butyloxy]-phenyl}-8,9-dihydro-7H-benzocyclohepten-2-ol

12,3 g einer 10%igen ethanolischen 2-Mercaptomethylpyridin-Lösung werden bei Raumtemperatur tropfenweise mit 1,67 ml einer 30 %igen methanolischen Natriummethylatlösung versetzt und 15 min nachgerührt. Anschließend wird die Lösung zu einer Suspension von 1,70 g 5-[4-(4-lod-butyloxy)-phenyl]-6-phenyl-8,9-dihydro-7H-benzocyclohepten-2-ol (Bsp.: 22c) in 16 ml Methanol zugetropft und für 2h bei 80°C Badtemperatur unter Rückfluß erhitzt. Das abgekühlte Reaktionsgemisch wird vom Lösungsmittel befreit, der Rückstand mit halbgesättigter Natriumchloridlösung versetzt, dreimal mit Essigester extrahiert, mit gesättigter Natriumchloridlösung gewaschen, über Magnesiumsulfat getrocknet und im Vakuum eingeengt. Säulenchromatographische Reinigung an Kieselgel mit einem Hexan-Essigester-Gradienten liefert 1,37 g 6-Phenyl-5-{4-[4-(pyridin-2-ylmethylthio)-butyloxy]-phenyl}-8,9-dihydro-7H-benzocyclohepten-2-ol vom Schmp.: 155 °C.

### Beispiel 24

### 6-Phenyl-5-{4-[5-(pyridin-2-ylmethylthio)-pentyloxy]-phenyl}-8,9-dihydro-7Hbenzocyclohepten-2-ol

21,4 g einer 10 %igen ethanolischen 2-Mercaptomethylpyridin-Lösung werden bei Raumtem-peratur tropfenweise mit 2,91 ml einer 30 %igen methanolischen Natriummethylatlösung versetzt und 15 min nachgerührt. Anschließend wird die Lösung zu einer Suspension aus 2,52 g 5-[4-(5-Chlorpentyloxy)-phenyl]-6-phenyl-8,9-dihydro-7H-benzocyclohepten-2-ol (Bsp.: 8i) und 1,03 g Natriumiodid in 29 ml Methanol getropft und für 2h bei 80 °C Badtemperatur unter Rückfluß gekocht. Zur Aufarbeitung läßt man das Reaktionsgemisch auf Raumtemperatur kommen und entfernt das Lösungsmittel im Vakuum. Der Rückstand wird mit halbgesättigter Natriumchloridlösung versetzt, dreimal mit Essigester extrahiert, mit gesättigter Natriumchloridlösung gewaschen, über Magnesiumsulfat getrocknet und im Vakuum eingeengt. Umkristallisation aus Essigester führt zu 2,15 g 6-Phenyl-5-{4-[5-(pyridin-2-ylmethylthio)-pentyloxy]-phenyl}-8,9-dihydro-7H-benzocyclohepten-2-ol vom Schmp.: 159°C.

### Beispiel 25

### 6-Phenyl-5-{4-[6-(pyridin-2-ylmethylthio)-hexyloxy]-phenyl}-8,9-dihydro-7Hbenzocyclohepten-2-ol

16,8 g einer 10 %igen ethanolischen 2-Mercaptomethylpyridin-Lösung werden bei Raumtemperatur tropfenweise mit 2,30 ml einer 30 %igen methanolischen Natriummethylatlösung versetzt und 15 min nachgerührt. Anschließend wird die Lösung zu einer Suspension aus 2,05 g 5-[4-(6-Chlorhexyloxy)-phenyl]-6-phenyl-8,9-dihydro-7H-benzocyclohepten-2-ol (Bsp.: 28a) und 812 mg Natriumiodid in 22 ml Methanol getropft und für 2h bei 80 °C Badtemperatur unter Rückfluß gekocht. Zur Aufarbeitung läßt man das Reaktionsgemisch auf Raumtemperatur kommen und entfernt das Lösungsmittel im Vakuum. Der Rückstand wird mit halbgesättigter Natriumchloridlösung versetzt, dreimal mit Essigester extrahiert, mit gesättigter Kochsalzlösung gewaschen, über Magnesiumsulfat getrocknet und im Vakuum eingeengt. Umkristallisation aus Essigester führt zu 1,63 g 6-Phenyl-5-{4-[6-(pyridin-2-ylmethylthio}-hexyloxy]-phenyl}-8,9-dihydro-7H-benzocyclohepten-2-ol vom Schmp.: 127 °C.

### Beispiel 26

### 5-{4-[4-(4,4,5,5,5-Pentafluor-pentansulfinyl)-butyloxy]-phenyl}-6-phenyl-8,9-dihydro-7H-benzocyclohepten-2-ol

1,41 g 5-{4-[4-(4,4,5,5,5-Pentafluor-pentylthio)-butyloxy]-phenyl}-6-phenyl-8,9-dihydro-7H-benzocyclohepten-2-ol (Bsp.: 22d) werden in 40 ml Methanol und 2,32 ml Wasser suspendiert, mit 550 mg Natriumperiodat versetzt und 12 Stunden bei Raumtemperatur gerührt. Anschließend wird das Methanol im Vakuum abgezogen, der Rückstand in Wasser gegeben, dreimal mit Essigester extrahiert, mit gesättigter Natriumchloridlösung gewaschen, über Magnesiumsulfat getrocknet und im Vakuum eingeengt. Verreiben des Rohprodukts mit Hexan führt zu 1,39 g 5-{4-[4-(4,4,5,5,5-Pentafluor-pentansulfinyl)-butyloxy]-phenyl}-6-phenyl-8,9-dihydro-7Hbenzocyclohepten-2-ol vom Schmp.: 155 °C.

### Beispiel 27

### 6-Phenyl-5-{4-[4-(pyridin-2-ylmethansulfinyl)-butyloxy]-phenyl}-8,9-dihydro-7Hbenzocyclohepten-2-ol

990 mg 6-Phenyl-5-{4-[4-(pyridin-2-ylmethylthio)-butyloxy]-phenyl}-8,9-dihydro-7Hbenzocyclohepten-2-ol (Bsp.: 23) werden in 3,2 ml Methanol und 1,85 ml Wasser suspendiert, mit 440 mg Natriumperiodat versetzt und über Nacht bei Raumtemperatur gerührt. Tagsdarauf wird der Ansatz für 75 min auf 50 °C erwärmt. Zur Aufarbeitung wird das Lösungsmittel im Vakuum entfernt, der Rückstand mit Wasser versetzt, dreimal mit Essigester extrahiert, mit gesättigter Natriumchloridlösung gewaschen, über Magnesiumsulfat getrocknet und im Vakuum eingeengt. Reinigung des Rohprodukts an Kieselgel mit einem Methylenchlorid-Methanol-Gradienten liefert 612 mg 6-Phenyl-5-{4-[4-(pyridin-2-ylmethansulfinyl)-butyloxy]-phenyl}-8,9-dihydro-7H-benzocyclohepten-2-ol vom Schmp.: 162 °C.

### Beispiel 28

### 5-{4-[6-(4,4,5,5,5-Pentafluor-pentylthio)-hexyloxy]-phenyl}-6-phenyl-8,9-dihydro-7Hbenzocyclohepten-2-ol

### a) 5-[4-(6-Chlor-hexyloxy)-phenyl]-6-phenyl-8,9-dihydro-7H-benzocyclohepten-2-ol

5,42 g 2-{5-[4-(6-Chlor-hexyloxy)-phenyl]-6-phenyl-8,9-dihydro-7H-benzocyclohepten-2-yloxy}-tetrahydropyran (Bsp.: 19a) werden in 100 ml Tetrahydrofuran und 10 ml Wasser gelöst, mit 4,64 g Oxalsäure versetzt und 75 min bei 100 °C Badtemperatur gerührt. Anschließend kühlt man das Reaktionsgemisch auf Raumtemperatur ab und entfernt das Lösungsmittel im Vakuum. Der Rückstand wird mit Wasser versetzt, dreimal mit Essigester extrahiert, neutral gewaschen, über Magnesiumsulfat getrocknet und im Vakuum eingeengt. Säulenchromatographische Trennung an Kieselgel mit einem Hexan-Essigester-Gradienten führt zu 4,17 g 5-[4-(6-Chlor-hexyloxy)-phenyl]-6-phenyl-8,9-dihydro-7H-benzocyclohepten-2-ol vom Schmp.: 193°C.

### b) 5-[4-(6-lod-hexyloxy)-phenyl]-6-phenyl-8,9-dihydro-7H-benzocyclohepten-2-ol

2,05 g 5-[4-(6-Chlor-hexyloxy)-phenyl]-6-phenyl-8,9-dihydro-7H-benzocyclohepten-2-ol werden in 70 ml Ethylmethylketon vorgelegt, mit 2,60 g Natriumiodid versetzt und 12h bei 100 °C Badtemperatur gerührt. Die abgekühlte Reaktionsmischung wird im Vakuum eingeengt, mit Wasser versetzt und dreimal mit Essigester extrahiert. Die organische Phase wird anschließend mit Natriumthiosulfatlösung und gesättigter Natriumchloridlösung gewaschen. Trocknen über Magnesiumsulfat und Einengen im Vakuum liefert 2,50 g 5-[4-(6-lod-hexyloxy)-phenyl]-6-phenyl-8,9-dihydro-7Hbenzocyclohepten-2-ol, welches ohne weitere Aufreinigung in der nächsten Stufe eingesetzt wird.

### c) 5-{4-[6-(4,4,5,5,5-Pentafluor-pentylthio)-hexyloxy]-phenyl}-6-phenyl-8,9-dihydro-7H-benzocyclohepten-2-ol

Eine Lösung von 1,37 g 4,4,5,5,5-Pentafluorpentylthioacetat in 6 ml Methanol wird mit 1,1 ml einer 30%igen, methanolischen Natriummethylatlösung bei Raumtemperatur 0,5 Stunden gerührt. Diese Lösung tropft man zu einer Suspension von 2,48 g 5-[4-(6-lod-hexyloxy)-phenyl]-6-phenyl-8,9-dihydro-7H-benzocyclohepten-2-ol in 25 ml Methanol und 25 ml Diethylether und rührt 4 Stunden bei Raumtemperatur. Anschließend wird der Ansatz i. Vak. eingeengt, auf Wasser gegeben, dreimal mit Essigester extrahiert, neutralgewaschen, über Magnesiumsulfat getrocknet und i.Vak. eingeengt. Umkristallisation des Rohprodukts aus Hexan/Diethylether liefert 2,44 g 5-{4-[6-(4,4,5,5,5-Pentafluor-pentylthio)-hexyloxy]-phenyl}-6-phenyl-8,9-dihydro-7H-benzocyclohepten-2-ol vom Schmp.: 166 °C.

### Beispiel 29

### 5-{4-[6-(4,4,5,5,5-Pentafluor-pentansulfinyl)-hexyloxy]-phenyl}-6-phenyl-8,9-dihydro-7H-benzocyclohepten-2-ol

2,1 g 5-{4-[6-(4,4,5,5,5-Pentafluor-pentylthio)-hexyloxy]-phenyl}-6-phenyl-8,9-dihydro-7H-benzocyclohepten-2-ol (Bsp.: 28c) werden in 56 ml Methanol und 3,3 ml Wasser vorgelegt, mit 782 mg Natriumperiodat versetzt und 12 Stunden bei Raumtemperatur gerührt. Anschließend wird das Methanol im Vakuum abgezogen, der Rückstand in Wasser gegeben, dreimal mit Essigester extrahiert, mit gesättigter Natriumchloridlösung gewaschen, über Magnesiumsulfat getrocknet und im Vakuum eingeengt. Man erhält 2,13 g 5-{4-[6-(4,4,5,5,5-Pentafluor-pentansulfinyl)-hexyloxy]-phenyl}-6-phenyl-8,9-dihydro-7H-benzocyclohepten-2-ol vom Schmp.: 121 °C.

### Beispiel 30

### 6-Phenyl-5-{4-[6-(pyridin-2-ylmethansulfinyl)-hexyloxy]-phenyl}-8,9-dihydro-7Hbenzocyclohepten-2-ol

1,30 g 6-Phenyl-5-{4-[6-(pyridin-2-ylmethylthio)-hexyloxy]-phenyl}-8,9-dihydro-7Hbenzocyclohepten-2-ol (Bsp.: 25) werden in 40 ml Methanol und 2,3 ml Wasser vorgelegt, mit 550 mg Natriumperiodat versetzt und über Nacht bei Raumtemperatur gerührt. Tagsdarauf wird für 6h auf 40 °C erwärmt und nochmals über Nacht bei Raumtemperatur gerührt. Anschließend wird das Methanol im Vakuum abgezogen, der Rückstand mit Wasser versetzt, dreimal mit Essigester extrahiert, mit gesättigter Natriumchloridlösung gewaschen, über Magnesiumsulfat getrocknet und im Vakuum eingeengt. Säulenchromatographische Trennung an Kieselgel mit einem Methylenchlorid-Methanol-Gradienten liefert 464 mg 6-Phenyl-5-{4-[6-(pyridin-2-ylmethansulfinyl)-hexyloxy]-phenyl}-8,9-dihydro-7H-benzocyclohepten-2-ol vom Schmp.: 80 °C.

### Beispiel 31

### 6-Phenyl-5-{4-[5-(pyridin-2-ylmethansulfinyl)-pentyloxy]-phenyl}-8,9-dihydro-7Hbenzocyclohepten-2-ol

1,86 g 6-Phenyl-5-{4-[5-(pyridin-2-ylmethylthio)-pentyloxy]-phenyl}-8,9-dihydro-7Hbenzocyclohepten-2-ol (Bsp.: 24) werden in 58 ml Methanol und 3,4 ml Wasser vorgelegt, mit 805 mg Natriumperiodat versetzt, über Nacht bei Raumtemperatur und tagsdarauf 6h bei 40°C gerührt. Anschließend wird das Methanol im Vakuum abgezogen, der Rückstand mit Wasser versetzt, dreimal mit Essigester extrahiert, mit gesättigter Natriumchloridlösung gewaschen, über Magnesiumsulfat getrocknet und im Vakuum eingeengt. Man erhält 2,97 g Rohprodukt, welches säulenchromatographisch mit einem Methylenchlorid/Methanol-Gradienten gereinigt wird. Umkristallisation aus Methylenchlorid/Ether liefert 635 mg 6-Phenyl-5-{4-[5-(pyridin-2-ylmethansulfinyl)-pentyloxy]-phenyl}-8,9-dihydro-7H-benzocyclohepten-2-ol vom Schmp.: 176 °C.

### Beispiel 32

### 5-{4-[5-(Furan-2-ylmethylthio)-pentyloxy]-phenyl}-6-phenyl-8,9-dihydro-7Hbenzocyclohepten-2-ol

0,6 ml Furfurylmercaptan in 2 ml Methanol wird bei Raumtemperatur tropfenweise mit 1,1 ml einer 30 %igen methanolischen Natriummethylatlösung versetzt und 15 min nachgerührt. Anschließend wird die Lösung zu einer Suspension aus 1,08 g 5-[4-(5-lodpentyloxy)-phenyl]-6-phenyl-8,9-dihydro-7H-benzocyclohepten-2-ol (Bsp.: 8j) in 10 ml Methanol getropft und für 2h auf 80°C Badtemperatur erwärmt. Zur Aufarbeitung läßt man das Reaktionsgemisch auf Raumtemperatur kommen, rührt in halbgesättigte Natriumchloridlösung ein, extrahiert dreimal mit Essigester, wäscht mit gesättigter Natriumchloridlösung, trocknet über Magnesiumsulfat und engt im Vakuum ein. Umkristallisation aus Hexan führt zu 920 mg 5-{4-[5-(Furan-2-ylmethylthio)-pentyloxy]-phenyl}-6-phenyl-8,9-dihydro-7H-benzocyclohepten-2-ol vom Schmp.: 171°C.

### Beispiel 33

### 6-Phenyl-5-{4-[5-(thien-2-ylmethylthio)-pentyloxy]-phenyl}-8,9-dihydro-7Hbenzocyclohepten-2-ol

0,24 ml 2-Thienylmethylmercaptan werden in 1 ml Methanol gelöst und bei Raumtemperatur mit 0,5 ml 30%iger methanolischer Natriummethylatlösung tropfenweise versetzt. Es wird noch 15 min nachgerührt, bevor diese Lösung zu einer Suspension von 524 mg 5-[4-(5-Iodpentyloxy)-phenyl]-6-phenyl-8,9-dihydro-7Hbenzocyclohepten-2-ol (Bsp.: 8j) in 5 ml Methanol hinzugefügt und für 2h auf 80 °C Badtemperatur erhitzt wird. Das auf Raumtemperatur abgekühlte Reaktionsgemisch wird in halbgesättigte Natriumchloridlösung gegeben, dreimal mit Essigester extrahiert, über Magnesiumsulfat getrocknet und im Vakuum eingeengt. Umkristallisation aus Hexan liefert 451 mg 6-Phenyl-5-{4-[5-(thien-2-ylmethylthio)-pentyloxy]-phenyl}-8,9-dihydro-7H-benzocyclohepten-2-ol vom Schmp.: 178 °C.

### Beispiel 34 und 35

### 5-{4-[5-(Furan-2-ylmethansulfinyl)-pentyloxy]-phenyl}-6-phenyl-8,9-dihydro-7Hbenzocyclohepten-2-ol und 5-{4-[5-(Furan-2-ylmethansulfonyl)-pentyloxy]-phenyl}-6-phenyl-8,9-dihydro-7Hbenzocyclohepten-2-ol

510 mg 5-{4-[5-(Furan-2-ylmethylthio)-pentyloxy]-phenyl}-6-phenyl-8,9-dihydro-7Hbenzocyclohepten-2-ol (Bsp.: 32) werden in 16 ml Methanol und 0,95 ml Wasser gelöst, mit 225 mg Natriumperiodat versetzt und über Nacht bei Raumtemperatur gerührt. Tagsdarauf werden nochmals 55 mg Natriumperiodat hinzugefügt und 1h bei 50°C gerührt. Das abgekühlte Reaktionsgemisch wird in halbgesättigte Natriumchloridlösung gegeben, dreimal mit Essigester extrahiert, mit gesättigter Natriumchloridlösung gewaschen, über Natriumsulfat getrocknet und im Vakuum eingeengt. Säulenchromatographische Reinigung an Kieselgel mit einem Hexan-Essigester-Gradienten führt zu 498 mg 5-{4-[5-(Furan-2-ylmethansulfinyl)-pentyloxy]-phenyl}-6-phenyl-8,9-dihydro-7H-benzocyclohepten-2-ol vom Schmp.: 168 °C und 16 mg 5-{4-[5-(Furan-2-ylmethansulfonyl}-pentyloxy]-phenyl}-6-phenyl-8,9-dihydro-7Hbenzocyclohepten-2-ol vom Schmp.: 186 °C.

### Beispiel 36 und 37

### 6-Phenyl-5-{4-[5-(thien-2-ylmethansulfonyl)-pentyloxy]-phenyl}-8,9-dihydro-7Hbenzocyclohepten-2-ol und 6-Phenyl-5-{4-[5-(thien-2-ylmethansulfinyl)-pentyloxy]-phenyl}-8,9-dihydro-7Hbenzocyclohepten-2-ol

386 mg 6-Phenyl-5-{4-[5-(thien-2-ylmethylthio)-pentyloxy]-phenyl}-8,9-dihydro-7Hbenzocyclohepten-2-ol (Bsp.: 33) werden analog zu Beispiel 34/35 umgesetzt. Nach chromatographischer Reinigung an Kieselgel mit einem Hexan-Essigester-Gradienten erhält man 28 mg 6-Phenyl-5-{4-[5-(thien-2-ylmethansulfonyl)-pentyloxy)-phenyl}-8,9-dihydro-7H-benzocyclohepten-2-ol vom Schmp.: 212°C und 312 mg 6-Phenyl-5-{4-[5-(thien-2-ylmethansulfinyl)-pentyloxy]-phenyl}-8,9-dihydro-7Hbenzocyclohepten-2-ol vom Schmp.: 174-177 °C.

### Beispiel 38

### 5-{4-[5-(3,3,4,4,5,5,5-Heptafluor-pentylthio)-pentyloxy)-phenyl}-6-phenyl-8,9-dihydro-7H-benzocyclohepten-2-ol

### a) (3,3,4,4,5,5,5-Heptafluor-pentyl)-(5-{4-[6-phenyl-2-(tetrahydropyran-2-yloxy)-8,9-dihydro-7H-benzocyclohepten-5-yl]-phenoxy}-pentyl)-sulfid

404 mg S-(5-{4-[6-Phenyl-2-(tetrahydropyran-2-yloxy)-8,9-dihydro-7H-benzocyclohepten-5-yl]-phenoxy}-pentyl)-thioacetat (Bsp.: 12b) in 1 ml Methanol wird bei Raumtemperatur tropfenweise mit 0,14 ml 30%iger methanolischer Natriummethylatlösung versetzt. Es wird noch 30 min bei Raumtemperatur nachgerührt, bevor 187 mg Heptafluor-5-iodpentan in 4 ml Methanol zugetropft werden. Nach 90 min Rühren bei Raumtemperatur wird der Ansatz in halbgesättigte Natriumchloridlösung gegeben, dreimal mit Essigester extrahiert, über Magnesiumsulfat getrocknet und im Vakuum eingeengt. Säulenchromatographische Reinigung des Rückstands an Kieselgel mit einem Hexan-Essigester-Gradienten liefert 120 mg (3,3,4,4,5,5,5-Heptafluor-pentyl)-(5-{4-[6-phenyl-2-(tetrahydropyran-2-yloxy)-8,9-dihydro-7H-benzocyclohepten-5-yl]-phenoxy}-pentyl)-sulfid.

### b) 5-{4-[5-(3,3,4,4,5,5,5-Heptafluor-pentylthio)-pentyloxy]-phenyl)-6-phenyl-8,9-dihydro-7H-benzocyclohepten-2-ol

110 mg (3,3,4,4,5,5,5-Heptafluor-pentyl)-(5-{4-[6-phenyl-2-(tetrahydropyran-2-yloxy)-8,9-dihydro-7H-benzocyclohepten-5-yl]-phenoxy}-pentyl)-sulfid werden in 2,5 ml Methanol und 0,25 ml Wasser gelöst, mit 100 mg Oxalsäure versetzt und 2h bei 50 °C gerührt. Das erkaltete Reaktionsgemisch wird mit ca. 5 ml Wasser versetzt und 30 min gerührt. Der ausgefallene Niederschlag wird abgesaugt, gut nachgewaschen und im Vakuum getrocknet. Man erhält 86 mg 5-{4-[5-(3,3,4,4,5,5,5-Heptafluorpentylthio)-pentyloxy]-phenyl}-6-phenyl-8,9-dihydro-7H-benzocyclohepten-2-ol vom Schmp. 156°C.

### Beispiel 39

### 5-{4-[2-(2-Hydroxy-ethylamino)-ethoxy]-phenyl}-6-phenyl-8,9-dihydro-7Hbenzocyclohepten-2-ol

Eine Lösung von 1 g 5-[4-(2-lodethyloxy)-phenyl]-6-phenyl-8,9-dihydro-7Hbenzocyclohepten-2-ol (Beispiel 11c) in 10 ml Methanol wird mit 1,24 ml 2-Aminoethanol 0,5 Stunden bei 160 °C Badtemperatur refluxiert. Dann wird auf Natriumhydrogencarbonatlösung gegeben, dreimal mit Essigester/Methanol (4/1) extrahiert, neutralgewaschen, über Natriumsulfat getrocknet, i.Vak. eingeengt und aus Essigester/Methanol umkristallisiert. Man erhält 621 mg 5-{4-[2-(2-Hydroxyethylamino)-ethoxy]-phenyl}-6-phenyl-8,9-dihydro-7H-benzocyclohepten-2-ol als Kristalle vom Schmelzpunkt 176-178 °C.

Ausgangsmaterial
1-lod-3-(4,4,5,5,5-pentafluorpentylthio)-propan
1-lod-5-(4,4,5,5,5-pentafluorpentansulfinyl)-pentan
1-lod-5-(4,4,5,5,5-pentafluorpentansulfonyl)-pentan
N-Butyl-2-iodethansulfinyl-N-methyl-acetamid

### Beispiel 40

### 5-{4-[5-(4-Fluor-butylthio)-pentyloxy]-phenyl}-6-phenyl-8,9-dihydro-7Hbenzocyclohepten-2-ol

### a) (4-Fluor-butyl)-(5-{4-[6-phenyl-2-(tetrahydropyran-2-yloxy)-8,9-dihydro-7Hbenzocyclohepten-5-yl]-phenoxy}-pentyl)-sulfid

500 mg S-(5-{4-[6-Phenyl-2-(tetrahydropyran-2-yloxy)-8,9-dihydro-7H-benzocyclohepten-5-yl]-phenoxy}-pentyl)-thioacetat (Bsp.: 12b) werden in 5 ml Tetrahydrofuran und 7,4 ml Methanol gelöst und bei Raumtemperatur tropfenweise mit 0,2 ml einer 30%igen methanolischen Natriummethylatlösung versetzt. Nach beendeter Zugabe wird noch 30 min bei Raumtemperatur gerührt, bevor 0,15 ml 1-Brom-4-fluorbutan bei der gleichen Temperatur zugetropft werden. Nach 90 min wird das Reaktionsgemisch mit halbgesättigter Natriumchloridlösung versetzt, dreimal mit Essigester extrahiert, neutral gewaschen, über Magnesiumsulfat getrocknet und im Vakuum eingeengt. Säulenchromatographische Reinigung an Kieselgel mit einem Hexan-Essigester-Gradienten liefert 344 mg (4-Fluor-butyl)-(5-{4-[6-phenyl-2-(tetrahydropyran-2-yloxy)-8,9-dihydro-7H-benzocyclohepten-5-yl]-phenoxy}-pentyl)-sulfid als Schaum.

### b) 5-{4-[5-(4-Fluor-butylthio)-pentyloxy]-phenyl}-6-phenyl-8,9-dihydro-7Hbenzocyclohepten-2-ol

336 mg (4-Fluor-butyl)-(5-{4-[6-phenyl-2-(tetrahydropyran-2-yloxy)-8,9-dihydro-7Hbenzocyclohepten-5-yl]-phenoxy}-pentyl)-sulfid werden in 9 ml Methanol, 10 ml Tetrahydrofuran und 0,92 ml Wasser gelöst, mit 368 mg Oxalsäure versetzt und bei 50 °C gerührt. Nach 2 Stunden erfolgt die weitere Zugabe von 184 mg Oxalsäure. Nach 24 Stunden wird das Reaktionsgemisch auf Raumtemperatur abgekühlt, mit 25 ml Wasser versetzt und 30 min gerührt. Der ausgefallene Niederschlag wird abgesaugt und getrocknet. Man erhält 275 mg 5-{4-[5-(4-Fluor-butylthio)-pentyloxy]-phenyl}-6-phenyl-8,9-dihydro-7H-benzocyclohepten-2-ol vom Schmp.: 163 °C.

### Beispiel 41

### 5-{4-[5-(4-Fluor-butansulfinyl)-pentyloxy]-phenyl}-6-phenyl-8,9-dihydro-7Hbenzocyclohepten-2-ol

181 mg 5-{4-[5-(4-Fluor-butylthio)-pentyloxy]-phenyl}-6-phenyl-8,9-dihydro-7H-benzocyclohepten-2-ol (Bsp.: 40b) werden in 6 ml Methanol und 0,5 ml Wasser gelöst, mit 81 mg Natriumperiodat versetzt und 2 Stunden bei 50 °C gerührt. Zur Aufarbeitung wird das Reaktionsgemisch mit verdünnter Natriumchloridlösung versetzt, dreimal mit Methylenchlorid extrahiert, mit Wasser gewaschen, über Magnesiumsulfat getrocknet und im Vakuum eingeengt. Der Rückstand wird mit 10 ml Ether ausgerührt, der Feststoff abgesaugt und getrocknet. Man erhält 173 mg 5-{4-[5-(4-Fluorbutansulfinyl)-pentyloxy]-phenyl}-6-phenyl-8,9-dihydro-7H-benzocyclohepten-2-ol vom Schmp.: 233 °C.

### Beispiel 42

### 6-Phenyl-5-{4-[5-(4,4,4-trifluor-butylthio)-pentyloxy]-phenyl}-8,9-dihydro-7Hbenzocyclohepten-2-ol

### a) (5-{4-[6-Phenyl-2-(tetrahydropyran-2-yloxy)-8,9-dihydro-7H-benzocyclohepten-5-yl]-phenoxy}-pentyl)-(4,4,4-trifluor-butyl)-sulfid

500 mg S-(5-{4-[6-Phenyl-2-(tetrahydropyran-2-yloxy)-8,9-dihydro-7H-benzocyclohepten-5-yl]-phenoxy}-pentyl)-thioacetat (Bsp.: 12b) werden in 5 ml Tetrahydrofuran und 7,4 ml Methanol gelöst und bei Raumtemperatur tropfenweise mit 0,2 ml einer 30%igen methanolischen Natriummethylatlösung versetzt. Nach beendeter Zugabe wird noch 30 min bei Raumtemperatur gerührt, bevor 325 mg 4,4,4-Trifluor-1-iodbutan bei der gleichen Temperatur hinzugefügt werden. Nach 90 min wird das Reaktionsgemisch mit halbgesättigter Natriumchloridlösung versetzt, dreimal mit Essigester extrahiert, neutral gewaschen, über Magnesiumsulfat getrocknet und im Vakuum eingeengt. Säulenchromatographische Reinigung an Kieselgel mit einem Hexan-Essigester-Gradienten liefert 314 mg (5-{4-[6-Phenyl-2-(tetrahydropyran-2-yloxy)-8,9-dihydro-7H-benzocyclohepten-5-yl]-phenoxy}-pentyl)-(4,4,4-trifluor-butyl)-sulfid als Schaum.

### b) 6-Phenyl-5-{4-[5-(4,4,4-trifluor-butylthio)-pentyloxy]-phenyl}-8,9-dihydro-7Hbenzocyclohepten-2-ol

310 mg (5-{4-[6-Phenyl-2-(tetrahydropyran-2-yloxy)-8,9-dihydro-7H-benzocyclohepten-5-yl]-phenoxy}-pentyl)-(4,4,4-trifluor-butyl)-sulfid werden in 8 ml Methanol, 7 ml Tetrahydrofuran und 0,8 ml Wasser gelöst, mit 319 mg Oxalsäure versetzt und 3 Stunden bei 50 °C gerührt. Anschließend wird das Reaktionsgemisch auf Raumtemperatur abgekühlt, mit 25 ml Wasser versetzt und 30 min gerührt. Der ausgefallene Niederschlag wird abgesaugt und getrocknet. Man erhält 243 mg 6-Phenyl-5-{4-[5-(4,4,4-trifluor-butylthio)-pentyloxy]-phenyl}-8,9-dihydro-7Hbenzocyclohepten-2-ol vom Schmp.: 168 °C.

### Beispiel 43

### 6-Phenyl-5-{4-[5-(4,4,4-trifluor-butansulfinyl)-pentyloxy]-phenyl}-8,9-dihydro-7Hbenzocyclohepten-2-ol

109 mg 6-Phenyl-5-{4-[5-(4,4,4-trifluor-butylthio)-pentyloxy]-phenyl}-8,9-dihydro-7Hbenzocyclohepten-2-ol werden bei 0 °C in 4 ml Tetrahydrofuran gelöst, mit 44 mg m-Chlor-perbenzoesäure versetzt und bei 0 °C gerührt. Nach 15 min werden nochmals 15 mg m-Chlor-perbenzoesäure hinzugefügt und das Reaktionsgemisch für weitere 15 min bei 0 °C gerührt. Zur Aufarbeitung wird das Reaktionsgemisch in 10 %ige Natriumthiosulfat-Lösung gegeben, dreimal mit Methylenchlorid extrahiert, mit Wasser gewaschen, über Magnesiumsulfat getrocknet und im Vakuum eingeengt. Säulenchromatographische Reinigung an Kieselgel mit einem Methylenchlorid-Methanol-Gradienten führt zu 82 mg 6-Phenyl-5-{4-[5-(4,4,4-trifluor-butansulfinyl)-pentyloxy]-phenyl}-8,9-dihydro-7H-benzocyclohepten-2-ol vom Schmp.: 145 °C.

### Beispiel 44

### 5-(4-{5-[Methyl-(4,4,5,5,5-pentafluorpentyl)-amino]-pentyloxy}-phenyl)-6-phenyl-8,9-dihydro-7H-benzocyclohepten-2-ol

### a) Methyl-(5-{4-[6-phenyl-2-(tetrahydropyran-2-yloxy)-8,9-dihydro-7Hbenzocyclohepten-5-yl]-phenoxy}-pentyl)-amin

914 mg 2-{5-[4-(5-lodpentyloxy)-phenyl]-6-phenyl-8,9-dihydro-7H-benzocyclohepten-2-yloxy}tetrahydropyran (Bsp.: 12a) werden in einem Druckrohr in 5 ml Tetrahydrofuran gelöst und bei -15 °C ca. 800 mg Methylamin einkondensiert. Anschließend läßt man das verschlossene Druckrohr über Nacht bei Raumtemperatur stehen. Zur Aufarbeitung wird das Gefäß auf -15 °C abgekühlt, überschüssiges Methylamin bei Raumtemperatur im Stickstoffstrom abgedampft, der Ansatz in verdünnte Natriumchloridlösung gegeben, dreimal mit Methylenchlorid extrahiert, über Magnesiumsulfat getrocknet und im Vakuum eingeengt. Man erhält 809 mg Methyl-(5-{4-[6-phenyl-2-(tetrahydropyran-2-yloxy)-8,9-dihydro-7H-benzocyclohepten-5-yl]-phenoxy}-pentyl)-amin als Rohprodukt, welches ohne weitere Aufreinigung in der nächsten Stufe eingesetzt wird.

### b) Methyl-(4,4,5,5,5-pentafluorpentyl)-(5-{4-[6-phenyl-2-(tetrahydropyran-2-yloxy)-8,9-dihydro-7H-benzocyclohepten-5-yl]-phenoxy}-pentyl)-amin

809 mg Methyl-(5-{4-[6-phenyl-2-(tetrahydropyran-2-yloxy)-8,9-dihydro-7H-benzocyclohepten-5-yl]-phenoxy}-pentyl)-amin werden in 15 ml N-Methyl-pyrrolidon gelöst, portionsweise mit insgesamt 698 mg4,4,5,5,5-Pentafluor-pentyltosylat versetzt und 3 Stunden bei 80 °C gerührt. Nachdem der Ansatz auf Raumtemperatur abgekühlt ist, wird das Reaktionsgemisch in verdünnte Natriumchloridlösung gegeben, dreimal mit Diethylether extrahiert, über Magnesiumsulfat getrocknet und im Vakuum eingeengt. Säulenchromatographische Reinigung an Kieselgel mit einem Methylenchlorid-Methanol-Gradienten führt zu 1,8 g Methyl-(4,4,5,5,5-pentafluorpentyl)-(5-{4-[6-' phenyl-2-(tetrahydropyran-2-yloxy)-8,9-dihydro-7H-benzocyclohepten-5-yl]-phenoxy}pentyl)-amin, welches noch mit N-Methyl-pyrrolidon verunreinigt ist.

### c) 5-(4-{5-[Methyl-(4,4,5,5,5-pentafluorpentyl)-amino]-pentyloxy}-phenyl)-6-phenyl-8,9-dihydro-7H-benzocyclohepten-2-ol

1,8 g Methyl-(4,4,5,5,5-pentafluorpentyl)-(5-{4-[6-phenyl-2-(tetrahydropyran-2-yloxy)-8,9-dihydro-7H-benzocyclohepten-5-yl]-phenoxy}-pentyl)-amin, welches noch mit N-Methyl-pyrrolidon verunreinigt ist, wird in 25 ml Methanol und 2,5 ml Wasser gelöst, mit 1,85 g Oxalsäure versetzt und 90 min bei 50 °C gerührt. Nachdem das Reaktionsgemisch abgekühlt ist, wird es in verdünnte Natriumhydrogencarbonatlösung gegeben, dreimal mit Diethylether extrahiert, mit Wasser gewaschen, über Magnesiumsulfat getrocknet und im Vakuum eingeengt. Der Rückstand wird an Kieselgel mit einem Methylenchlorid-Methanol-Gradienten gesäult. Man erhält 214 mg 5-(4-{5-[Methyl-(4,4,5,5,5-pentafluorpentyl)-amino]-pentyloxy}-phenyl)-6-phenyl-8,9-dihydro-7H-benzocyclohepten-2-ol.

### Herstellung des Ausgangsmaterials

### 4,4,5,5,5-Pentafluor-pentyltosylat

6,4 g p-Toluolsulfonylchlorid werden in 10 ml Pyridin vorgelegt, mit 5,44 g 4,4,5,5,5-Pentafluor-pentan-1-ol bei 0 °C tropfenweise versetzt und nach beendeter Zugabe 2 Stunden bei Raumtemperatur gerührt. Zur Aufarbeitung wird der Ansatz in eiskalte 2N Salzsäure eingetragen, dreimal mit Diethylether extrahiert, mit gesättigter Natriumchloridlösung gewaschen, über Magnesiumsulfat getrocknet und im Vakuum eingeengt. Säulenchromatographische Reinigung an Kieselgel mit einem Hexan-Essigester-Gradienten liefert 8,7 g 4,4,5,5,5-Pentafluor-pentyltosylat als klare Flüssigkeit.

### Beispiel 45

### 5-[4-(5-Benzylthio-pentyloxy)-phenyl]-6-phenyl-8,9-dihydro-7H-benzocyclohepten-2-ol

### a) Benzyl-(5-{4-[6-phenyl-2-(tetrahydropyran-2-yloxy)-8,9-dihydro-7Hbenzocyclohepten-5-yl]-phenoxy}-pentyl)-sulfid

1,5 g S-(5-{4-[6-Phenyl-2-(tetrahydropyran-2-yloxy)-8,9-dihydro-7H-benzocyclohepten-5-yl]-phenoxy}-pentyl)-thioacetat (Bsp.: 12b) werden in 10 ml Tetrahydrofuran und 22 ml Methanol gelöst und bei Raumtemperatur tropfenweise mit 0,6 ml einer 30%igen methanolischen Natriummethylatlösung versetzt. Nach beendeter Zugabe wird noch 30 min bei Raumtemperatur gerührt, bevor 0,5 ml Benzylbromid bei der gleichen Temperatur zugetropft werden. Nach 2 Stunden wird das Reaktionsgemisch mit verdünnter Natriumchloridlösung versetzt, dreimal mit Essigester extrahiert, neutral gewaschen, über Magnesiumsulfat getrocknet und im Vakuum eingeengt. Säulenchromatographische Reinigung an Kieselgel mit einem Hexan-Essigester-Gradienten liefert 1,12 g Benzyl-(5-{4-[6-phenyl-2-(tetrahydropyran-2-yloxy)-8,9-dihydro-7H-benzocyclohepten-5-yl]-phenoxy}-pentyl)-sulfid als Schaum.

### b) 5-[4-(5-Benzylthio-pentyloxy)-phenyl]-6-phenyl-8,9-dihydro-7H-benzocyclohepten-2-ol

1,12 g Benzyl-(5-{4-[6-phenyl-2-(tetrahydropyran-2-yloxy)-8,9-dihydro-7H-benzocyclohepten-5-yl]-phenoxy}-pentyl)-sulfid werden in 27 ml Methanol, 3 ml Tetrahydrofuran und 2,7 ml Wasser gelöst, mit 1,16 g Oxalsäure versetzt und 3 Stunden bei 50 °C gerührt. Anschließend wird das Reaktionsgemisch auf Raumtemperatur abgekühlt, mit 5 ml Wasser versetzt und 5 min gerührt. Der ausgefallene Niederschlag wird abgesaugt und getrocknet. Man erhält 855 mg 5-[4-(5-Benzylthio-pentyloxy)-phenyl]-6-phenyl-8,9-dihydro-7H-benzocyclohepten-2-ol vom Schmp.: 178-186 °C.

### Beispiel 46

### 5-[4-(5-Benzylsulfinyl-pentyloxy)-phenyl]-6-phenyl-8,9-dihydro-7H-benzocyclohepten-2-ol

555 mg 5-[4-(5-Benzylthio-pentyloxy)-phenyl]-6-phenyl-8,9-dihydro-7H-benzocyclohepten-2-ol (Bsp.: 45) werden in 17 ml Methanol, 15 ml Essigester und 1 ml Wasser gelöst, mit 241 mg Natriumperiodat versetzt und zunächst bei Raumtemperatur, später 3 Stunden bei 50 °C gerührt. Zur Aufarbeitung wird das Reaktionsgemisch mit verdünnter Natriumchloridlösung versetzt, dreimal mit Methylenchlorid extrahiert, mit Wasser gewaschen, über Magnesiumsulfat getrocknet und im Vakuum eingeengt. Durch säulenchromatographische Reinigung an Kieselgel mit einem Hexan-Essigester-Gradienten erhält man 469 mg 5-[4-(5-Benzylsulfinyl-pentyloxy)-phenyl]-6-phenyl-8,9-dihydro-7H-benzocyclohepten-2-ol vom Schmp.: 196 °C.

### Beispiel 47

### 5-{4-[5-(4-Methyl-benzylthio)-pentyloxy]-phenyl}-6-phenyl-8,9-dihydro-7Hbenzocyclohepten-2-ol

### a) (4-Methyl-benzyl)-(5-{4-[6-phenyl-2-(tetrahydropyran-2-yloxy)-8,9-dihydro-7Hbenzocyclohepten-5-yl]-phenoxy}-pentyl)-sulfid

1,5 g S-(5-{4-[6-Phenyl-2-(tetrahydropyran-2-yloxy)-8,9-dihydro-7H-benzocyclohepten-5-yl]-phenoxy}-pentyl)-thioacetat (Bsp.: 12b) werden in 5 ml Tetrahydrofuran und 20 ml Methanol gelöst und bei Raumtemperatur tropfenweise mit 0,6 ml einer 30%igen methanolischen Natriummethylatlösung versetzt. Nach beendeter Zugabe wird noch 30 min bei Raumtemperatur gerührt, bevor 757 mg 4-Methyl-benzylbromid in 5 ml Tetrahydrofuran bei der gleichen Temperatur zugetropft werden. Nach 2 Stunden wird das Reaktionsgemisch mit verdünnter Natriumchloridlösung versetzt, dreimal mit Essigester extrahiert, neutral gewaschen, über Magnesiumsulfat getrocknet und im Vakuum eingeengt. Säulenchromatographische Reinigung an Kieselgel mit einem Hexan-Essigester-Gradienten liefert 1,24 g (4-Methyl-benzyl)-(5-{4-[6-phenyl-2-(tetrahydropyran-2-yloxy)-8,9-dihydro-7H-benzocyclohepten-5-yl]-phenoxy}-pentyl)-sulfid als Schaum.

### b) 5-{4-[5-(4-Methyl-benzylthio)-pentyloxy]-phenyl}-6-phenyl-8,9-dihydro-7Hbenzocyclohepten-2-ol

250 mg (4-Methyl-benzyl)-(5-{4-[6-phenyl-2-(tetrahydropyran-2-yloxy)-8,9-dihydro-7H-benzocyclohepten-5-yl]-phenoxy}-pentyl)-sulfid werden in 6 ml Methanol, 1 ml Tetrahydrofuran und 0,6 ml Wasser gelöst, mit 258 mg Oxalsäure versetzt und 3 Stunden bei 50 °C gerührt. Anschließend wird das Reaktionsgemisch auf Raumtemperatur abgekühlt, mit 5 ml Wasser versetzt und 5 min gerührt. Der ausgefallene Niederschlag wird abgesaugt und getrocknet. Man erhält 196 mg 5-{4-[5-(4-Methyl-benzylthio)-pentyloxy]-phenyl}-6-phenyl-8,9-dihydro-7Hbenzocyclohepten-2-ol vom Schmp.: 165-169 °C.

### Beispiel 48

### 5-{4-[5-(4-Methyl-benzylsulfinyl)-pentyloxy]-phenyl}-6-phenyl-8,9-dihydro-7Hbenzocyclohepten-2-ol

600 mg 5-{4-[5-(4-Methyl-benzylthio)-pentyloxy]-phenyl}-6-phenyl-8,9-dihydro-7Hbenzocyclohepten-2-ol (Bsp.: 47b) werden in 18 ml Methanol, 15 ml Essigester und 1 ml Wasser gelöst, mit 252 mg Natriumperiodat versetzt und zunächst bei Raumtemperatur, später 3 Stunden bei 50 °C gerührt. Zur Aufarbeitung wird das Reaktionsgemisch mit verdünnter Natriumchloridlösung versetzt, dreimal mit Methylenchlorid extrahiert, mit Wasser gewaschen, über Magnesiumsulfat getrocknet und im Vakuum eingeengt. Durch säulenchromatographische Reinigung an Kieselgel mit einem Hexan-Essigester-Gradienten erhält man 248 mg 5-{4-[5-(4-Methylbenzylsulfinyl)-pentyloxy]-phenyl}-6-phenyl-8,9-dihydro-7H-benzocyclohepten-2-ol.

### Beispiel 49

### 6-Phenyl-5-{4-[5-(4-trifluormethyl-benzylthio)-pentyloxy]-phenyl}-8,9-dihydro-7Hbenzocyclohepten-2-ol

### a) (5-{4-[6-Phenyl-2-(tetrahydropyran-2-yloxy)-8,9-dihydro-7H-benzocyclohepten-5-yl]-phenoxy}-pentyl)-(4-trifluormethyl-benzyl)-sulfid

1,5 g S-(5-{4-[6-Phenyl-2-(tetrahydropyran-2-yloxy)-8,9-dihydro-7H-benzocyclohepten-5-yl]-phenoxy}-pentyl)-thioacetat (Bsp.: 12b) werden in 10 ml Tetrahydrofuran und 20 ml Methanol gelöst und bei Raumtemperatur tropfenweise mit 0,6 ml einer 30%igen methanolischen Natriummethylatlösung versetzt. Nach beendeter Zugabe wird noch 30 min bei Raumtemperatur gerührt, bevor 977 mg 4-(Trifluormethyl)-benzylbromid in 5 ml Tetrahydrofuran bei der gleichen Temperatur zugetropft werden. Nach 2 Stunden wird das Reaktionsgemisch mit verdünnter Natriumchloridlösung versetzt, dreimal mit Essigester extrahiert, neutral gewaschen, über Magnesiumsulfat getrocknet und im Vakuum eingeengt. Säulenchromatographische Reinigung an Kieselgel mit einem Hexan-Essigester-Gradienten liefert 1,15 g (5-{4-[6-Phenyl-2-(tetrahydropyran-2-yloxy)-8,9-dihydro-7H-benzocyclohepten-5-yl]-phenoxy}-pentyl)-(4-trifluormethyl-benzyl)-sulfid als Schaum.

### b) 6-Phenyl-5-{4-[5-(4-trifluormethyl-benzylthio)-pentyloxy]-phenyl}-8,9-dihydro-7Hbenzocyclohepten-2-ol

1,14 g (5-{4-[6-Phenyl-2-(tetrahydropyran-2-yloxy)-8,9-dihydro-7H-benzocyclohepten-5-yl]-phenoxy}-pentyl)-(4-trifluormethyl-benzyl)-sulfid werden in 27 ml Methanol, 3 ml Tetrahydrofuran und 2,7 ml Wasser gelöst, mit 1,16 g Oxalsäure versetzt und 3 Stunden bei 50 °C gerührt. Anschließend wird das Reaktionsgemisch auf Raumtemperatur abgekühlt, mit 5 ml Wasser versetzt und 5 min gerührt. Der ausgefallene Niederschlag wird abgesaugt und getrocknet. Man erhält 933 mg 6-Phenyl-5-{4-(5-(4-trifluormethyl-benzylthio)-pentyloxy]-phenyl}-8,9-dihydro-7Hbenzocyclohepten-2-ol vom Schmp.: 147 °C.

### Beispiel 50

### 6-Phenyl-5-{4-[5-(4-trifluormethyl-benzylsulfinyl)-pentyloxy]-phenyl}-8,9-dihydro-7Hbenzocyclohepten-2-ol

600 mg 6-Phenyl-5-{4-[5-(4-trifluormethyl-benzylthio)-pentyloxy]-phenyl}-8,9-dihydro-7H-benzocyclohepten-2-ol (Bsp.: 49b) werden in 16 ml Methanol, 15 ml Essigester und 1 ml Wasser gelöst, mit 230 mg Natriumperiodat versetzt und zunächst bei Raumtemperatur, später 3 Stunden bei 50 °C gerührt. Zur Aufarbeitung wird das Reaktionsgemisch mit verdünnter Natriumchloridlösung versetzt, dreimal mit Methylenchlorid extrahiert, mit Wasser gewaschen, über Magnesiumsulfat getrocknet und im Vakuum eingeengt. Durch säulenchromatographische Reinigung an Kieselgel mit einem Hexan-Essigester-Gradienten erhält man 494 mg 6-Phenyl-5-{4-[5-(4-trifluormethyl-benzylsulfinyl)-pentyloxy]-phenyl}-8,9-dihydro-7H-benzocyclohepten-2-ol.

### Beispiel 51

### 6-Phenyl-5-[4-(5-phenylthio-pentyloxy)-phenyl]-8,9-dihydro-7H-benzocyclohepten-2-ol

### a) Phenyl-(5-{4-[6-phenyl-2-(tetrahydropyran-2-yloxy)-8,9-dihydro-7Hbenzocyclohepten-5-yl]-phenoxy}-pentyl)-sulfid

1,0 g 2-{5-[4-(5-Iodpentyloxy)-phenyl]-6-phenyl-8,9-dihydro-7H-benzocyclohepten-2-yloxy}-tetrahydropyran (Bsp.: 12a) werden in 5 ml Tetrahydrofuran gelöst und bei Raumtemperatur mit 0,34 ml 30%iger wässriger Kaliumhydroxydlösung und 0,17 ml Thiophenol versetzt. Anschließend wird 4 Stunden bei 90 °C gerührt. Zur Aufarbeitung läßt man das Reaktionsgemisch auf Raumtemperatur kommen, verdünnt mit Diethylether, wäscht dreimal mit Wasser, trocknet über Magnesiumsulfat und engt im Vakuum ein. Säulenchromatographische Reinigung an Kieselgel mit einem Hexan-Essigester-Gradienten liefert 816 mg Phenyl-(5-{4-[6-phenyl-2-(tetrahydropyran-2-yloxy)-8,9-dihydro-7H-benzocyclohepten-5-yl]-phenoxy}pentyl)-sulfid als Schaum.

### b) 6-Phenyl-5-[4-(5-phenylthio-pentyloxy)-phenyl]-8,9-dihydro-7H-benzocyclohepten-2-ol

816 mg Phenyl-(5-{4-[6-phenyl-2-(tetrahydropyran-2-yloxy)-8,9-dihydro-7H-benzocyclohepten-5-yl]-phenoxy}-pentyl)-sulfid werden in 20 ml Methanol, 5 ml Tetrahydrofuran und 2 ml Wasser gelöst, mit 890 mg Oxalsäure versetzt und 90 min bei 50 °C gerührt. Anschließend wird das Reaktionsgemisch auf Raumtemperatur abgekühlt, mit 25 ml Wasser versetzt und 30 min gerührt. Der ausgefallene Niederschlag wird abgesaugt und getrocknet. Man erhält 670 mg 6-Phenyl-5-[4-(5-phenylthio-pentyloxy)-phenyl]-8,9-dihydro-7H-benzocyclohepten-2-ol vom Schmp.: 192 °C.

### Beispiel 52 und 53

### 6-Phenyl-5-[4-(5-phenylsulfinyl-pentyloxy)-phenyl]-8,9-dihydro-7H-benzocyclohepten-2-ol und 6-Phenyl-5-[4-(5-phenylsulfonyl-pentyloxy)-phenyl]-8,9-dihydro-7Hbenzocyclohepten-2-ol

109 mg 6-Phenyl-5-[4-(5-phenylthio-pentyloxy)-phenyl]-8,9-dihydro-7H-benzocyclohepten-2-ol (Bsp.: 51 b) werden bei 0°C in 8 ml Methylenchlorid vorgelegt und mit 57 mg m-Chlorperbenzoesäure versetzt. Nach 1,5 Stunden Rühren in der Kälte, wird die Reaktion durch Zugabe von Natriumthiosulfatlösung beendet. Anschließend wird der Ansatz mit gesättigter Natriumhydrogencarbonatlösung versetzt, dreimal mit Methylenchlorid extrahiert, neutral gewaschen, über Magnesiumsulfat getrocknet und im Vakuum zur Trockne eingeengt. Präparative Dünnschichtchromatographie mit einem Hexan-Essigester-Gemisch liefert 22 mg 6-Phenyl-5-[4-(5-phenylsulfonylpentyloxy)-phenyl]-8,9-dihydro-7H-benzocyclohepten-2-ol vom Schmp. 162 °C und 75 mg 6-Phenyl-5-[4-(5-phenylsulfinyl-pentyloxy)-phenyl]-8,9-dihydro-7H-benzocyclohepten-2-ol.

### Beispiel 54

### 5-{4-[5-(4-tert.-Butyl-phenylthio)-pentyloxy]-phenyl}-6-phenyl-8,9-dihydro-7Hbenzocyclohepten-2-ol

### a) (4-tert.-Butyl-phenyl)-(5-{4-[6-phenyl-2-(tetrahydropyran-2-yloxy)-8,9-dihydro-7Hbenzocyclohepten-5-yl]-phenoxy}-pentyl)-sulfid

1,5 g 2-{5-[4-(5-lodpentyloxy)-phenyl]-6-phenyl-8,9-dihydro-7H-benzocyclohepten-2-yloxy}-tetrahydropyran (Bsp.: 12a) werden in 7,5 ml Tetrahydrofuran gelöst und bei Raumtemperatur mit 0,51 ml 30%iger wässriger Kaliumhydroxyd-Lösung und 409 mg 4-tert.-Butylthiophenol versetzt. Anschließend wird 1 Stunde bei 90 °C gerührt. Zur Aufarbeitung läßt man das Reaktionsgemisch auf Raumtemperatur kommen, verdünnt mit Diethylether, wäscht zweimal mit Wasser, trocknet über Magnesiumsulfat und engt im Vakuum ein. Säulenchromatographische Reinigung an Kieselgel mit einem Hexan-Diethylether-Gradienten liefert 1,35 g (4-tert.-Butylphenyl)-(5-{4-[6-phenyl-2-(tetrahydropyran-2-yloxy)-8,9-dihydro-7Hbenzocyclohepten-5-yl]-phenoxy}-pentyl)-sulfid als Schaum.

### b) 5-{4-[5-(4-tert.-Butyl-phenylthio)-pentyloxy]-phenyl}-6-phenyl-8,9-dihydro-7Hbenzocyclohepten-2-ol

1,34 g (4-tert.-Butyl-phenyl)-(5-{4-[6-phenyl-2-(tetrahydropyran-2-yloxy)-8,9-dihydro-7H-benzocyclohepten-5-yl]-phenoxy}-pentyl)-sulfid werden in 30 ml Methanol, 3 ml Tetrahydrofuran und 3 ml Wasser gelöst, mit 1,33 g Oxalsäure versetzt und 5 Stunden bei 50 °C gerührt. Anschließend wird das Reaktionsgemisch auf Raumtemperatur abgekühlt, mit 10 ml Wasser versetzt und 15 min gerührt. Der ausgefallene Niederschlag wird abgesaugt und getrocknet. Präparative Säulenchromatographie an Kieselgel mit einem Hexan-Essigester-Gradienten liefert 946 mg 5-{4-[5-(4-tert.-Butyl-phenylthio)-pentyloxy]-phenyl}-6-phenyl-8,9-dihydro-7Hbenzocyclohepten-2-ol vom Schmp.: 139 °C.

### Beispiel 55 und 56

### 5-{4-[5-(4-tert.-Butyl-phenylsulfinyl)-pentyloxy]-phenyl}-6-phenyl-8,9-dihydro-7Hbenzocyclohepten-2-ol und 5-{4-[5-(4-tert.-Butyl-phenylsulfonyl)-pentyloxy]-phenyl}-6-phenyl-8,9-dihydro-7Hbenzocyclohepten-2-ol

450 mg 5-{4-[5-(4-tert.-Butyl-phenylthio)-pentyloxy]-phenyl}-6-phenyl-8,9-dihydro-7Hbenzocyclohepten-2-ol (Bsp.: 54b) werden bei 0°C in 30 ml Methylenchlorid vorgelegt und mit 206 mg m-Chlorperbenzoesäure versetzt. Nach 1 Stunde Rühren in der Kälte, wird der Ansatz mit gesättigter Natriumhydrogencarbonatlösung versetzt, dreimal mit Methylenchlorid extrahiert, neutral gewaschen, über Magnesiumsulfat getrocknet und im Vakuum zur Trockne eingeengt. Präparative Säulenchromatographie an Kieselgel mit einem Hexan-Essigester-Gradienten liefert 32 mg 5-{4-[5-(4-tert.-Butyl-phenylsulfonyl)-pentyloxy]-phenyl}-6-phenyl-8,9-dihydro-7H-benzocyclohepten-2-ol und 412 mg 5-{4-[5-(4-tert.-Butyl-phenylsulfinyl)-pentyloxy]-phenyl}-6-phenyl-8,9-dihydro-7H-benzocyclohepten-2-ol.

### Beispiel 57

### 6-Phenyl-5-{4-[5-(4-trifluormethyl-phenylthio)-pentyloxy]-phenyl}-8,9-dihydro-7Hbenzocyclohepten-2-ol

### a) (5-{4-[6-Phenyl-2-(tetrahydropyran-2-yloxy)-8,9-dihydro-7H-benzocyclohepten-5-yl]-phenoxy}-pentyl)-(4-trifluormethyl-phenyl)-sulfid

1,5 g 2-{5-[4-(5-lodpentyloxy)-phenyl]-6-phenyl-8,9-dihydro-7H-benzocyclohepten-2-yloxy}-tetrahydropyran (Bsp.: 12a) werden in 7,5 ml Tetrahydrofuran gelöst und bei Raumtemperatur mit 0,51 ml 30%iger wässriger Kaliumhydroxyd-Lösung und 438 mg 4-(Trifluormethyl)-thiophenol versetzt. Anschließend wird 1 Stunde bei 90 °C gerührt. Zur Aufarbeitung läßt man das Reaktionsgemisch auf Raumtemperatur kommen, verdünnt mit Diethylether, wäscht zweimal mit Wasser, trocknet über Magnesiumsulfat und engt im Vakuum ein. Säulenchromatographische Reinigung an Kieselgel mit einem Hexan-Diethylether-Gradienten liefert 1,28 g (5-{4-[6-Phenyl-2-(tetrahydropyran-2-yloxy)-8,9-dihydro-7H-benzocyclohepten-5-yl]-phenoxy}-pentyl)-(4-trifluormethyl-phenyl)-sulfid.

### b) 6-Phenyl-5-{4-[5-(4-trifluorrnethyl-phenylthio)-pentyloxy]-phenyl}-8,9-dihydro-7Hbenzocyclohepten-2-ol

1,28 g (5-{4-[6-Phenyl-2-(tetrahydropyran-2-yloxy)-8,9-dihydro-7H-benzocyclohepten-5-yl]-phenoxy}-pentyl)-(4-trifluormethyl-phenyl)-sulfid werden in 29 ml Methanol, 3 ml Tetrahydrofuran und 3 ml Wasser gelöst, mit 1,25 g Oxalsäure versetzt und 5 Stunden bei 50 °C gerührt. Anschließend wird das Reaktionsgemisch auf Raumtemperatur abgekühlt, mit 10 ml Wasser versetzt und 15 min gerührt. Der ausgefallene Niederschlag wird abgesaugt und getrocknet. Umkristallisation aus Essigester liefert 890 mg 6-Phenyl-5-{4-[5-(4-trifluormethyl-phenylthio)-pentyloxy]-phenyl}-8,9-dihydro-7H-benzocyclohepten-2-ol vom Schmp.: 189 °C.

### Beispiel 58 und 59

### 6-Phenyl-5-{4-[5-(4-trifluormethyl-phenylsulfinyl)-pentyloxy]-phenyl}-8,9-dihydro-7Hbenzocyclohepten-2-ol und 6-Phenyl-5-{4-[5-(4-trifluormethyl-phenylsulfonyl)-pentyloxy]-phenyl}-8,9-dihydro-7Hbenzocyclohepten-2-ol

425 mg 6-Phenyl-5-{4-[5-(4-trifluormethyl-phenylthio)-pentyloxy]-phenyl}-8,9-dihydro-7H-benzocyclohepten-2-ol (Bsp.: 57b) werden bei 0°C in 28 ml Methylenchlorid vorgelegt und mit 190 mg m-Chlorperbenzoesäure versetzt. Nach 1 Stunde Rühren in der Kälte, wird der Ansatz mit gesättigter Natriumhydrogencarbonatlösung versetzt, dreimal mit Methylenchlorid extrahiert, neutral gewaschen, über Magnesiumsulfat getrocknet und im Vakuum zur Trockne eingeengt. Präparative Säulenchromatographie an Kieselgel mit einem Hexan-Essigester-Gradienten liefert 71 mg 6-Phenyl-5-{4-[5-(4-trifluormethyl-phenylsulfonyl)-pentyloxy]-phenyl}-8,9-dihydro-7H-benzocyclohepten-2-ol vom Schmp.: 187 °C und 325 mg 6-Phenyl-5-{4-[5-(4-trifluormethyl-phenylsulfinyl)-pentyloxy]-phenyl}-8,9-dihydro-7Hbenzocyclohepten-2-ol.

## Patentansprüche

1. Benzocycloheptene der allgemeinen Formel I 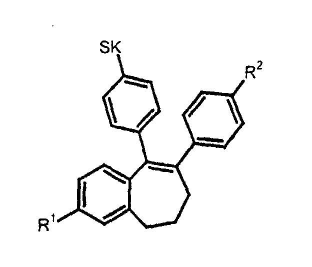 worin
R¹ und R² unabhängig voneinander für ein Wasserstoffatom, eine Hydroxygruppe, eine gegebenenfalls substituierte C₁-C₁₀-Alkoxygruppe, eine gegebenenfalls substituierte C₁-C₁₀-Alkanoyloxygruppe oder eine gegebenenfalls substituierte C₇-C₁₅-Aroyloxygruppe, sowie
SK für eine Seitenkette -A-B-Z,
wobei
A eine direkte Bindung oder ein Sauerstoffatom,
B eine gerad- oder verzweigtkettige, gegebenenfalls substituierte
Alkylen-, Alkenylen- oder Alkinylengruppe mit bis zu 10
Kohlenstofatomen,
Z eine Gruppe -D-SOₓ-E-G, eine Aminogruppe -NR⁷R⁸ oder ein
Substituent G,
worin
D eine direkte Bindung oder eine Gruppe -NR³(R⁴-), R³ eine gerad- oder verzweigtkettige Alkyl-, Alkenyl- oder Alkinylgruppe mit bis zu 10 Kohlenstoffatomen sowie R⁴ eine gerad- oder verzweigtkettige, gegebenenfalls substituierte Alkylen-, Alkenylenoder Alkinylengruppe mit bis zu 10 Kohlenstoffatomen, wobei das Stickstoffatom auch in ein 4- bis 7-gliedriges Ringsystem eingebaut sein kann,
x 0, 1 oder 2,
E eine gerad- oder verzweigtkettige, gegebenenfalls substituierte Alkylen-, Alkenylen- oder Alkinylengruppe mit bis zu 10 Kohlenstofatomen,
G eine teilweise oder vollständig fluorierte, gerad- oder verzweigtkettige Alkylgruppe mit bis zu 5 Kohlenstoffatomen, ein gegebenenfalls substituierter Aryl- oder Heteroarylrest, ein Carbamoylrest -C(O)-NR⁵R⁶ mit R⁵ und R⁶ in der Bedeutung von R⁷ und R⁸, ein Halogenatom oder ein Wasserstoffatom,
R⁷ und R⁸ unabhängig voneinander ein Wasserstoffatom, ein gerad- oder verzweigtkettiger, gegebenenfalls teilweise fluorierter Alkyl-, Alkenyl- oder Alkinylrest mit bis zu 14 Kohlenstoffatomen, der durch ein bis drei Heteroatome -O- und -S- und Gruppierungen -NR⁹-, worin R⁹ ein Wasserstoffatom oder ein C₁-C₃-Alkylrest ist, unterbrochen sein kann, ein gegebenenfalls einoder zweifach substituierter Aryl- oder Heteroarylrest, ein gegebenenfalls ein- oder zweifach substituierter C₃-C₁₀-Cycloalkylrest, ein gegebenenfalls ein- oder zweifach substituierter C₄-C₁₅-Cycloalkylalkylrest, ein gegebenenfalls ein- oder zweifach substituierter C₇-C₂₀-Aralkylrest, ein gegebenenfalls ein- oder zweifach substituierter Heteroaryl-C₁-C₈-alkylrest oder ein gegebenenfalls substituerter Aminoalkylrest, ein Biphenylenrest oder ein Rest der Formel -C(O)R¹⁰, worin R¹⁰ die vorstehend für R⁷ bzw. R⁸ angegebenen Bedeutungen haben kann,
R⁷ und R⁸ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält und gegebenenfalls substituiert ist, bilden, ist,
bedeutet, und in -A-B-Z, wenn A für ein Sauerstoffatom und Z für G steht, G nicht ein Wasserstoffatom oder ein Halogenatom sein kann, oder wenn A für ein Sauerstoffatom und Z für eine Aminogruppe -NR⁷R⁸, worin R⁷ und R⁸ jeweils eine Methylgruppe bedeuten oder gemeinsam mit dem Stickstoffatom einen Pyrrolidinring bilden, steht, B mindestens 3 Kohlenstoffatome aufweist,
steht, sowie ausgenommen der Verbindung der allgemeinen Formel I, worin R¹=R²=Wasserstoff, SK=-A-B-Z, A = O, B = -(CH₂)₂- und Z=N(C₂H₅)₂.

2. Verbindungen der allgemeinen Formel I nach Anspruch 1, worin R¹ und/oder R² für ein Wasserstoffatom stehen.

3. Verbindungen der allgemeinen Formel I nach Anspruch 1, worin A ein Sauerstoffatom ist..

4. Verbindungen der allgemeinen Formel I nach Anspruch 1, worin B eine geradkettige Alkenylenkette mit 1 bis 6 Kohlenstoffatomen ist.

5. Verbindungen der allgemeinen Formel I nach Anspruch 1, worin die Seitenkette SK aus der Gruppe der folgenden Seitenketten ausgewählt ist
-O-(CH₂)₅S(CH₂)₃C₂F₅
-O-(CH₂)₅SO(CH₂)₃C₂F₅
O-(CH₂)₅SO₂(CH₂)₃C₂F₅
-O-(CH₂)₂-N(CH3)-(CH₂)₃-S-(CH₂)₃C₂F₅
-O-(CH₂)₂-N(CH3)-(CH₂)₃-SO-(CH₂)₃C₂F₅
-O-(CH₂)₅S(CH₂)-C(O)N(CH₃)-(CH₂)₃CH₃
-O-(CH₂)₅SO(CH₂)-C(O)N(CH₃)-(CH₂)₃CH₃
-O-(CH₂)₂-NH(CH₂)OH
-O-(CH₂)₂-N(CH3)-(CH₂)₂-SO(CH₂)-C(O)N(CH₃)-(CH₂)₃CH₃
-O-(CH₂)₂-N(CH3)-(CH₂)₂-SO₂(CH₂)-C(O)N(CH₃)-(CH₂)₃CH₃
-O-(CH₂)₆S(CH₂)-C(O)N(CH₃)-(CH₂)₃CH₃
-O-(CH₂)₆SO(CH₂)-C(O)N(CH₃)-(CH₂)₃CH₃
-O-CH₃
-O-(CH₂)₅-F
-O-(CH₂)₄-F
-O-(CH₂)₃-F
-O-(CH₂)₂-F
-O-(CH₂)₅-Cl
-O-(CH₂)₄-Cl
-O-(CH₂)₃-Cl
-O-(CH₂)₂-Cl
-O-(CH₂)₆S(CH₂)₃C₂F₅
-O-(CH₂)₆SO(CH₂)₃C₂F₅
-O-(CH₂)₆SO(CH₂)-2-Pyridyl
-O-(CH₂)₅SO(CH₂)-2-Pyridyl
-O-(CH₂)₅S(CH₂)₂C₃F₇
-O-(CH₂)₄S(CH₂)₃C₂F₅
-O-(CH₂)₄SO(CH₂)₃C₂F₅
-O-(CH₂)₄SO₂(CH₂)₃C₂F₅
-O-(CH₂)₄S(CH₂)-2-Pyridyl
-O-(CH₂)₄SO(CH₂)-2-Pyridyl
-O-(CH₂)₅S(CH₂)-2-Pyridyl
-O-(CH₂)₅SO(CH₂)-2-Pyridyl
-O-(CH₂)₆S(CH₂)-2-Pyridyl
-O-(CH₂)₆SO(CH₂)-2-Pyridyl
-O-(CH₂)₅S(CH₂)-2-Furyl
-O-(CH₂)₅SO(CH₂)-2-Furyl
-O-(CH₂)₅SO₂(CH₂)-2-Furyl
-O-(CH₂)₅S(CH₂)-2-Thienyl
-O-(CH₂)₅SO(CH₂)-2-Thienyl
-O-(CH₂)₅S(CH₂)₄-F
-O-(CH₂)₅SO(CH₂)₄-F
-O-(CH₂)₅S(CH₂)₃-CF₃
-O-(CH₂)₅SO(CH₂)₃-CF₃
-O-(CH₂)₅-N(CH3)-(CH₂)₃-C₂F₅
-O-(CH₂)₅S(CH₂)-Phenyl
-O-(CH₂)₅SO(CH₂)-2-Phenyl
-O-(CH₂)₅S(CH₂)-p-Tolyl
-O-(CH₂)₅SO(CH₂)-p-Tolyl
-O-(CH₂)₅S(CH₂)-p-CF₃-Phenyl
-O-(CH₂)₅SO(CH₂)-p-CF₃-Phenyl
-O-(CH₂)₅S-Phenyl
-O-(CH₂)₅SO-Phenyl
-O-(CH₂)₅S-(p-Tolyl)
-O-(CH₂)₅SO-(p-Tolyl)
-O-(CH₂)₅S-(p-CF₃-Phenyl)
-O-(CH₂)₅SO-(p-CF₃-Phenyl)

6. Verbindungen der allgemeinen Formel I nach Anspruch 1, worin die Seitenkette SK aus der Gruppe der folgenden Seitenketten ausgewählt ist
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂)₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₈F₁₇
-(CH₂)₅N(CH₃)H
-(CH₂)₅N(CH₃)(CH₂)₉H
-(CH₂)₅-1-Pyrrolidinyl
-(CH₂)₉S(CH₂)₃C₂F₅
-(CH₂)₉SO(CH₂)₃C₂F₅
-(CH₂)₉SO₂(CH₂)₃C₂F₅

7. Verbindungen der allgemeinen Formel I nach Anspruch 1, worin die Seitenkette SK ausgewählt ist aus der allgemeinen Teilformel 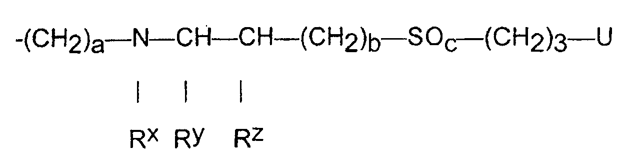 wobei
a 4, 5 oder 6,
b 0, 1 oder 2,
c 0, 1 oder 2,
R^{x} ein Wasserstoffatom oder eine C₁₋₅-Alkylgruppe,
R^{y} und R^{z} je ein Wasserstoffatom, oder
R^{x} und R^{y} gemeinsam eine Alkylengruppe ―(CH₂)_{d}― mit d = 2, 3, 4 oder 5 und R^{z} ein Wasserstoffatom oder
R^{x} und R^{z} gemeinsam eine Alkylengruppe ―(CH₂)ₑ― mit e = 2, 3 oder 4 und R^{y} ein Wasserstoffatom, und
U ein unsubstituierter oder ein- bis fünffach fluorierter Ethylrest ist, oder der terminale Substituent -(CH₂)₃-U in der Seitenkette durch einen gegebenenfalls substituierten Aryl- oder Heteroarylrest, der direkt oder über eine Mono-, Di- oder Trimethylengruppe an das Schwefelatom gebunden ist, ersetzt ist,

8. Verbindungen der allgemeinen Formel I nach Anspruch 7, worin SK die Seitenkette -(CH₂)₅N(CH₃)(CH₂)₃S(CH₂)₃C₂F₅ ist.

9. Verbindungen der allgemeinen Formel I nach Anspruch 7, worin SK die Seitenkette (CH₂)₅N(R^{x})(CHR^{y})CH₂S(CH₂)₃C₂F₅ mit R^{x}+R^{y} = -(CH₂)₃- ist.

10. Verbindungen der allgemeinen Formel I, nämlich
(4,4,5,5,5-Pentafluorpentyl)-{5-[4-(6-phenyl-8,9-dihydro-7H-benzocyclohepten-5-yl)-phenoxy}-pentyl}-sulfid
(4,4,5,5,5-Pentafluorpentyl)-{5-[4-(6-phenyl-8,9-dihydro-7H-benzocyclohepten-5-yl)-phenoxy]-pentyl}-sulfoxid
Methyl-[3-(4,4,5,5,5-pentafluorpentylthio)-propyl]-{2-[4-(6-phenyl-8,9-dihydro-7Hbenzocyclohepten-5-yl)-phenoxy]-ethyl}-amin
Methyl-[3-(4,4,5,5,5-pentafluorpentylsulfinyl)-propyl]-{2-[4-(6-phenyl-8,9-dihydro-7H-benzocyclohepten-5-yl)-phenoxy]-ethyl}-amin
S-{5-[4-(6-Phenyl-8,9-dihydro-7H-benzocyclohepten-5-yl)-phenoxy]-pentyl}thioacetat
N-Butyl-N-methyl-2-{5-[4-(6-phenyl-8,9-dihydro-7H-benzocyclohepten-5-yl)-phenoxy]-pentylthio}-acetamid
N-Butyl-N-methyl-2-{5-[4-(6-phenyl-8,9-dihydro-7H-benzocyclohepten-5-yl)-phenoxy]-pentansulfinyl}-acetamid
5-{4-[5-(4,4,5,5,5-Pentafluor-pentylthio)-pentyloxy]-phenyl}-6-phenyl-8,9-dihydro-7H-benzocyclohepten-2-ol
5-{4-[5-(4,4,5,5,5-Pentafluor-pentanesulfinyl)-pentyloxy]-phenyl}-6-phenyl-8,9-dihydro-7H-benzocyclohepten-2-ol
5-[4-(2-{Methyl-[3-(4,4,5,5,5-pentafluor-pentansulfinyl)-propyl]-amino}-ethoxy)-phenyl]-6-phenyl-8,9-dihydro-7H-benzocyclohepten-2-ol
5-[4-(2-{Methyl-[3-(4,4,5,5,5-pentafluor-pentylthio)-propyl]-amino}-ethoxy)-phenyl]-6-phenyl-8,9-dihydro-7H-benzocyclohepten-2-ol
N-Butyl-N-methyl-2-{5-[4-(2-hydroxy-6-phenyl-8,9-dihydro-7H-benzocyclohepten-5-yl)-phenoxy]-pentylthio}-acetamid
5-{4-[5-(4,4,5,5,5-Pentafluor-pentanesulfonyl)-pentyloxy]-phenyl}-6-phenyl-8,9-dihydro-7H-benzocyclohepten-2-ol
N-Butyl-N-methyl-2-{5-[4-(2-hydroxy-6-phenyl-8,9-dihydro-7H-benzocyclohepten-5-yl)-phenoxy]-pentansulfinyl}-acetamid
N-Butyl-2-[2-({2-[4-(2-hydroxy-6-phenyl-8,9-dihydro-7H-benzocyclohepten-5-yl)-phenoxy]-ethyl}-methyl-amino)-ethansulfinyl]-N-methyl-acetamid
N-Butyl-2-[2-({2-[4-(2-hydroxy-6-phenyl-8,9-dihydro-7H-benzocyclohepten-5-yl)-phenoxy]-ethyl}-methyl-amino)-ethansulfonyl]-N-methyl-acetamid
6-(4-Hydroxy-phenyl)-5-{4-[5-(4,4,5,5,5-pentafluor-pentansulfonyl)-pentyloxy]-phenyl}-8,9-dihydro-7H-benzocyclohepten-2-ol
N-Butyl-2-{6-[4-(2-hydroxy-6-phenyl-8,9-dihydro-7H-benzocyclohepten-5-yl)-phenoxy]-hexansulfinyl}-N-methyl-acetamid
N-Butyl-2-{6-[4-(2-hydroxy-6-phenyl-8,9-dihydro-7H-benzocyclohepten-5-yl)-phenoxy]-hexylthio}-N-methyl-acetamid
6-(4-Hydroxy-phenyl)-5-{4-[5-(4,4,5,5,5-pentafluorpentylthio)-pentyloxy]-phenyl}-8,9-dihydro-7H-benzocyclohepten-2-ol
6-(4-Hydroxy-phenyl)-5-{4-[5-(4,4,5,5,5-pentafluorpentan-sulfinyl)-pentyloxy]-phenyl}-8,9-dihydro-7H-benzocyclohepten-2-ol
5-{4-[4-(4,4,5,5,5-Pentafluor-pentylthio)-butyloxy]-phenyl}-6-phenyl-8,9-dihydro-7H-benzocyclohepten-2-ol
6-Phenyl-5-{4-[4-(pyridin-2-ylmethylthio)-butyloxy]-phenyl}-8,9-dihydro-7Hbenzocyclohepten-2-ol
6-Phenyl-5-{4-[5-(pyridin-2-ylmethylthio)-pentyloxy]-phenyl}-8,9-dihydro-7Hbenzocyclohepten-2-ol
6-Phenyl-5-{4-[6-(pyridin-2-ylmethylthio)-hexyloxy]-phenyl}-8,9-dihydro-7Hbenzocyclohepten-2-ol
5-{4-[4-(4,4,5,5,5-Pentafluor-pentansulfinyl)-butyloxy]-phenyl}-6-phenyl-8,9-dihydro-7H-benzocyclohepten-2-ol
6-Phenyl-5-{4-[4-(pyridin-2-ylmethansulfinyl)-butyloxy]-phenyl}-8,9-dihydro-7Hbenzocyclohepten-2-ol
5-{4-[6-(4,4,5,5,5-Pentafluor-pentylthio)-hexyloxy]-phenyl}-6-phenyl-8,9-dihydro-7H-benzocyclohepten-2-ol
5-{4-[6-(4,4,5,5,5-Pentafluor-pentansulfinyl)-hexyloxy]-phenyl}-6-phenyl-8,9-dihydro-7H-benzocyclohepten-2-ol
6-Phenyl-5-{4-[6-(pyridin-2-ylmethansulfinyl)-hexyloxy]-phenyl}-8,9-dihydro-7Hbenzocyclohepten-2-ol
6-Phenyl-5-{4-[5-(pyridin-2-ylmethansulfinyl)-pentyloxy]-phenyl}-8,9-dihydro-7Hbenzocyclohepten-2-ol
5-{4-[5-(Furan-2-ylmethylthio)-pentyloxy]-phenyl}-6-phenyl-8,9-dihydro-7Hbenzocyclohepten-2-ol
6-Phenyl-5-{4-[5-(thien-2-ylmethylthio)-pentyloxy]-phenyl}-8,9-dihydro-7Hbenzocyclohepten-2-ol
5-{4-[5-(Furan-2-ylmethansulfinyl)-pentyloxy]-phenyl}-6-phenyl-8,9-dihydro-7Hbenzocyclohepten-2-ol
5-{4-[5-(Furan-2-ylmethansulfonyl)-pentyloxy]-phenyl}-6-phenyl-8,9-dihydro-7Hbenzocyclohepten-2-ol
6-Phenyl-5-{4-[5-(thien-2-ylmethansulfonyl)-pentyloxy]-phenyl}-8,9-dihydro-7Hbenzocyclohepten-2-ol
6-Phenyl-5-{4-[5-(thien-2-ylmethansulfinyl)-pentyloxy]-phenyl}-8,9-dihydro-7Hbenzocyclohepten-2-ol
5-{4-[5-(3,3,4,4,5,5,5-Heptafluor-pentylthio)-pentyloxy]-phenyl}-6-phenyl-8,9-dihydro-7H-benzocyclohepten-2-ol
5-{4-[2-(2-Hydroxy-ethylamino)-ethoxy]-phenyl}-6-phenyl-8,9-dihydro-7Hbenzocyclohepten-2-ol
5-{4-[5-(4-Fluor-butylthio)-pentyloxy]-phenyl}-6-phenyl-8,9-dihydro-7Hbenzocyclohepten-2-ol
5-{4-[5-(4-Fluor-butansulfinyl)-pentyloxy]-phenyl}-6-phenyl-8,9-dihydro-7Hbenzocyclohepten-2-ol
6-Phenyl-5-{4-[5-(4,4,4-trifluor-butylthio)-pentyloxy]-phenyl}-8,9-dihydro-7Hbenzocyclohepten-2-ol
6-Phenyl-5-{4-[5-(4,4,4-trifluor-butansulfinyl)-pentyloxy]-phenyl}-8,9-dihydro-7Hbenzocyclohepten-2-ol
5-(4-{5-[Methyl-(4,4,5,5,5-pentafluorpentyl)-amino]-pentyloxy}-phenyl)-6-phenyl-8,9-dihydro-7H-benzocyclohepten-2-ol
5-[4-(5-Benzylthio-pentyloxy)-phenyl]-6-phenyl-8,9-dihydro-7H-benzocyclohepten-2-ol
5-[4-(5-Benzylsulfinyl-pentyloxy)-phenyl]-6-phenyl-8,9-dihydro-7Hbenzocyclohepten-2-ol
5-{4-[5-(4-Methyl-benzylthio)-pentyloxy]-phenyl}-6-phenyl-8,9-dihydro-7Hbenzocyclohepten-2-ol
5-{4-[5-(4-Methyl-benzylsulfinyl)-pentyloxy]-phenyl}-6-phenyl-8,9-dihydro-7Hbenzocyclohepten-2-ol
6-Phenyl-5-{4-[5-(4-trifluormethyl-benzylthio)-pentyloxy]-phenyl}-8,9-dihydro-7Hbenzocyclohepten-2-ol
6-Phenyl-5-{4-[5-(4-trifluormethyl-benzylsulfinyl)-pentyloxy]-phenyl}-8,9-dihydro-7H-benzocyclohepten-2-ol
6-Phenyl-5-[4-(5-phenylthio-pentyloxy)-phenyl]-8,9-dihydro-7H-benzocyclohepten-2-ol
6-Phenyl-5-[4-(5-phenylsulfinyl-pentyloxy)-phenyl]-8,9-dihydro-7Hbenzocyclohepten-2-ol
6-Phenyl-5-[4-(5-phenylsulfonyl-pentyloxy)-phenyl]-8,9-dihydro-7Hbenzocyclohepten-2-ol
5-{4-[5-(4-tert.-Butyl-phenylthio)-pentyloxy]-phenyl}-6-phenyl-8,9-dihydro-7Hbenzocyclohepten-2-ol
5-{4-[5-(4-tert.-Butyl-phenylsulfinyl)-pentyloxy]-phenyl}-6-phenyl-8,9-dihydro-7Hbenzocyclohepten-2-ol
5-{4-[5-(4-tert.-Butyl-phenylsulfonyl)-pentyloxy]-phenyl}-6-phenyl-8,9-dihydro-7Hbenzocyclohepten-2-ol
6-Phenyl-5-{4-[5-(4-trifluormethyl-phenylthio)-pentyloxy]-phenyl}-8,9-dihydro-7Hbenzocyclohepten-2-ol
6-Phenyl-5-{4-[5-(4-trifluormethyl-phenylsulfinyl)-pentyloxy]-phenyl}-8,9-dihydro-7H-benzocyclohepten-2-ol
6-Phenyl-5-{4-[5-(4-trifluormethyl-phenylsulfonyl)-pentyloxy]-phenyl}-8,9-dihydro-7H-benzocyclohepten-2-ol.

11. Arzneimittel, enthaltend mindestens eine Verbindung der allgemeinen Formel I gemäß Anspruch 1 sowie einen pharmazeutisch verträglichen Träger.

12. Verwendung der Verbindungen der allgemeinen Formel I gemäß Anspruch 1 zur Herstellung von Arzneimitteln

13. Verwendung von Verbindungen der allgemeinen Formel I gemäß Anspruch 1 in Kombination mit einem oder mehreren reinen Estrogenen zur Herstellung von Arzneimitteln zur Behandlung gynäkologischer Störungen.

14. Verwendung von Verbindungen der allgemeinen Formel I gemäß Anspruch 1 in Kombination mit einem oder mehreren reinen Estrogenen zur Herstellung von Arzneimitteln für die Hormonersatztherapie.

15. Verwendung von Verbindungen der allgemeinen Formel I gemäß Anspruch 1 in Kombination mit einem oder mehreren reinen Estrogenen zur Herstellung von Arzneimitteln für die weibliche Fertilitätskontrolle.

## Claims

1. Benzocycloheptenes of general formula I 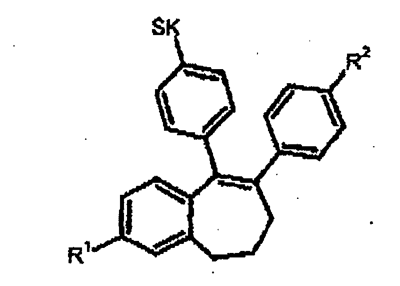 in which
R¹ and R², independently of one another, stand for a hydrogen atom, a hydroxy group, an optionally substituted C₁-C₁₀ alkoxy group, an optionally substituted C₁-C₁₀ alkanoyloxy group or an optionally substituted C₇-C₁₅ aroyloxy group, and
SK stands for a side chain -A-B-Z, where
A means a direct bond or an oxygen atom,
B means a straight-chain or branched-chain, optionally substituted alkylene, alkenylene or alkinylene group with up to 10 carbon atoms,
Z means a group -D-SOₓ₋E-G, an amino group -NR⁷R⁸ or a substituent G,
in which
D means a direct bond or a group -NR³(R⁴-), R³ means a straight-chain or branched-chain alkyl, alkenyl or alkinyl group with up to 10 carbon atoms and R⁴ means a straight-chain or branched-chain, optionally substituted alkylene, alkenylene or alkinylene group with up to 10 carbon atoms, where the nitrogen atom can also be incorporated in a 4- to 7-membered ring system,
x means 0, 1 or 2,
E means a straight-chain or branched-chain, optionally substituted alkylene, alkenylene or alkinylene group with up to 10 carbon atoms,
G means a partially or completely fluorinated, straight-chain or branched-chain alkyl group with up to 5 carbon atoms, an optionally substituted aryl or heteroaryl radical, a carbamoyl radical -C(O)-NR⁵R⁶ with R⁵ and R⁶ in the meaning of R⁷ and R⁸, a halogen atom or a hydrogen atom,
R⁷ and R⁸, independently of one another, mean a hydrogen atom, a straight-chain or branched-chain, optionally partially fluorinated alkyl, alkenyl or alkinyl radical with up to 14 carbon atoms, which can be interrupted by one to three heteroatoms -O- and -S- and groupings -NR⁹-, in which R⁹ means a hydrogen atom or a C₁-C₃ alkyl radical, an aryl or heteroaryl radical that is optionally substituted in one or two places, a C₃-C₁₀ cycloalkyl radical that is optionally substituted in one or two places, a C₄-C₁₅ cycloalkylalkyl radical that is optionally substituted in one or two places, a C₇-C₂₀ aralkyl radical that is optionally substituted in one or two places, a heteroaryl-C₁-C₈ alkyl radical that is optionally substituted in one or two places or an optionally substituted aminoalkyl radical, a biphenylene radical or a radical of formula -C(O)R¹⁰, in which R¹⁰ can have the meanings that are indicated above for R⁷ or R⁸,
R⁷ and R⁸ with the nitrogen atom, to which they are bonded, form a saturated or unsaturated heterocycle with 5 or 6 chain links, which optionally contains one or two additional heteroatoms, selected from nitrogen, oxygen and sulfur, and optionally is substituted, and in -A-B-Z, if A stands for an oxygen atom and Z stands for G, G cannot be a hydrogen atom or a halogen atom, or if A stands for an oxygen atom and Z stands for an amino group -NR⁷R⁸, in which R⁷ and R⁸ in each case mean a methyl group or together with the nitrogen atom form a pyrrolidine ring, B has at least 3 carbon atoms, and excluding the compound of general formula I in which R¹/R² = hydrogen, SK = -A-B-Z, A = O, B = -(CH₂)₂-and Z = N(C₂H₅)₂.

2. Compounds of general formula I according to claim 1, in which R¹ and/or R² stand for a hydrogen atom.

3. Compounds of general formula I according to claim 1, in which A is an oxygen atom.

4. Compounds of general formula I according to claim 1, in which B is a straight-chain alkylene chain with 1 to 6 carbon atoms.

5. Compounds of general formula I according to claim 1, in which side chain SK is selected from the group of the following side chains
-O-(CH₂)₅S(CH₂)₃C₂F₅
-O-(CH₂)₅SO(CH₂)₃C₂F₅
-O-(CH₂)₅SO₂(CH₂)₃C₂F₅
-O-(CH₂)₂-N(CH₃)-(CH₂)₃-S-(CH₂)₃C₂F₅
-O-(CH₂)₂-N(CH₃)-(CH₂)₃-SO-(CH₂)₃C₂F₅
-O-(CH₂)₅S(CH₂)-C(O)N(CH₃)-(CH₂)₃CH₃
-O-(CH₂)₅SO(CH₂)-C(O)N(CH₃)-(CH₂)₃CH₃
-O-(CH₂)₂-NH(CH₂)OH
-O-(CH₂)₂-N(CH₃)-(CH₂)₂-SO(CH₂)-C(O)N(CH₃)-(CH₂)₃CH₃
-O-(CH₂)₂-N(CH₃)-(CH₂)₂-SO₂(CH₂)-C(O)N(CH₃)-(CH₂)₃CH₃
-O-(CH₂)₆S(CH₂)-C(O)N(CH₃)-(CH₂)₃CH₃
-O-(CH₂)₆SO(CH₂)-C(O)N(CH₃)-(CH₂)₃CH₃
-O-CH₃
-O-(CH₂)₅-F
-O-(CH₂)₄-F
-O-(CH₂)₃-F
-O-(CH₂)₂-F
-O-(CH₂)₅-Cl
-O-(CH₂)₄-Cl
-O-(CH₂)₃-Cl
-O-(CH₂)₂-Cl
-O-(CH₂)₆S(CH₂)₃C₂F₅
-O-(CH₂)₆SO(CH₂)₃C₂F₅
-O-(CH₂)₆SO(CH₂)-2-pyridyl
-O-(CH₂)₅SO(CH₂)-2-pyridyl
-O-(CH₂)₅S(CH₂)₂C₃F₇
-O-(CH₂)₄S(CH₂)₃C₂F₅
-O-(CH₂)₄SO(CH₂)₃C₂F₅
-O-(CH₂)₄SO₂(CH₂)₃C₂F₅
-O-(CH₂)₄S(CH₂)-2-pyridyl
-O-(CH₂)₄SO(CH₂)-2-pyridyl
-O-(CH₂)₅S(CH₂)-2-pyridyl
-O-(CH₂)₅SO(CH₂)-2-pyridyl
-O-(CH₂)₆S(CH₂)-2-pyridyl
-O-(CH₂)₆SO(CH₂)-2-pyridyl
-O-(CH₂)₅S(CH₂)-2-furyl
-O-(CH₂)₅SO(CH₂)-2-furyl
-O-(CH₂)₅SO₂(CH₂)-2-furyl
-O-(CH₂)₅S(CH₂)-2-thienyl
-O-(CH₂)₅SO(CH₂)-2-thienyl
-O-(CH₂)₅S(CH₂)₄-F
-O-(CH₂)₅SO(CH₂)₄-F
-O-(CH₂)₅S(CH₂)₃-CF₃
-O-(CH₂)₅SO(CH₂)₃-CF₃
-O-(CH₂)₅-N(CH₃)-(CH₂)₃-C₂F₅
-O-(CH₂)₅S(CH₂)-phenyl
-O-(CH₂)₅SO(CH₂)-2-phenyl
-O-(CH₂)₅S(CH₂)-p-tolyl
-O-(CH₂)₅SO(CH₂)-p-tolyl
-O-(CH₂)₅S(CH₂)-p-CF₃-phenyl
-O-(CH₂)₅SO(CH₂)-p-CF₃-phenyl
-O-(CH₂)₅S-phenyl
-O-(CH₂)₅SO-phenyl
-O-(CH₂)₅S-(p-tolyl)
-O-(CH₂)₅SO-(p-tolyl)
-O-(CH₂)₅S-(p-CF₃-phenyl)
-O-(CH₂)₅SO-(p-CF₃-phenyl).

6. Compounds of general formula I according to claim 1, in which side chain SK is selected from the group of the following side chains
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂)₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₈F₁₇
-(CH₂)₅N(CH₃)H
-(CH₂)₅N(CH₃)(CH₂)₉H
-(CH₂)₅-1-pyrrolidinyl
-(CH₂)₉S(CH₂)₃C₂F₅
-(CH₂)₉SO(CH₂)₃C₂F₅
-(CH₂)₉SO₂(CH₂)₃C₂F₅.

7. Compounds of general formula I according to claim 1, in which side chain SK is selected from the general partial formula 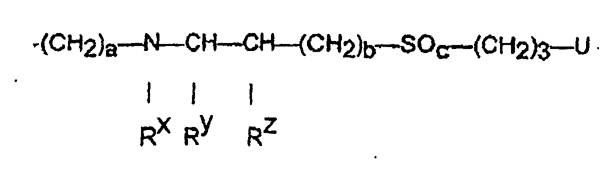 where
a is 4, 5 or 6,
b is 0, 1 or 2,
c is 0, 1 or 2,
R^{x} is a hydrogen atom or a C₁₋₅ alkyl group,
R^{y} and R^{z} are each a hydrogen atom, or
R^{x} and R^{y} together are an alkylene group -(CH₂)_{d}- with d=2, 3, 4 or 5, and R^{z} is a hydrogen atom or
R^{x} and R^{z} together are an alkylene group -(CH₂)ₑ- with e=2, 3 or 4 and R^{y} is a hydrogen atom, and
U is an unsubstituted ethyl radical or an ethyl radical that is fluorinated in one to five places, or
the terminal substituent -(CH₂)₃-U in the side chain is replaced by an optionally substituted aryl or heteroaryl radical, which is bonded directly or via a mono-, di- or trimethylene group to the sulfur atom.

8. Compounds of general formula I according to claim 7, in which SK is the side chain - (CH₂)₅N(CH₃)(CH₂)₃S(CH₂)₃C₂F₅.

9. Compounds of general formula I according to claim 7, in which SK is the side chain (CH₂)₅N(R^{x}) (CHR^{y})CH₂S(CH₂)₃C₂F₅ with R^{x}+R^{y} = -(CH₂)₃-.

10. Compounds of general formula I, namely (4,4,5,5,5-pentafluoropentyl)-{5-[4-(6-phenyl-8,9-dihydro-7H-benzocyclohepten-5-yl)-phenoxy]-pentyl}-sulfide, (4,4,5,5,5-pentafluoropentyl)-{5-[4-(6-phenyl-8,9-di-hydro-7H-benzocyclohepten-5-yl)-phenoxy]-pentyl}-sulfoxide, methyl-[3-(4,4,5,5,5-pentafluoropentylthio)-propyl]-{2-[4-(6-phenyl-8,9-dihydro-7H-benzocyclohepten-5-yl)-phenoxy]-ethyl}-amine, methyl-[3-(4,4,5,5,5-pentafluoropentylsulfinyl)-propyl]-{2-[4-(6-phenyl-8,9-dihydro-7H-benzocyclohepten-5-yl)-phenoxy]-ethyl}-amine, S-{5-[4-(6-phenyl-8,9-dihydro-7H-benzocyclohepten-5-yl)-phenoxy]-pentyl}-thioacetate, N-butyl-N-methyl-2-{5-[4-(6-phenyl-8,9-dihydro-7H-benzocyclohepten-5-yl)-phenoxy]-pentylthio}-acetamide, N-butyl-N-methyl-2-{5-[4-(6-phenyl-8,9-dihydro-7H-benzocyclohepten-5-yl)-phenoxy]-pentanesulfinyl}-acetamide, 5-{4-[5-(4,4,5,5,5-pentafluoro-pentylthio)-pentyloxy]-phenyl}-6-phenyl-8,9-dihydro-7H-benzocyclohepten-2-ol, 5-{4-[5-(4,4,5,5,5-pentafluoro-pentanesulfinyl)-pentyloxy]-phenyl}-6-phenyl-8,9-dihydro-7H-benzocyclohepten-2-ol, 5-[4-(2-{methyl-[3-(4,4,5,5,5-pentafluoro-pentanesulfinyl)-propyl]-amino}-ethoxy]-phenyl]-6-phenyl-8,9-dihydro-7H-benzocyclohepten-2-ol, 5-[4-(2-{methyl-[3-(4,4,5,5,5-pentafluoro-pentylthio)-propyl]-amino}-ethoxy)-phenyl]-6-phenyl-8,9-dihydro-7H-benzocyclohepten-2-ol, N-butyl-N-methyl-2-{5-[4-(2-hydroxy-6-phenyl-8,9-dihydro-7H-benzocyclohepten-5-yl)-phenoxy]-pentylthio}-acetamide, 5-{4-[5-(4,4,5,5,5-pentafluoro-pentanesulfonyl)-pentyloxy]-phenyl}-6-phenyl-8,9-dihydro-7H-benzocyclohepten-2-ol, N-butyl-N-methyl-2-{5-[4-(2-hydroxy-6-phenyl-8,9-dihydro-7H-benzocyclohepten-5-yl)-phenoxy]-pentanesulfinyl}-acetamide, N-butyl-2-[2-({2-[4-(2-hydroxy-6-phenyl-8,9-dihydro-7H-benzocyclohepten-5-yl)-phenoxy]-ethyl}-methyl-amino)-ethanesulfinyl]-N-methyl-acetamide, N-butyl-2-[2-({2-[4-(2-hydroxy-6-phenyl-8,9-dihydro-7H-benzocyclohepten-5-yl)-phenoxy]-ethyl}-methyl-amino)-ethanesulfonyl]-N-methyl-acetamide, 6-(4-hydroxy-phenyl)-5-{4-[5-(4,4,5,5,5-pentafluoropentanesulfonyl)-pentyloxy]-phenyl}-8,9-dihydro-7H-benzocyclohepten-2-ol, N-butyl-2-{6-[4-(2-hydroxy-6-phenyl-8,9-dihydro-7H-benzocyclohepten-5-yl)-phenoxy]-hexanesulfinyl}-N-methyl-acetamide, N-butyl-2-{6-[4-(2-hydroxy-6-phenyl-8,9-dihydro-7H-benzocyclohepten-5-yl)-phenoxy]-hexylthio}-N-methyl-acetamide, 6- (4-hydroxyphenyl)-5-{4-[5-(4,4,5,5,5-pentafluoropentylthio)-pentyloxy]-phenyl}-8,9-dihydro-7H-benzocyclohepten-2-ol, 6-(4-hydroxy-phenyl)-5-{4-[5-(4,4,5,5,5-pentafluoropentane-sulfinyl)-pentyloxy]-phenyl}-8,9-dihydro-7H-benzocyclohepten-2-ol, 5-{4-[4-(4,4,5,5,5-pentafluoro-pentylthio)-butyloxy]-phenyl}-6-phenyl-8,9-dihydro-7H-benzocyclohepten-2-ol, 6-phenyl-5-{4-[4-(pyridin-2-ylmethylthio)-butyloxy]-phenyl}-8,9-dihydro- . 7H-benzocyclohepten-2-ol, 6-phenyl-5-{4-[5-(pyridin-2-ylmethylthio) -pentyloxy]-phenyl}-8,9-dihydro-7H-benzocyclohepten-2-ol, 6-phenyl-5-{4-[6-(pyridin-2-ylmethylthio)-hexyloxy]-phenyl}-8,9-dihydro-7H-benzocyclohepten-2-ol, 5-{4-[4-(4,4,5,5,5-pentafluoropentanesulfinyl)-butyloxy]-phenyl}-6-phenyl-8,9-dihydro-7H-benzocyclohepten-2-ol, 6-phenyl-5-{4-[4-(pyridin-2-ylmethanesulfinyl)-butyloxy]-phenyl}-8,9-dihydro-7H-benzocyclohepten-2-ol, 5-{4-[6-(4,4,5,5,5-pentafluoropentylthio)-hexyloxy]-phenyl}-6-phenyl-8,9-dihydro-7H-benzocyclohepten-2-ol, 5-{4-[6-(4,4,5,5,5-pentafluoropentanesulfinyl)-hexyloxy]-phenyl}-6-phenyl-8,9-dihydro-7H-benzocyclohepten-2-ol, 6-phenyl-5-{4-[6-(pyridin-2-ylmethanesulfinyl)-hexyloxy]-phenyl}-8,9-dihydro-7H-benzocyclohepten-2-ol, 6-phenyl-5-{4-[5-(pyridin-2-ylmethanesulfinyl)-pentyloxy]-phenyl}-8,9-dihydro-7H-benzocyclohepten-2-ol, 5-{4-[5-(furan-2-ylmethylthio)-pentyloxy]-phenyl}-6-phenyl-8,9-dihydro-7H-benzocyclohepten-2-ol, 6-phenyl-5-{4-[5-(thien-2-ylmethylthio)-pentyloxy]-phenyl}-8,9-dihydro-7H-benzocyclohepten-2-ol, 5-{4-[5-(furan-2-ylmethanesulfinyl)-pentyloxy]-phenyl}-6-phenyl-8,9-dihydro-7H-benzocyclohepten-2-ol, 5-{4-[5-(furan-2-ylmethanesulfonyl)-pentyloxy]-phenyl}-6-phenyl-8,9-dihydro-7H-benzocyclohepten-2-ol, 6-phenyl-5-{4-[5-(thien-2-ylmethanesulfonyl)-pentyloxy]-phenyl}-8,9-dihydro-7H-benzocyclohepten-2-ol, 6-phenyl-5-{4-[5-(thien-2-ylmethanesulfinyl)-pentyloxy]-phenyl}-8,9-dihydro-7H-benzocyclohepten-2-ol, 5-{4-[5-(3,3,4,4,5,5,5-heptafluoro-pentylthio)-pentyloxy]-phenyl}-6-phenyl-8,9-dihydro-7H-benzocyclohepten-2-ol, 5-{4-[2-(2-hydroxy-ethylamino)-ethoxy]-phenyl}-6-phenyl-8,9-dihydro-7H-benzocyclohepten-2-ol, 5-{4-[5-(4-fluorobutylthio)-pentyloxy]-phenyl}-6-phenyl-8,9-dihydro-7H-benzocyclohepten-2-ol, 5-{4-[5-(4-fluorobutanesulfinyl)-pentyloxy]-phenyl}-6-phenyl-8,9-dihydro-7H-benzocyclohepten-2-ol, 6-phenyl-5-{4-[5-(4,4,4-trifluoro-butylthio)-pentyloxy]-phenyl}-8,9-dihydro-7H-benzocyclohepten-2-ol, 6-phenyl-5-{4-[5-(4,4,4-trifluoro-butanesulfinyl)-pentyloxy]-phenyl}-8,9-. dihydro-7H-benzocyclohepten-2-ol, 5-(4-{5-[methyl-(4,4,5,5,5-pentafluoropentyl)-amino]-pentyloxy}-phenyl)-6-phenyl-8,9-dihydro-7H-benzocyclohepten-2-ol, 5-[4-(5-benzylthio-pentyloxy)-phenyl]-6-phenyl-8,9-dihydro-7H-benzocyclohepten-2-ol, 5-[4-(5-benzylsulfinylpentyloxy)-phenyl]-6-phenyl-8,9-dihydro-7H-benzocyclohepten-2-ol, 5-{4-[5-(4-methyl-benzylthio)-pentyloxy]-phenyl}-6-phenyl-8,9-dihydro-7H-benzocyclohepten-2-ol, 5-{4-[5-(4-methylbenzylsulfinyl)-pentyloxy]-phenyl}-6-phenyl-8,9-dihydro-7H-benzocyclohepten-2-ol, 6-phenyl-5-{4-[5-(4-trifluoromethyl-benzylthio)-pentyloxy]-phenyl}-8,9-dihydro-7H-benzocyclohepten-2-ol, 6-phenyl-5-{4-[5-(4-trifluoromethyl-benzylsulfinyl)-pentyloxy]-phenyl}-8,9-dihydro-7H-benzocyclohepten-2-ol, 6-phenyl-5-[4-(5-phenylthio-pentyloxy)-phenyl]-8,9-dihydro-7H-benzocyclohepten-2-ol, 6-phenyl-5-[4-(5-phenylsulfinylpentyloxy)-phenyl]-8,9-dihydro-7H-benzocyclohepten-2-ol, 6-phenyl-5-[4-(5-phenylsulfonyl-pentyloxy)-phenyl]-8,9-dihydro-7H-benzocyclohepten-2-ol, 5-{4-[5-(4-tert-butylphenylthio)-pentyloxy]-phenyl}-6-phenyl-8,9-dihydro-7H-benzocyclohepten-2-ol, 5-{4-[5-(4-tert-butylphenylsulfinyl)-pentyloxy)-phenyl)-6-phenyl-8,9-dihydro-7H-benzocyclohepten-2-ol, 5-{4-[5-(4-tert-butylphenylsulfonyl)-pentyloxy]-phenyl}-6-phenyl-8,9-dihydro-7H-benzocyclohepten-2-ol, 6-phenyl-5-{4-[5-(4-trifluoromethyl-phenylthio)-pentyloxy]-phenyl}-8,9-dihydro-7H-benzocyclohepten-2-ol, 6-phenyl-5-{4-[5-(4-trifluoromethyl-phenylsulfinyl)-pentyloxy]-phenyl}-8,9-dihydro-7H-benzocyclohepten-2-ol, 6-phenyl-5-{4-[5-(4-trifluoromethyl-phenylsulfonyl)-pentyloxy]-phenyl}-8,9-dihydro-7H-benzocyclohepten-2-ol.

11. Pharmaceutical agents that contain at least one compound of general formula I according to claim 1 as well as a pharmaceutically compatible vehicle.

12. Use of the compounds of general formula I according to claim 1 for the production of pharmaceutical agents.

13. Use of compounds of general formula I according to claim 1 in combination with one or more pure estrogens for the production of pharmaceutical agents for the treatment of gynecological disorders.

14. Use of compounds of general formula I according to claim 1 in combination with one or more pure estrogens for the production of pharmaceutical agents for hormone replacement therapy.

15. Use of compounds of general formula I according to claim 1 in combination with one or more pure estrogens for the production of pharmaceutical agents for female fertility control.

## Revendications

1. Benzocycloheptènes de la formule générale I 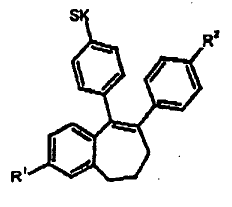 dans laquelle
R¹ et R² représentent indépendamment l'un de l'autre un atome d'hydrogène, un groupe hydroxyle, un groupe alcoxy en C₁ à C₁₀ éventuellement substitué, un groupe alcanoyloxy en C₁ à C₁₀ éventuellement substitué ou un groupe aroyloxy en C₇ à C₁₅ éventuellement substitué, ainsi que
SK représente une chaîne latérale -A-B-Z,
où
A est une liaison directe ou un atome d'oxygène,
B est un groupe alkylène, alcénylène ou alcynylène linéaire ou ramifié, éventuellement substitué comportant jusqu'à 10 atomes de carbone,
Z est un groupe -D-SOₓ-E-G, un groupe amino -NR⁷R⁸ ou un substituant G,
où
D est une liaison directe ou un groupe -NR³(R⁴-), R³ un groupe alkyle, alcényle ou alcynyle linéaire ou ramifié comportant jusqu'à 10 atomes de carbone et R⁴ un groupe alkylène, alcénylène ou alcynylène linéaire ou ramifié, éventuellement substitué, comportant jusqu'à 10 atomes de carbone, l'atome d'azote pouvant aussi être introduit dans un système de noyau tétragonal à heptagonal,
x a une valeur de 0, 1 ou 2,
E est un groupe alkylène, alcénylène ou alcynylène linéaire ou ramifié, éventuellement substitué, comportant jusqu'à 10 atomes de carbone,
G est un groupe alkyle partiellement ou totalement fluoré, linéaire ou ramifié, comportant jusqu'à 5 atomes de carbone, un radical aryle ou hétéroaryle éventuellement substitué, un radical carbamoyle -C(O)-NR⁵R⁶ avec R⁵ et R⁶ ayant la signification de R⁷ et R⁸, un atome d'halogène ou un atome d'hydrogène, R⁷ et R⁸ représentant indépendamment l'un de l'autre un atome d'hydrogène, un groupe alkyle, alcényle ou alcynyle linéaire ou ramifié, éventuellement partiellement fluoré, comportant jusqu'à 14 atomes de carbone, qui peut être interrompu par un à trois hétéroatomes -O- et -S- et groupements -NR⁹, où R⁹ est un atome d'hydrogène ou un groupe alkyle en C₁ à C₃, un radical aryle ou hétéroaryle éventuellement substitué une ou deux fois, un radical cycloalkyle en C₃ à C₁₀ éventuellement substitué une ou deux fois, un radical cycloalkylalkyle en C₄ à C₁₅ éventuellement substitué une ou deux fois, un radical aralkyle en C₇ à C₂₀ éventuellement substitué une ou deux fois, un radical hétéroaryl(C₁-C₈)alkyle éventuellement substitué une ou deux fois ou un radical aminoalkyle éventuellement substitué, un radical biphénylène ou un radical de formule -C(O)R¹⁰, où R¹⁰ peut avoir les signification indiquées plus haut pour R⁷ et/ou R⁸,
R⁷ et R⁸ forment avec l'atome d'azote auquel ils sont liés un hétérocycle saturé ou insaturé à 5 ou 6 membres cycliques, qui contient éventuellement un ou deux autres hétéroatomes, choisis parmi l'azote, l'oxygène et le soufre, et est éventuellement substitué,
et dans -A-B-Z, lorsque A est un atome d'oxygène et Z représente G, G ne peut pas être un atome d'hydrogène ou un atome d'halogène, ou bien, lorsque A représente un atome d'oxygène et Z un groupe amino -NR⁷R⁸, où R⁷ et R⁸ représentent à chaque fois un groupe méthyle ou forment ensemble avec l'atome d'azote un noyau de pyrrolidine, B présente au moins 3 atomes de carbone,
ainsi qu'à l'exclusion du composé de la formule générale I, où R¹ = R² = hydrogène, SK = A-B-Z-, A = 0, B = -(CH₂)₂- et Z = N(C₂H₅)₂.

2. Composés de la formule générale I selon la revendication 1, dans lesquels R¹ et/ou R² représentent un atome d'hydrogène.

3. Composés de la formule générale I selon la revendication 1, dans lesquels A est un atome d'oxygène.

4. Composés de la formule générale I selon la revendication 1, dans lesquels B est une chaîne d'alcénylène linéaire comportant de 1 à 6 atomes de carbone.

5. Composés de la formule générale I selon la revendication 1, dans lesquels la chaîne latérale SK est choisie dans le groupe des chaînes latérales suivantes:
-O-(CH₂)₅S(CH₂)₃C₂F₅
-O-(CH₂)₅SO(CH₂)₃C₂F₅
-O-(CH₂)₅SO₂(CH₂)₃C₂F₅
-O-(CH₂)₂-N(CH₃)-(CH₂)₃-S(CH₂)₃C₂F₅
-O-(CH₂)₂-N(CH₃)-(CH₂)₃-SO-(CH₂)₃C₂F₅
-O-(CH₂)₅S(CH₂)-C(O)N(CH₃)-(CH₂)₃CH₃
-O-(CH₂)₅SO(CH₂)-C(O)N(CH₃)-(CH₂)₃CH₃
-O-(CH₂)₂-NH(CH₂)OH
-O-(CH₂)₂-N(CH₃)-(CH₂)₂-SO(CH₂)-C(O)N(CH₃)-(CH₂)₃CH₃
-O-(CH₂)₂-N(CH₃)-(CH₂)₂-SO₂(CH₂)-C(O)N(CH₃)-(CH₂)₃CH₃
-O-(CH₂)₆S(CH₂)-C(O)N(CH₃)-(CH₂)₃CH₃
-O-(CH₂)₆SO(CH₂)-C(O)N(CH₃)-(CH₂)₃CH₃
-O-CH₃
-O-(CH₂)₅-F
-O-(CH₂)₄-F,
-O-(CH₂)₃-F
-O-(CH₂)₂-F
-O-(CH₂)₅-Cl
-O-(CH₂)₄-Cl
-O-(CH₂)₃-Cl
-O-(CH₂)₂-Cl
-O-(CH₂)₆S(CH₂)₃C₂F₅
-O-(CH₂)₆SO(CH₂)₃C₂F₅
-O-(CH₂)₆SO(CH₂)-2-pyridyle
-O-(CH₂)₅SO(CH₂)-2-pyridyle
-O-(CH₂)₅S(CH₂)₂C₃F₇
-O-(CH₂)₄S(CH₂)₃C₂F₅
-O-(CH₂)₄SO(CH₂)₃C₂F₅
-O-(CH₂)₄SO₂(CH₂)₃C₂F₅
-O-(CH₂)₄S(CH₂)-2-pyridyle
-O-(CH₂)₄SO(CH₂)-2-pyridyle
-O-(CH₂)₅S(CH₂)-2-pyridyle
-O-(CH₂)₅SO(CH₂)-2-pyridyle
-O-(CH₂)₆S(CH₂)-2-pyridyle
-O-(CH₂)₆SO(CH₂)-2-pyridyle
-O-(CH₂)₅S(CH₂)-2-furyle
-O-(CH₂)₅SO(CH₂)-2-furyle
-O-(CH₂)₅SO₂(CH₂)-2-furyle
-O-(CH₂)₅S(CH₂)-2-thiényle
-O-(CH₂)₅SO(CH₂)-2-thiényle
-O-(CH₂)₅S(CH₂)₄-F
-O-(CH₂)₅SO(CH₂)₄-F
-O-(CH₂)₅SO(CH₂)₃-CF₃
-O-(CH₂)₅SO(CH₂)₃-CF₃
-O-(CH₂)₅-N(CH₃)-(CH₂)₃-C₂F₅
-O-(CH₂)₅S(CH₂)-phényle
-O-(CH₂)₅SO(CH₂)-2-phényle
-O-(CH₂)₅S(CH₂)-p-tolyle
-O-(CH₂)₅SO(CH₂)-p-tolyle
-O-(CH₂)₅S(CH₂)-p-CF₃-phényle
-O-(CH₂)₅SO(CH₂)-p-CF₃-phényle
-O-(CH₂)₅S-phényle
-O-(CH₂)₅SO-phényle
-O-(CH₂)₅S-(p-tolyle)
-O-(CH₂)₅SO-(p-tolyle)
-O-(CH₂)₅S-(p-CF₃-phényle)
-O-(CH₂)₅SO-(p-CF₃-phényle)

6. Composés de la formule générale I selon la revendication 1, où la chaîne latérale SK est choisie dans le groupe des chaînes latérales suivantes:
- (CH₂)₅N(CH₃)(CH₂)₃C₂F₅
- (CH₂)₅N(CH₃)(CH₂)₆C₂F₅
- (CH₂)₅N(CH₃)(CH₂)₇C₂F₅
- (CH₂)₅N(CH₃)(CH₂)₈C₂F₅
- (CH₂)₆N(CH₃)(CH₂)₆C₂F₅
- (CH₂)₆N(CH₃)(CH₂)₇C₂F₅
- (CH₂)₆N(CH₃)(CH₂)₈C₂F₅
- (CH₂)₅N(CH₃)(CH₂)₂C₄F₉
- (CH₂)₅N(CH₃)(CH₂)₃C₆F₁₃
- (CH₂)₅N(CH₃)(CH₂)₃C₈F₁₇
- (CH₂)₅N(CH₃)(CH₂)₆C₄F₉
- (CH₂)₅N(CH₃)(CH₂)₆C₆F₁₃
- (CH₂)₅N(CH₃)(CH₂)₆C₈F₁₇
- (CH₂)₅N(CH₃)H
- (CH₂)₅N(CH₃) (CH₂)₉H
- (CH₂)5-1-pyrrolidinyle
- (CH₂)₉S(CH₂)₃C₂F₅
- (CH₂)₉SO(CH₂)₃C₂F₅
- (CH₂)₉SO₂(CH₂)₃C₂F₅

7. Composés de la formule générale I selon la revendication 1, dans lesquels la chaîne latérale SK est choisie dans la formule générale partielle 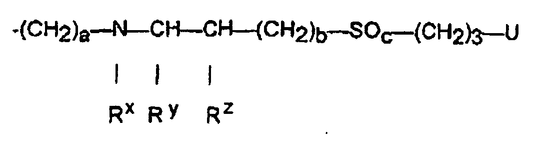 dans laquelle
a a une valeur de 4, 5 ou 6,
b a une valeur de 0, 1 ou 2,
c a une valeur de 0, 1 ou 2,
R^{x} est un atome d'hydrogène ou un groupe alkyle en C₁₋₅,
R^{y} et R^{z} représentent chacun un atome d'hydrogène, ou
R^{x} et R^{y} représentent ensemble un groupe alkylène -(CH₂)_{d}- avec d = 2, 3, 4 ou 5 et R^{z} est un atome d'hydrogène ou
R^{x} et R^{z} représentent ensemble un groupe alkylène -(CH₂)ₑ avec e = 2, 3 ou 4 et R^{y} est un atome d'hydrogène, et
U est un radical éthyle non substitué ou bien fluoré de une à cinq fois, ou
bien le substituant terminal -(CH₂)₃-U dans la chaîne latérale est remplacé par un radical aryle ou hétéroaryle éventuellement substitué, qui est lié à l'atome de soufre directement ou via un groupe mono-, di- ou triméthylène.

8. Composés de la formule générale I selon la revendication 7, dans lesquels SK est la chaîne latérale -(CH₂)₅N(CH₃)(CH₂)₃S(CH₂)₃C₂F₅.

9. Composés de la formule générale I selon la revendication 7, dans lesquels SK est la chaîne latérale (CH₂)₅N(R^{x}) (CHR^{y})CH₂S(CH₂)₃C₂F₅ avec R^{X}+R^{Y}= - (CH₂)₃-.

10. Composés de la formule générale I, à savoir sulfure de (4,4,5,5,5-pentafluoropentyl)-{5-[4-(6-phényl-8,9-dihydro-7H-benzocycloheptén-5-yl)-phénoxy]-pentyle} sulfoxyde de (4,4,5,5,5-pentafluoropentyl)-{5-[4-(6-phényl-8,9-dihydro-7H-benzocycloheptén-5-yl)-phénoxy]-pentyle} méthyl-[3-(4,4,5,5,5-pentafluoropentylthio)-propyl]-{2-[4-(6-phényl-8,9-dihydro-7H-benzocycloheptén-5-yl)-phénoxy]éthyl}-amine méthyl-[3-(4,4,5,5,5-pentafluoropentylsulfinyl)-propyl]-{2-[4-(6-phényl-8,9-dihydro-7H-benzocycloheptén-5-yl)-phénoxy]éthyl}-amine thioacétate de S-{5-[4-(6-phényl-8,9-dihydro-7H-benzocycloheptén-5-yl)-phénoxy]-pentyle} N-butyl-N-méthyl-2-{5-{4-(6-phényl-8,9-dihydro-7H-benzocycloheptén-5-yl)-phénoxy]-pentylthio}-acétamide N-butyl-N-méthyl-2-{5-[4-(6-phényl-8,9-dihydro-7H-benzocycloheptén-5-yl)-phénoxy]-pentanesulfinyl}-acétamide 5-{4-[5-(4,4,5,5,5-pentafluoropentylthio)-pentyloxy]-phényl}-6-phényl-8,9-dihydro-7H-benzocycloheptén-2-ol 5-{4-[5-(4,4,5,5,5-pentafluoropentanesulfinyl)-pentyloxy]-phényl}-6-phényl-8,9-dihydro-7H-benzocycloheptén-2-ol 5-[4-(2-{méthyl-[3-(4,4,5,5,5-pentafluoropentanesulfinyl)-propyl]-amino}-éthoxy)phényl]-6-phényl-8,9-dihydro-7H-benzocycloheptén-2-ol 5-[4-(2-{méthyl-[3-(4,4,5,5,5-pentafluoropentylthio)-propyl]-amjno}-éthoxy)-phényl]-6-phényl-8,9-dihydro-7H-benzocycloheptén-2-ol N-butyl-N-méthyl-2-{5-[4-(2-hydroxy-6-phényl-8,9-dihydro-7H-benzocycloheptén-5-yl)-phénoxy]-pentylthio}-acétamide 5-{4-[5-(4,4,5,5,5-pentafluoropentanesulfonyl)-pentyloxy]-phényl}-6-phényl-8,9-dihydro-7H-benzocycloheptén-2-ol N-butyl-N-méthyl-2-{5-[4-(2-hydroxy-6-phényl-8,9-dihydro-7H-benzocycloheptén-5-yl)-phénoxy]-pentanesulfinyl}-acétamide N-butyl-2-[2-({2-[4-(2-hydroxy-6-phényl-8,9-dihydro-7H-benzocycloheptén-5-yl)phénoxy]-éthyl}-méthyl-amino)-éthanesulfinyl]-N-méthylacétamide N-butyl-2-[2-({2-[4-(2-hydroxy-6-phényl-8,9-dihydro-7H-benzocycloheptén-5-yl)-phénoxy]-éthyl}-méthylamino)-éthanesulfonyl]-N-méthylacétamide 6-(4-hydroxyphényl)-5-{4-[5-(4,4,5,5,5-pentafluoropentanesulfonyl)-pentyloxy]-phényl}-8,9-dihydro-7H-benzocycloheptén-2-ol N-butyl-2-{6-[4-(2-hydroxy-6-phényl-8,9-dihydro-7H-benzocycloheptén-5-yl)-phénoxy]-hexanesulfinyl}-N-méthylacétamide N-butyl-2-{6-[4-(2-hydroxy-6-phényl-8,9-dihydro-7H-benzocycloheptén-5-yl)-phénoxy]-hexylthio}-N-méthylacétamide 6-(4-hydroxyphényl)-5-{4-[5-(4,4,5,5,5-pentafluoropentylthio)-pentyloxy]-phényl}-8,9-dihydro-7H-benzocycloheptén-2-ol 6-(4-hydroxyphényl)-5-{4-[5-(4,4,5,5,5-pentafluoropentanesulfinyl)-pentyloxy]-phényl}-8,9-dihydro-7H-benzocycloheptén-2-ol 5-{4-[4-(4,4,5,5,5-pentafluoropentylthio)-butyloxy]-phényl}-6-phényl-8,9-dihydro-7H-benzocycloheptén-2-ol 6-phényl-5-{4-[4-(pyridin-2-ylméthylthio)-butyloxy]phényl}-8,9-dihydro-7H-benzocycloheptén-2-ol 6-phényl-5-{4- [5-(pyridin-2-ylméthylthio)-pentyloxy]-phényl}-8,9-dihydro-7H-benzocycloheptén-2-ol 6-phényl-5-{4-[6-(pyridin-2-ylméthylthio)-hexyloxy]-phényl}-8,9-dihydro-7H-benzocycloheptén-2-ol 5-{4-[4-(4,4,5,5,5-pentafluoropentanesulfinyl)-butyloxy]-phényl}-6-phényl-8,9-dihydro-7H-benzocycloheptén-2-ol 6-phényl-5-{4-[4-(pyridin-2-ylméthanesulfinyl)-butyloxy]-phényl}-8.9-dihydro-7H-benzocycloheptén-2-ol 5-{4-[6-(4,4,5,5,5-pentafluoropentylthio)-hexyloxy]-phényl}-6-phényl-8,9-dihydro-7H-benzocycloheptén-2-ol 5-{4-[6-(4,4,5,5,5-pentafluoropentanesulfinyl)-hexyloxy]-phényl}-6-phényl-8,9-dihydro-7H-benzocycloheptén-2-ol 6-phényl-5-{4-[6-(pyridin-2-ylméthanesulfinyl)-hexyloxy]-phényl}-8,9-dihydro-7H-benzocycloheptén-2-ol 6-phényl-5-{4-(5-[pyridin-2-ylméthanesulfinyl)-pentyloxy]-phényl}-8,9-dihydro-7H-benzocycloheptén-2-ol 5-{4-[5-(furan-2-ylméthylthio)-pentyloxy]-phényl}-6-phényl-8,9-dihydro-7H-benzocycloheptén-2-ol 6-phényl-5-{4-[5-(thién-2-ylméthylthio)-pentyloxy]-phényl}-8,9-dihydro-7H-benzocylcloheptén-2-ol 5-{4-[5-(furan-2-ylméthanesulfinyl)-pentyloxy)-phényl}-6-phényl-8,9-dihydro-7H-benzocycloheptén-2-ol 5-{4-[5-(furan-2-ylméthanesulfonyl)-pentyloxy)-phényl}-6-phényl-8,9-dihydro-7H-benzocycloheptén-2-ol 6-phényl-5-{4-[5-(thién-2-ylméthanesulfonyl)-pentyloxy]-phényl}-8,9-dihydro-7H-benzocycloheptén-2-ol 6-phényl-5-{4-[5-(thién-2-ylméthanesulfinyl)-pentyloxy]-phényl}-8,9-dihydro-7H-benzocycloheptén-2-ol 5-{4-[5-(3,3,4,4,5,5,5-heptafluoropentylthio)-pentyloxy]-phényl}-6-phényl-8,9-dihydro-7H-benzocycloheptén-2-ol 5-{4-[2-(2-hydroxyéthylamino)-éthoxy]-phényl}-6-phényl-8,9-dihydro-7H-benzocycloheptén-2-ol 5-{4-[5-(4-fluorobutylthio)-pentyloxy]-phényl}-6-phényl-8,9-dihydro-7H-benzocycloheptén-2-ol 5-{4-[5-(4-fluorobutanesulfinyl)-pentyloxy]-phényl}-6-phényl-8,9-dihydro-7H-benzocycloheptén-2-ol 6-phényl-5-{4-[5-(4,4,4-trifluorobutylthio)-pentyloxy]-phényl}-8,9-dihydro-7H-benzocycloheptén-2-ol 6-phényl-5-{4-[5-(4,4,4-trifluorobutanesulfinyl)-pentyloxy]-phényl}-8,9-dihydro-7H-benzocycloheptén-2-ol 5-(4-{5-[méthyl-(4,4,5,5,5-pentafluoropentyl)-amino]-pentyloxy}-phényl-6-phényl-8,9-dihydro-7H-benzocycloheptén-2-ol 5-[4-(5-benzylthiopentyloxy)-phényl]-6-phényl-8,9-dihydro-7H-benzocycloheptén-2-ol 5-[4-(5-benzylsulfinylpentyloxy)-phényl]-6-phényl-8,9-dihydro-7H-benzocycloheptén-2-ol 5-{4-[5-(4-méthylbenzylthio)-pentyloxy]-phényl}-6-phényl-8,9-dihydro-7H-benzocycloheptén-2-ol 5-{4-[5-(4-méthylbenzylsulfinyl)-pentyloxy]-phényl}-6-phényl-8,9-dihydro-7H-benzocycloheptén-2-ol 6-phényl-5-{4-[5-(4-trifluorométhyl-benzylthio)-pentyloxy}-phényl]-8,9-dihydro-7H-benzocycloheptén-2-ol 6-phényl-5-{4-[5-[4-trifluorométhyl-benzylsulfinyl)-pentyloxy]-phényl}-8,9-dihydro-7H-benzocycloheptén-2-ol 6-phényl-5-[4-(5-phénylthio-pentyloxy)-phényl]-8,9-dihydro-7H-benzocycloheptén-2-ol 6-phényl-5-[4-(5-phénylsulfinyl-pentyloxy)-phényl]-8,9-dihydro-7H-benzocycloheptén-2-ol 6-phényl-5-[4-(5-phénylsulfonyl-pentyloxy)-phényl]-8,9-dihydro-7H-benzocycloheptén-2-ol 5-{4-[5-(4-tert-butyl-phénylthio)-pentyloxy]-phényl}-6-phényl-8,9-dihydro-7H-benzocycloheptén-2-ol 5-{4-[5-(4-tert.-butyl-phénylsulfinyl)-pentyloxy]-phényl}-6-phényl-8,9-dihydro-7H-benzocycloheptén-2-ol 5-{4-[5-(4-tert.-butyl-phénylsulfonyl)-pentyloxy]-phényl}-6-phényl-8,9-dihydro-7H-benzocycloheptén-2-ol 6-phényl-5-{4-[5-(4-trifluorométhyl-phénylthio)-pentyloxy]-phényl}-8,9-dihydro-7H-benzocycloheptén-2-ol 6-phényl-5-{4-[5-(4-trifluorométhyl-phénylsulfinyl)-pentyloxy]-phényl}-8,9-dihydro-7H-benzocycloheptén-2-ol 6-phényl-5-{4-[5-(4-trifluorométhyl-phénylsulfonyl)-pentyloxy]-phényl}-8,9-dihydro-7H-benzocycloheptén-2-ol.

11. Médicament contenant au moins un composé de la formule générale I selon la revendication 1 ainsi qu'un excipient pharmaceutiquement compatible.

12. Utilisation des composés selon la formule générale I selon la revendication 1 pour la préparation de médicaments.

13. Utilisation de composés de la formule générale I selon la revendication 1 en combinaison avec un ou plusieurs oestrogènes purs pour la préparation de médicaments destinés au traitement de troubles gynécologiques.

14. Utilisation de composés de la formule générale I selon la revendication 1 en combinaison avec un ou plusieurs oestrogènes purs pour la préparation de médicaments destinés à la thérapie de remplacement d'hormones.

15. Utilisation de composés de la formule générale I selon la revendication 1 en combinaison avec un ou plusieurs oestrogènes purs pour la préparation de médicaments destinés au contrôle de la fertilité féminine.
